# EUROPEAN PATENT SPECIFICATION

(11) **EP 1 487 343 B1**
(45) Date of publication and mention of the grant of the patent: **31.12.2008**
(21) Application number: 03711417.0
(22) Date of filing: 05.03.2003
(51) Int. Cl.: G01N 33/58, A61B 6/00

(54) **BIOSPECIFIC CONTRAST AGENTS**
BIOSPEZIFISCHE KONTRASTMITTEL
PRODUITS DE CONTRASTE BIOSPECIFIQUES

(30) Priority: 05.03.2002 US 361924 P
(43) Date of publication of application: 22.12.2004
(73) Proprietor: BOARD OF REGENTS, THE UNIVERSITY OF TEXAS SYSTEM, Austin, Texas 78701 (US)
(72) Inventor: SOKOLOV, Konstantin, Austin, TX 78726 (US); KORGEL, Brian, A., Round Rock, TX 78681 (US); ELLINGTON, Andrew, D., Austin, TX 78703 (US); RICHARDS-KORTUM, Rebecca, Austin, TX 78739 (US)
(74) Representative: Dehmel, Albrecht
(86) International application number: PCT/US2003/006730
(87) International publication number: WO 2003/075765

(56) References cited:
- WO-A-00/55367
- WO-A-01/30967
- WO-A-97/31259
- US-A- 5 939 021
- US-A- 6 159 445
- US-A1- 2001 055 764
- US-B1- 6 251 616
- US-B1- 6 287 768
- HAINFELD JAMES F ET AL: "New frontiers in gold labeling" JOURNAL OF HISTOCHEMISTRY AND CYTOCHEMISTRY, vol. 48, no. 4, April 2000 (2000-04), pages 471-480, XP002368643 ISSN: 0022-1554
- ROBINSON J M ET AL: "Applications of gold cluster compounds in immunocytochemistry and correlative microscopy: Comparison with colloidal gold" JOURNAL OF MICROSCOPY (OXFORD), vol. 199, no. 3, September 2000 (2000-09), pages 163-179, XP002368644 ISSN: 0022-2720
- FRITZSCHE W: "DNA-GOLD CONJUGATES FOR THE DETECTION OF SPECIFIC MOLECULAR INTERACTIONS" JOURNAL OF BIOTECHNOLOGY, ELSEVIER SCIENCE PUBLISHERS B.V., AMSTERDAM, NL, vol. 82, no. 1, November 2001 (2001-11), pages 37-46, XP001166865 ISSN: 0168-1656
- HOFFMAN P ET AL: "ADENOVIRUS E3 PROTEIN CAUSES CONSTITUTIVELY INTERNALIZED EPIDERMAL GROWTH FACTOR RECEPTORS TO ACCUMULATE IN A PRELYSOSOMAL COMPARTMENT, RESULTING IN ENHANCED DEGRADATION" MOLECULAR AND CELLULAR BIOLOGY, AMERICAN SOCIETY FOR MICROBIOLOGY, WASHINGTON, US, vol. 14, no. 6, June 1994 (1994-06), pages 3695-3706, XP009013081 ISSN: 0270-7306
- MULTHAUPT HINKE A B ET AL: "Ultrastructural localization of human papilloma virus by nonradioactive in situ hybridization on tissue of human cervical intraepithelial neoplasia" LABORATORY INVESTIGATION, vol. 67, no. 4, 1992, pages 512-518, XP008060273 ISSN: 0023-6837
- LINARES-CRUZ GUSTAVO ET AL: "Combined analysis of in situ hybridization, cell cycle and structural/markers using reflectance and immunofluorescence confocal microscopy" HISTOCHEMICAL JOURNAL, vol. 27, no. 1, 1995, pages 15-23, XP008060291 ISSN: 0018-2214
- CHAN W C W ET AL: "Luminescent quantum dots for multiplexed biological detection and imaging" CURRENT OPINION IN BIOTECHNOLOGY, LONDON, GB, vol. 13, no. 1, February 2002 (2002-02), pages 40-46, XP002256995 ISSN: 0958-1669
- NIDA ET AL: "Fluorescent nanocrystals for use in early cervical cancer detection" GYNECOLOGIC ONCOLOGY, ACADEMIC PRESS, LONDON, GB, vol. 99, no. 3, December 2005 (2005-12), pages S89-S94, XP005217250 ISSN: 0090-8258

## Description

### Background of the Invention

### 1. Field of the Invention

The present invention relates to metallic nanoparticles attached to a molecular probe specific for a biomarker for use in in vivo diagnosis of cervical cancer through reflectance imaging, wherein the metallic nanoparticle(s) exhibit characteristic optical scattering when subjected to reflactance imaging.

### 2. Description of Related Art

Cancer is the second leading cause of death in the U.S. exceeded only by heart disease. The majority of cancers are of epithelial origin. Earlier detection of preinvasive curable epithelial neoplasia remains the best way to ensure patient survival and quality of life. The American Cancer Society estimated that 1,200,000 people would be diagnosed with cancer in 1999, resulting in 563,000 deaths.

Cervical cancer is the third most common cancer in women worldwide and the leading cause of cancer mortality in women in developing countries. The curable precursor to cervical cancer is cervical intra-epithelial neoplasia (CIN). In the U.S. over $6 billion is spent annually in the evaluation and treatment of low-grade precursor lesions. Approximately 50 million Pap smears are done annually in the U.S. to screen for cervical cancer and its precursor [1]; of these, the NCI estimates 6-7% are abnormal. Mass screening of asymptomatic women with the Pap smear is considered one of the most successful public health measures in the prevention of cancer [2]; the decline in the incidence and mortality of cervical cancer over the last 40 years have been attributed mainly to the introduction of this screening test.

Cervical cancer goes undetected in developing countries because of the cost of the tests and the lack of trained personnel and resources.. In the U.S., resources are wasted on the evaluation and treatment of lesions not likely to progress to cancer. Both screening and detection could be vastly improved by *in vivo* optical imaging technologies that improve, automate, and decrease the cost of screening and detection.

Despite the tremendous potential of optical techniques for identifying cancers and pre-cancers (such as cervical cancers and pre-cancers), optical clinical applications used for detection are still limited by low intrinsic contrast between normal and diseased tissues, especially at the earliest stages of pre-cancer development. Notwithstanding that contrast may be increased using conventional, exogenous agents (such as Acetic Acid), conventional optical techniques could be greatly improved if even further contrast enhancing mechanisms could be exploited. In particular, it would be greatly beneficial if contrast could be increased in a targeted manner - *i.e.,* if distinctive contrast agents could be associated with specific biomarkers (biospecific contrast agents).

Numerous studies using biopsy specimens have shown that cancer specific biomarkers can significantly improve the ability to recognize and grade cervical pre-cancers and to use this information to predict whether the lesion will progress to higher grades of pre-cancer and cancer. However, all currently known biomarkers must be assessed *in vitro -* there is a large gap between clinically available methods of *in vivo* tissue analysis of tissue and the current techniques to quantitatively assess biomarkers.

An important new approach to treating cervical pre-cancer is chemoprevention. Chemoprevention refers to the use of chemical agents to prevent or delay the development of cancer in healthy populations or patients with precancerous tissue changes. [3,4]. Several chemoprevention trials have been carried out in patients with CIN. [3]. Despite their promise, chemoprevention studies have several inherent problems. One is that many patients hesitate to enroll in such trials because they require multiple biopsies throughout the period when the chemopreventive agent is given; biopsies are processed to quantitatively measure biomarkers associated with cancer progression and assess drug response. A second problem is that the biopsy process itself can interrupt the natural progression of the lesion. Many times these lesions are small enough that the biopsy is the cure; frequent biopsies make it difficult to accurately assess drug response. Thus, tools to assess quantitative biomarkers that do not require biopsy could considerably improve chemoprevention studies.

In view of at least the foregoing, there is a need for new, improved techniques that at least (a) improve cancer and pre-cancer screening and detection (including cervical cancer and pre-cancer), (b) improve optical imaging techniques by providing increased contrast to targeted regions, (c) close the gap between clinically available methods of *in vivo* tissue analysis and techniques to quantitatively assess biomarkers, and (d) assess quantitative biomarkers, while not requiring biopsy, to improve chemoprevention studies. Such techniques would be beneficial in, for example, the screening, detection, identification, monitoring, and diagnosis and corresponding treatment of a wide range of maladies including cancers and pre-cancers. Even more particularly, such techniques may be especially beneficial for cervical cancers and pre-cancers. With such techniques in place, it is hoped that the incidence of cancers such as cervical cancer, and the costs of detecting cancer and its precursors, may be reduced in the U.S. and in the developing world.

Any shortcomings referenced above are not intended to be exhaustive, but rather are among many that tend to impair the effectiveness of previously known techniques concerning . Other noteworthy problems may also exist; however, those mentioned here are sufficient to demonstrate that methodology appearing in the art have not been altogether satisfactory and that a significant need exists for the techniques described and claimed herein.

### Summary of the Invention

Shortcomings of the prior art are reduced or eliminated by the techniques disclosed herein. These techniques are applicable to a vast number of applications, including but not limited to any application that would benefit from the use of biospecific contrast agents. However the invention relates to the optical detection of cervical cancers and pre-cancers. More specifically, these techniques are applicable to the detection of cervical cancers and pre-cancers using reflectance imaging by biospecific contrast agents made up of reflective nanoparticles.

One aspect of the present invention is (are) one or more metallic nanoparticle(s) attached to a molecular probe specific for a biomarker for use in *in vivo* diagnosis of cervical cancer through reflectance imaging, wherein the metallic nanoparticle(s) exhibit characteristic optical scattering when subjected to reflectance imaging. In a preferred embodiment of the present invention the one or more metallic nanoparticle(s) comprise(s) gold or silver. In another preferred embodiment of the present invention the biomarker is EGFR, MMP-2 or MMP-9.

Another aspect of the present invention is the use of one or more metallic nanoparticle(s) attached to a molecular probe specific for a biomarker in the manufacture of a diagnostic agent for the *in vivo* diagnosis of cervical cancer through reflectance imaging, wherein the metallic nanoparticle(s) exhibit characteristic optical scattering when subjected to reflectance imaging. In a preferred embodiment of the present invention the one or more metallic nanoparticle(s) comprise(s) gold or silver. In another preferred embodiment of the present invention the biomarker is EGFR, MMP-2 or MMP-9.

*In vivo* optical imaging involves, modern nano-chemistry, combinatorial chemistry and molecular engineering, permitting optical imaging with molecular specificity. Optically interrogated contrast agents based on metal nanoparticles are attached to probe molecules with a high affinity to a specific biomarker on the surface of pre-cancerous and cancerous cells. This combination of optical imaging with cancer specific contrast agents may increase optical contrast between normal and neoplastic tissue and provide useful molecular-specific information to assist clinicians in earlier detection and monitoring of pre-cancers. The techniques described here accordingly may significantly benefit health care by reducing the number of unnecessary biopsies, enabling combined diagnosis and therapy, and reducing the need for clinical expertise.

Techniques of this invention address some of the major shortcomings of *in vivo* optical imaging: low signal (especially in the case of fluorescence), low contrast between normal and diseased tissue, and lack of molecular specificity. To address these problems a combination of photonic probes (e.g., metal nanoparticles and quantum dots) and cancer specific molecular probes may be used. This combination may result in contrast agents that provide bright optical signals with no or very little effects of photobleaching, enhanced contrast between normal and malignant tissue, and molecular specificity characteristic for histopathologic immunostains.

By the provision of quantitative information about biomolecular signatures of cancer in the living body optical detection and monitoring of neoplasia, may be further improved. This, in turn, may reduce the number of unnecessary biopsies, enable combined diagnosis and therapy, and reduce the need for clinical expertise. Using techniques described herein may lead to at least three important clinical outcomes: (1) photonic probes with increased molecular sensitivity and specificity may lead to inexpensive, improved screening strategies that can be used in the U.S. and developing world to reduce the incidence of cancer; (2) photonic probes that specifically increase contrast between normal and pre-cancerous tissue may reduce the costs of detecting pre-cancers; and (3) photonic probes that may be quantitatively assessed without the need for biopsy may greatly facilitate monitoring of cancerous tissue in a wide range of applications, including but not limited to chemoprevention studies.

Optical interrogation described herein may provide non-invasive, real-time assessment of tissue pathology, while contrast agents may give molecular specificity and selectivity. The combination of these optical imaging techniques with the cancer-specific contrast agents may increase optical contrast between normal and neoplastic tissue and provide useful molecular-specific information to assist clinicians in earlier detection of pre-cancers. These innovations may significantly improve the specificity and selectivity of pre-cancer detection.

As will be readily understood by those of skill in the art having the benefit of the present disclosure, the techniques described herein are not limited to applications involving the analysis of pre-cancerous or cancerous tissue. Rather, the techniques may be applied to a wide range of applications including but not limited to the analysis of unpurified human fluids such as whole blood, serum, or urine for the presence of circulating cancer cells and cancer related biomarkers. Such applications may thus be used to achieve novel approaches toward a more general form of cancer screening and diagnosis.

Although the present invention is limited to cervical cancers and pre-cancers, those having skill in the art will understand that the techniques described herein may be applied with equal success to various other systems. The cervix has been focused upon for number of reasons. Cervical lesions have long been thought to be the best model for progression from mildly dysplastic lesions to severely dysplastic lesions to invasive cancer. These factors make the cervix a unique organ, well suited to the development of screening and diagnostic interventions. However, the proposed activities provide an example of a new venue for development of molecular optical imaging modalities for pre-cancer detection (and detection of other conditions) that can be extended to many organ sites, as will be understood by those of skill in the art having the benefit of the present disclosure.

As used herein, "characteristic" as used in, for instance, "characteristic optical scattering" shall be interpreted broadly to mean "distinctive" or "having a feature that helps to distinguish a thing." In particular, "characteristic optical scattering" brought about by a metallic nanoparticle may be distinguished from optical scattering brought about by some other matter. As used herein, "biomarker" shall be interpreted broadly to a substance expressed, produced, or associated with a cell that distinguishes the cell from other cells in a mixture of cells such as tissues, organs, fluids, biological fluids, *etc.* The cell associated with a biomarker may distinguish cells that differ in growth state, cell lineage, stage of differentiation or de-differentiation, pathologic state (such as pre-cancerous, cancerous, neoplastic, hyperproliferative, or infected cells). A biomarker may, for example, distinguish endomertial cells that are abberently localized in non-uterine tissue or identify precancerous cells within a normal tissue. A biomarker may include, but is not limited to proteins, nucleic acids, lipids, carbohydrates, cellular organelles, receptors, cell surface proteins, transporters, antigen presenting complexes, and other molecules that are unique or over represented in certain cell types or growth states. As used herein, "molecular probe" shall be interpreted broadly to mean a molecule that preferentially binds a biomarker. A molecular probe includes, but is not limited to a proteins, polypeptides, peptides, peptide mimetics, nucleic acids, pepto nucleic acids (PNAs), antibodies, aptamers, small molecules (folic acid or mimics thereof), growth factors, lipids, lipoproteins, glycoproteins, cabohydrates, *etc.*

Other features and associated advantages will become apparent with reference to the following detailed description of specific embodiments in connection with the accompanying drawings.

### Brief Description of the Drawings

The following drawings form part of the present specification and are included to further demonstrate certain aspects of the present invention.
**FIG. 1** is a schematic diagram of *in vitro* selection of aptamers.
**FIG. 2** illustrates qdots attached to neuron using a site specific antibody. Brightfield (left) and fluorescence (right) images are shown. The bar represents 60 µm.
**FIG. 3** is a schematic diagram showing the integration of various aspects of the present disclosure.
**FIG. 4** is a UV-Vis spectra of isolated (a) and aggregated (b) metal nanoparticles.
**FIG. 5** illustrates a biotinylated bead labeled with streptavidin/particles conjugates.
**FIG. 6** illustrates the scattering of beads with high (left) and low (right) density of metal nanoparticles.
**FIG. 7** illustrates silica coated nanoparticles.
**FIG. 8** is a schematic diagram showing the preparation of conjugates of nanoparticles with antibodies and aptamers.
**FIG. 9** illustrates size-dependent luminescence of Si nanocrystals.
**FIG.10** shows scattering properties of gold nanoparticles.
**FIG. 11** shows optical images of SiHa cells labeled with anti-EGFR/gold conjugates.
**FIG. 12** shows laser scanning confocal reflectance and confocal fluorescence images of pre-cancerous and normal fresh cervical ex *vivo* tissue labeled with anti-EGFR/gold conjugates.
**FIG. 13** shows transmittance and reflectance images of engineered tissue constructs labeled with anti-EGFR/gold conjugates.
**FIG. 14** shows confocal reflectance (FIGS. 14A and 14C) and fluorescence (FIGS. 14B and 14D) images of SiHa cells on collagen I labeled with anti-MMP-9 /gold conjugates. The area in the white square in (A) is shown in more detail in (C). Arrows show polarized cells.
**FIG. 15** shows co-localized fluorescence and reflectance laser scanning confocal microscopic images obtained from SiHa cells incubated in anti-E7 gold nanoparticle conjugates with 10% PVP. Autofluorescence due to NAD(P)H is observed in the cytoplasm, while strong backscattering due to contrast agents is seen in the nucleus.

### Description of Illustrative Embodiments

Sensing cancer specific biomolecular signatures, or other specific biomolecular signatures, may significantly improve screening, diagnosis and prognosis, assist in design of treatment, and facilitate monitoring of disease. Currently, biomolecular signatures - such as cancer biomarkers - can only be assessed through invasive, painful biopsy. In this disclosure, techniques are divulged that combine the advantages of real-time, *in vivo* optical imaging with innovative, molecular specific contrast agents to provide a unique opportunity for highly selective and sensitive detection of, for instance, cancer related biomarkers in *vivo.*

Optically interrogated contrast agents may be based on metal nanocrystals and quantum dots attached to probe molecules with a high affinity to a specific biomarker on the surface of epithelial cancer cells. Optical interrogation may provide non-invasive real time assessment of tissue pathology, while contrast agents give molecular specificity and selectivity. The combination of optical imaging techniques with cancer specific contrast agents may increase optical contrast between normal and neoplastic tissue and provide useful molecular-specific information to assist clinicians in earlier detection of pre-cancers. Accordingly, the techniques disclosed herein may significantly improve the specificity and selectivity of the detection of conditions such as pre-cancer.

Because aspects of this disclosure involve optical methods, it should be noted that optical methods may be limited by relatively small penetration depth of light inside a turbid human tissue (about 1.5 mm); therefore, certain aspects of this disclosure may be better suited for application to epithelial tissue. The majority of cancers are of epithelial origin; hence, certain embodiments described herein find direct applicability to situations involving cancer. As is understood by those having skill in the art, however, there are several techniques that can be applied to increase penetration depth of light inside of tissue including but not limited to U.S. Patent No. 6,275,726.

### Optical Detection of Neoplasia

Optical technologies offer the ability to image tissue with unprecedented spatial and temporal resolution using low cost, portable devices; thus, they represent an ideal approach to image early neoplasia. Multiple *in vivo* optical imaging and spectroscopic modalities, including multi-spectral fluorescence imaging, [5,6] multi-spectral reflectance imaging with unpolarized [7] and polarized [8] light, confocal microscopy [9] and reflectance [10-14] and fluorescence [15-20] spectroscopy, have recently been explored as diagnostic tools in medicine. In the UV and visible regions of the spectrum, tissue reflectance spectra provide information about the wavelength dependent scattering of tissue as well as electronic absorption bands, primarily those of oxy- and deoxy hemoglobin. The most common naturally occurring fluorophores include the aromatic amino acids, the co-factors NAD(P)H and FAD, which describe the tissue metabolic rate, crosslinks associated with collagen and elastin, and porphyrins.

Different research has led to optical techniques to identify certain premalignant changes in the female genital tract. For example, optical techniques have been developed to address limitations of the Pap smear and colposcopy, the follow up test performed when a Pap smear is abnormal. [21-24]. Performance of some of these algorithms exceed that of the Pap smear and are comparable to colposcopy.

Another optical approach is *in vivo* confocal imaging, which provides the ability to non-invasively image epithelial cells using reflected light. In concept, this is similar to histologic analysis of biopsies, except that 3D resolution may be achieved without removing tissue, and contrast is provided without stains. Confocal images can localize reflected light in 3D with enough resolution to image individual cells and intra-cellular structure. Changes in refractive index provide contrast to sample intra-cellular detail; in epithelium, contrast is provided primarily by fluctuations in the nuclear refractive index related to chromatin texture. Backscattering can be enhanced dramatically with simple contrast agents.

Exemplary optical detection methods are discussed in US Patents Nos. 5,562,100; 5,612,540; 5,623,932; 5,697,373; 5,699,795; 5,842,995; 5,920,399; 5.929,985; 5,991,653; 6,095,982; 6,135,965; 6,187,289; 6,241,662 and 6,258,576.

### Non-specific Contrast Agents for Optical Imaging

Despite the tremendous potential of optical techniques for clinical applications, they still are limited by low intrinsic contrast between normal and diseased tissues, especially at the earliest stages of pre-cancer development. Therefore, many optical imaging techniques rely on the addition of exogenous agents to enhance intrinsic contrast. Acetic acid is commonly used during colposcopy to enhance contrast between normal and diseased regions in the cervix. [25]. Hypertonic saline may also be used during colposcopy for increased visualization. [26]. Both of these agents result in changes in the refractive index of the cell, making them potentially useful contrast agents for confocal reflectance imaging.

### Cancer Related Biomolecular Signatures - Biomarkers

Although optical imaging techniques can be used to analyze tissue pathology *in situ* in real time, they currently do not provide information about specific biomolecular signatures associated with cancer development. These biomolecular signatures or biomarkers can currently be assessed only through invasive biopsy and the use of quantitative immunochemical analyses *in vitro.* Researchers have worked extensively to assess cervical biomarkers, both for use in screening and diagnosis and in chemoprevention trials. [27,28]. A number of biomarkers of cancer progression have been identified in the cervix, including quantitative histology and cytology, PCNA, MIB-1, MPM-2, HPV viral load, EGFR, polyamines, and ploidy. [27].

Cervical biomarkers can be divided into several categories: cyto- and histologic markers, markers indicating altered proliferation, regulation, differentiation, and genomic instability. Cytologic and histopathologic markers include nuclear features, nucleolar features, and tissue architecture. [29,30]. Nuclear features of interest include grade, shape, area, optical density, texture, nuclear pleomorphism, and ploidy (as estimated by DNA content). Tissue architectural measurements exploit the finding that disordered nuclei are crowded and irregular. One rationale for the use of proliferation markers is that cells with high proliferative activity are more likely to be associated with premalignant and malignant tissues. [31,32]. Proliferation can be studied with Ki-67 in frozen sections and MIB-1 (an antibody to Ki-67) and proliferating cell nuclear antigen (PCNA) in archival specimens.

Regulation markers include tumor suppressors, HPV viral load and oncoproteins, oncogenes, growth factors and their receptors, polyamines, and arachidonic acid. These agents in their normal states help regulate cell growth. Their measurement may provide clues to the process of carcinogenesis. HPV can be quantitatively measured using PCR quantification of HPV 16 and 18 E7 mRNAs. [33]. At present, the measurement of mRNA is labor-intensive and requires sophisticated laboratory experience. Several types of selected protein kinases and their receptors have been identified to be important in the development of cancer. The tyrosine kinase subfamily includes epidermal growth factor receptor (EGFR), vascular endothelial growth factor (VEGF), platelet-derived growth factor (PDGF), Src, Lck, and others. Vascular atypia is the hallmark of colposcopic progression of CIN to cancer. Vascular growth factors have an important biologic role. Fujimoto *et al* [34] studied VEGF in normal cervix and all cell types of invasive cervical cancer. They observed increases in VEGF, which correlated with microvessel counts in cancers.

Differentiation markers include fibrilar proteins (keratins, involucrin, comifin), adhesion molecules (cell-cell: lectins, gap junction, desmosomes; cell-substrate: integrins, cadherins, laminins, fibronectin, proteoglycans, collagen), and glycoconjugates (mucins, blood group substances, and glycolipids).

### Molecular Probes of Biomarkers

### Immunohistopathology

Significant benefits of quantitative assessment and monitoring of cancer related biomarkers have stimulated development of probe molecules to selectively target biomarkers on tissue slices. For example, antibodies to epidermal growth factor (EGFR) are commercially available (Bigenex, CA). Immunostaning procedure to specifically target the antibodies to their cellular targets in cell and biopsy specimens are routinely used in cytology and histopathology. [35].

### Drug delivery

Selective delivery of therapeutic agents to cancer cells in a living body is another area of research where targeting of cancer specific biomarkers is intensively studied. [36-38]. Immunoliposome-mediated targeting using monoclonal antibodies to folate receptor, [36,37] CA-125, [36] and HER2/neu antigen [39] have been described. Problems associated with targeting delivery *in vivo* are thoroughly addressed in these studies.

### Aptamers

Traditionally, the identification of biomarkers and development of antibodies for their specific targeting has been a difficult and time-consuming process that does not always provide the best result for a particular application. For example, although many cancer related biomarkers have been identified, only few of them have shown promising results for cancer screening and prognosis, and it has been recognized that it may be true that only combinations of these biomarkers can provide the best discrimination between cancerous and normal tissue.

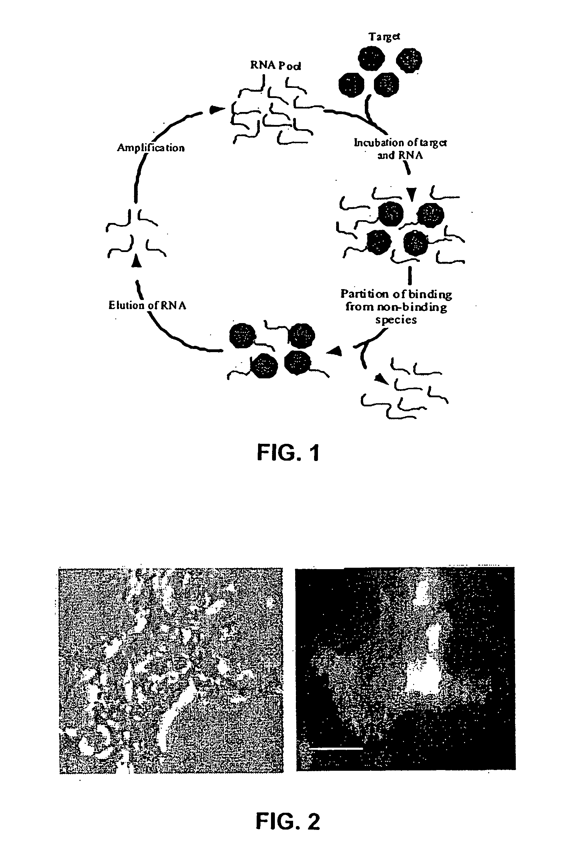

Numerous reviews have been written about the practice and products of *in vitro* selection of aptamers. [40-43]. FIG. 1 summarizes some relevant procedures that may be used in carrying out embodiments of the present disclosure.

The methods may utilize aptamers with unique or improved binding characteristics to a target that is unique to or over represented (as compared to a normal or non-target cell) in, around or on a cell of interest. An "aptamer" as used herein refers to a nucleic acid that binds a target molecule through interactions or conformations other than those of nucleic acid annealing/hybridization described herein. Methods for making and modifying aptamers, and assaying the binding of an aptamer to a target molecule may be assayed or screened for by any mechanism known to those of skill in the art (see for example, U.S. Patent Nos. 6,111,095, 5,861,501, 5,840,867, 5,792,613, 5,780,610, 5,780,449, 5,756,291 5,631,146 and 5,582,981; as well as PCT Publication Nos. WO92/14843, WO91/19813, and WO92/05285.

Aptamers are single- or double-stranded DNA or single-stranded RNA molecules that recognize and bind to a desired target molecule by virtue of their shapes. See, e.g., PCT Publication Nos. WO92/14843, WO91/19813, and WO92/05285. The SELEX procedure, described in U.S. Pat. No. 5,270,163 to Gold et al., Tuerk et al. (1990) Science 249:505-510, Szostak et al. (1990) Nature 346:818-822 and Joyce (1989) Gene 82:83-87, can be used to select for RNA or DNA aptamers that are target-specific. In the SELEX procedure, an oligonucleotide is constructed wherein an n-mer, preferably a random sequence tract of nucleotides thereby forming a "randomer pool" of oligonucleotides, is flanked by two polymerase chain reaction (PCR) primers. The construct is then contacted with a target molecule under conditions which favor binding of the oligonucleotides to the target molecule. Those oligonucleotides which bind the target molecule are: (a) separated from those oligonucleotides which do not bind the target molecule using conventional methods such as filtration, centrifugation, chromatography, or the like; (b) dissociated from the target molecule; and (c) amplified using conventional PCR technology to form a ligand-enriched pool of oligonucleotides. Further rounds of binding, separation, dissociation and amplification are performed until an aptamer with the desired binding affinity, specificity or both is achieved. The final aptamer sequence identified can then be prepared chemically or by in vitro transcription.

The length of a random sequence tract can range from 20 to over 150 residues, and can be even longer if multiple, random oligonucleotides are combined into a single pool by ligation or other methods. [44]. The number of individuals in a random sequence population is typically at least 10¹³ and can easily be over 10¹⁵. For most pools, this means that upwards of all possible 25-mers are present, and a proportionately smaller number of motifs longer than 25. Because of the redundancy of biological sequences, the sequence diversity of most random sequence pools likely rivals the sequence diversity of the Earth's biosphere.

Aptamers have been selected against a surprising range of targets, ranging from ions to small organics to peptides to proteins to supramolecular structures such as viruses and tissues. [42,45-48]. In particular, aptamers have been selected against a wide variety of proteins, including many nucleic acid binding proteins, such as T4 DNA polymerase [49] and HIV-1 Rev, [50] and multiple non-nucleic acid binding proteins. In general, anti-protein aptamers seem to recognize basic patches on protein surfaces. For example, the arginine-rich motifs (ARMs) of many viral proteins are recognized by aptamers (reviewed in [51]), the phosphate-binding pockets of both kinases [52] and phosphatases, [53] and the heparin-binding sites on many surface proteins and cytokines, such as basic fibroblast growth factor [54,55] and vascular endothelial growth factor. [56,57].

Aptamers also seem to have an affinity for pockets or cusps on protein surfaces, such as the combining sites of antibodies [58] or the active sites of enzymes. [59]. Almost all proteins have either surface pockets or basic patches (indeed, even proteins with negative pI's, such as T4 DNA polymerase, typically contain sites that can elicit aptamers). Most aptamer:target complexes have dissociation constants in the nanomolar range. Moreover, aptamers recognize their targets with high specificity, and can typically discriminate between protein targets that are highly homologous or differ by only a few amino acids. [52,60,61].

### Biophotonic Probes or Labels

Advances in nano-materials provide a wealth of optically-interrogatable markers to explore for in *vivo* detection. The invention based on metal nanoparticles that can be interrogated using optical reflectance. They may be linked to aptamer-based, antibody-based, or peptide-based probe molecules, as well as other small molecules that are known to preferentially bind to proliferating cells or tissues, to provide selective labeling of, for instance, pre-cancerous, cancerous, neoplastic, or hyperproliferative cervical epithelial cells.

### Metal Nanoparticles

Colloidal gold and silver nanoparticles exhibit beautiful and intense colors in the visible spectral region. Without being bound by theory, it is believed that these colors are the result of excitation of surface plasmon resonances in the metal particles and are extremely sensitive to particles' sizes, shapes, and aggregation state; dielectric properties of the surrounding medium; adsorption of ions on the surface of the particles; etc. [62].

The excitation of plasmon resonances leads to enhancement of the local electromagnetic field near the surface of the particles. [63]. This effect may serve as the basis for enhancement of many optical phenomena including but not limited to Raman scattering, fluorescence intensity, and photochemistry in close vicinity to the metal surface. Harnessing the unique properties of the surface plasmon resonances has led to development of a variety of applications in biology and bioanalytical chemistry. Surface enhanced Raman scattering (SERS) spectroscopy has been used to solve a number of unique biologically relevant problems [64,65] and was demonstrated to be capable of providing highly resolved vibrational information at the level of a single cell [65,66] and a single molecule. [67]. Surface enhanced fluorescence (SEF) spectroscopy has shown a great potential for development of sensitive bioanalytical procedures with reduced number of intermediate processing steps. [68].

In a new highly selective colorimetric DNA probe technique based on reversible assembly of oligonucleotide-capped gold colloid, a detection limit of about 10 femtomoles and sensitivity to a single base pair mismatch were achieved [69]. That method exploited changes in plasmon resonances of gold particles upon their aggregation. In those experiments, gold nanoparticles were conjugated with mercaptoalkyloligonucleotide probe molecules and were mixed with single stranded target oligonucleotides. The interactions between the target molecules and the conjugated nanoparticles brought the nanoparticles in close vicinity, inducing a dramatic red-to-blue macroscopic color change. Because of the strong optical absorption of gold particles, the proposed assay was about 50 times more sensitive than standard hybridization detection methods based on fluorescence detection.

Recently gold nanoshell-polymer composites were proposed as a candidate for photothermally triggered drug delivery system [70]. The nanoshells consisted of a dielectric (gold sulfide or silica) core and a gold shell. [71]. The optical resonances of that material can be shifted from the visible to the near infrared region by changing the relative thickness of the core and the shell layers. When the gold nanoshells are embedded inside a polymeric matrix, their illumination at wavelengths of gold plasmon resonances results in heat transfer to the local environment. This photothermal effect may be used to optically induce drug release in an implanted nanoshell-polymer composite drug delivery material [70].

Besides certain specific optically based applications, gold nanoparticles have been extensively used as molecular specific stains in electron microscopy of cells and tissues. [72,73]. For example, [465] describes the use of colloidal gold conjugated to a specific monoclonal antibody for binding to surface EGFRs to characterize the early receptor trafficking events leading to enhanced EGFR degradation in adenovirus infected cells. In this field, the fundamental principle of interactions between the gold particles and biomolecules, especially proteins, have been thoroughly studied. As a result, well established protocols have been developed for the labeling of a broad range of biomolecules with colloidal gold, including protein A, avidin, streptavidin, glucose oxidase, horseradish peroxidase, and IgG (antibodies).

Among all the fascinating properties of metal nanoparticles, the ability to resonantly scatter light at frequencies coinciding with the particles' surface plasmon resonances has, until now, yet to be explored or fully exploited for biological applications. The techniques of this disclosure, however, may use this property in the development of contrast agents for *in vivo* reflectance. In this disclosure, there are described innovative detection schemes that may allow users to fully harness these advantages for highly selective detection of, for instance, cancer related biomolecular signatures.

According to this disclosure, one may prepare closely spaced assemblies of nanocrystals through self-assembly and laser photochemistry and/or photolithography. One may conduct chemical modification of prepared assemblies and consequent immobilization of mono- and multilayers of bioorganic molecules on their surface (DNA, antibodies, avidin, phospholipids, etc). [68,74]. Metal nanocrystals, or other highly reflective nanocrystals, may be prepared with tailored optical properties. The nanocrystals may be characterized, modified, and conjugated with organic and bio-molecules. The prepared nanostructures may be applied for structure-functional characterization of complex biological samples such as proteins, synthetic bioactive polymers, nucleic acids, and single living cells using SERS and SEF spectroscopies. [65,68,74-79].

### Antibodies

It will be understood that polyclonal or monoclonal antibodies specific for a molecule that is expressed or over-expressed in a cell, tissue, or organ targeted for imaging, such as pre-cancerous, cancerous, neoplastic, or hyperproliferative cells; tissues; or organs, may be used in the practice of the described disclosure. Embodiments of this disclosure may include the *in vivo* or *in vitro* imaging, detection, or diagnosis of pre-cancerous, cancerous, neoplastic or hyperproliferative cells in a tissue or organ. The compositions and methods described herein may be used or provided in diagnostic kits for use in detecting and diagnosing cancer.

Thus, antibodies specific for proteins, polypeptides, peptides, lipids, carbohydrates, lipoproteins, or other molecules that are unique to or over represented in, on, or around pre-cancerous, cancerous, neoplastic or hyperproliferative cells in tissue or organ may be utilized. Means for preparing and characterizing antibodies are well known in the art (See, e.g., Antibodies: A Laboratory Manual, Cold Spring Harbor Laboratory, 1988). Antibodies used to detect, diagnose, identify or monitor a pre-cancerous, cancerous, neoplastic or hyperproliferative cells in a tissue or organ, as well precursors or derivatives of such cells may be generated using such standard techniques.

### Polyclonal Antibodies

Polyclonal antibodies to an antigen generally are raised in animals by multiple subcutaneous (sc) or intraperitoneal (ip) injections of the antigen and an adjuvant. It may be useful to conjugate the antigen or a fragment containing the target amino acid sequence or target molecule to a protein that is immunogenic in the species to be immunized, e.g. keyhole limpet hemocyanin, serum albumin, bovine thyroglobulin, or soybean trypsin inhibitor using a bifunctional or derivatizing agent, for example maleimidobenzoyl sulfosuccinimide ester (conjugation through cysteine residues), N-hydroxysuccinimide (through lysine residues), glytaraldehyde, succinic anhydride, SOCl₂, or R₁ NCNR, where R and R₁ are different alkyl groups.

Animals are immunized against the immunogenic conjugates or derivatives by combining 1 mg or 1 µg of conjugate (for rabbits or mice, respectively) with 3 volumes of Freud's complete adjuvant and injecting the solution intradermally at multiple sites. One month later the animals are boosted with 1/5 to 1/10 the original amount of conjugate in Freud's complete adjuvant by subcutaneous injection at multiple sites. 7 to 14 days later the animals are bled and the serum is assayed for antibody titer. Animals are boosted until the titer plateaus. An animal boosted with the conjugate of the same antigen, but conjugated to a different protein and/or through a different cross-linking reagent. Conjugates also can be made in recombinant cell culture as protein fusions. Also, aggregating agents such as alum are used to enhance the immune response.

### Monoclonal Antibodies

Monoclonal antibodies are obtained from a population of substantially homogeneous antibodies, i.e., the individual antibodies comprising the population are identical except for possible naturally occurring mutations that may be present in minor amounts. Thus, the modifier "monoclonal" indicates the character of the antibody as not being a mixture of discrete antibodies.

For example, the monoclonal antibodies may be made using the hybridoma method first described by Kohler & Milstein, Nature 256:495 (1975), or may be made by recombinant DNA methods (Cabilly, et al., U.S. Pat. No. 4,816,567).

In the hybridoma method, a mouse or other appropriate host animal, such as hamster is immunized as herein above described to elicit lymphocytes that produce or are capable of producing antibodies that will specifically bind to the antigen used for immunization. Alternatively, lymphocytes may be immunized *in vitro.* Lymphocytes then are fused with myeloma cells using a suitable fusing agent, such as polyethylene glycol, to form a hybridoma cell (Goding, Monoclonal Antibodies: Principles and Practice, pp.59-103 (Academic Press, 1986)).

DNA encoding a monoclonal antibody may be readily isolated and sequenced using conventional procedures (e.g., by using oligonucleotide probes that are capable of binding specifically to genes encoding the heavy and light chains of murine antibodies). The hybridoma cells serve as a preferred source of such DNA. Once isolated, the DNA may be placed into expression vectors, which are then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also may be modified, for example, by substituting the coding sequence for human heavy and light chain constant domains in place of the homologous murine sequences, Morrison, et al., Proc. Nat. Acad. Sci. 81, 6851 (1984), or by covalently joining to the immunoglobulin coding sequence all or part of the coding sequence for a non-immunoglobulin polypeptide. In that manner, "chimeric" or "hybrid" antibodies are prepared that have the binding specificity of an anti-cancer, pre-cancer, or hyperproliferative cell monoclonal antibody herein.

Typically such non-immunoglobulin polypeptides are substituted for the constant domains of an antibody or they are substituted for the variable domains of one antigen-combining site of an antibody to create a chimeric bivalent antibody comprising one antigen-combining site having specificity for a first antigen and another antigen-combining site having specificity for a different antigen.

Chimeric or hybrid antibodies also may be prepared *in vitro* using known methods in synthetic protein chemistry, including those involving crosslinking agents. For example, immunotoxins may be constructed using a disulfide exchange reaction or by forming a thioether bond. Examples of suitable reagents for this purpose include iminothiolate and methyl-4-mercaptobutyrimidate.

For diagnostic applications, the antibodies typically will be labeled with a detectable moiety (optically interrogated moiety). The detectable moiety can be any one which is capable of producing, either directly or indirectly, a detectable signal when optically interrogated. In particular, the detectable moiety may be an optical contrast agent, such as a metal or semiconductor nanoparticle. For example, the detectable moiety may be a gold, silver, composite, silicon nanoparticle.

Any method known in the art for separately conjugating the antibody to the detectable moiety may be employed, including those methods described by Hunter, et al., Nature 144:945 (1962); David, et al., Biochemistry 13:1014 (1974); Pain, et al., J. Immunol. Meth. 40:219 (1981); and Nygren, J. Histochem. and Cytochem. 30:407 (1982).

### Humanized Antibodies

Methods for humanizing non-human antibodies are well known in the art. Generally, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers (Jones et al., Nature 321, 522-525 (1986); Riechmann et al., Nature 332, 323-327 (1988); Verhoeyen et al., Science 239, 1534-1536 (1988)), by substituting rodent CDRs or CDR sequences for the corresponding sequences of a human antibody. Accordingly, such "humanized" antibodies are chimeric antibodies (Cabilly, supra), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

It is important that antibodies be humanized with retention of high affinity for the antigen and other favorable biological properties. To achieve this goal humanized antibodies are prepared by a process of analysis of the parental sequences and various conceptual humanized products using three dimensional models of the parental and humanized sequences. Three dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, i.e. the analysis of residues that influence the ability of the candidate immunoglobulin to bind its antigen. In this way, FR residues can be selected and combined from the consensus and import sequence so that the desired antibody characteristic, such as increased affinity for the target antigen(s), is achieved. In general, the CDR residues are directly and most substantially involved in influencing antigen binding.

### Human Antibodies

Human monoclonal antibodies can be made by the hybridoma method. Human myeloma and mouse-human heteromyeloma cell lines for the production of human monoclonal antibodies have been described, for example, by Kozbor, J. Immunol. 133, 3001 (1984), and Brodeur, et al., Monoclonal Antibody Production Techniques and Applications, pp.51-63 (Marcel Dekker, Inc., New York, 1987).

It is now possible to produce transgenic animals (e.g. mice) that are capable, upon immunization, of producing a repertoire of human antibodies in the absence of endogenous immunoglobulin production. For example, it has been described that the homozygous deletion of the antibody heavy chain joining region (J_{H}) gene in chimeric and germ-line mutant mice results in complete inhibition of endogenous antibody production. Transfer of the human germ-line immunoglobulin gene array in such germ-line mutant mice will result in the production of human antibodies upon antigen challenge. See, e.g. Jakobovits et al., Proc. Natl. Acad. Sci. USA 90, 2551-255 (1993); Jakobovits et al., Nature 362, 255-258 (1993).

Alternatively, the phage display technology (McCafferty et al., Nature 348, 552-553 (1990)) can be used to produce human antibodies and antibody fragments *in vitro,* from immunoglobulin variable (V) domain gene repertoires from unimmunized donors. According to this technique, antibody V domain genes are cloned in-frame into either a major or minor coat protein gene of a filamentous bacteriophage, such as M 13 or fd, and displayed as functional antibody fragments on the surface of the phage particle.

Because the filamentous particle contains a single-stranded DNA copy of the phage genome, selections based on the functional properties of the antibody also result in selection of the gene encoding the antibody exhibiting those properties. Thus, the phage mimics some of the properties of the B-cell. Phage display can be performed in a variety of formats; for their review see, e.g. Johnson, Kevin S. and Chiswell, David J., Current Opinion in Structural Biology 3, 564-571 (1993). Several sources of V-gene segments can be used for phage display. Clackson et al., Nature 352, 624-628 (1991) isolated a diverse array of anti-oxazolone antibodies from a small random combinatorial library of V genes derived from the spleens of immunized mice. A repertoire of V genes from unimmunized human donors can be constructed and antibodies to a diverse array of antigens (including self-antigens) can be isolated essentially following the techniques described by Marks et al., J. Mol. Biol. 222, 581-597 (1991), or Griffith et al., EMBO J. 12, 725-734 (1993). In a natural immune response, antibody genes accumulate mutations at a high rate (somatic hypermutation). Some of the changes introduced will confer higher affinity, and B cells displaying high-affinity surface immunoglobulin are preferentially replicated and differentiated during subsequent antigen challenge. This natural process can be mimicked by employing the technique known as "chain shuffling" (Marks et al., Bio/Technol. 10, 779-783 [1992]). In this method, the affinity of "primary" human antibodies obtained by phage display can be improved by sequentially replacing the heavy and light chain V region genes with repertoires of naturally occurring variants (repertoires) of V domain genes obtained from unimmunized donors. This technique allows the production of antibodies and antibody fragments with affinities in the nM range. A strategy for making very large phage antibody repertoires (also known as "the mother-of-all libraries") has been described by Waterhouse et al., Nucl. Acids Res. 21, 2265-2266 (1993), and the isolation of a high affinity human antibody directly from such large phage library is reported by Griffith et al., EMBO J. (1994), in press. Gene shuffling can also be used to derive human antibodies from rodent antibodies, where the human antibody has similar affinities and specificities to the starting rodent antibody. According to this method, which is also referred to as "epitope imprinting", the heavy or light chain V domain gene of rodent antibodies obtained by phage display technique is replaced with a repertoire of human V domain genes, creating rodent-human chimeras. Selection on antigen results in isolation of human variable capable of restoring a functional antigen-binding site, i.e. the epitope governs (imprints) the choice of partner. When the process is repeated in order to replace the remaining rodent V domain, a human antibody is obtained (see PCT patent application WO 93/06213, published Apr. 1, 1993). Unlike traditional humanization of rodent antibodies by CDR grafting, this technique provides completely human antibodies, which have no framework or CDR residues of rodent origin.

### Bispecific Antibodies

Bispecific antibodies are monoclonal, preferably human or humanized, antibodies that have binding specificities for at least two different antigens. One of the binding specificities is for a first antigen and the other one is for a second antigen.

Traditionally, the recombinant production of bispecific antibodies is based on the coexpression of two immunoglobulin heavy chain-light chain pairs, where the two heavy chains have different specificities (Millstein and Cuello, Nature 305, 537-539 (1983)). Because of the random assortment of immunoglobulin heavy and light chains, these hybridomas (quadromas) produce a potential mixture of 10 different antibody molecules, of which only one has the correct bispecific structure. The purification of the correct molecule, which is usually done by affinity chromatography steps, is rather cumbersome, and the product yields are low. Similar procedures are disclosed in PCT application publication No. WO 93/08829 (published May 13, 1993), and in Traunecker et al., EMBO 10, 3655-3659 (1991).

For further details of generating bispecific antibodies see, for example, Suresh et al., Methods in Enzymology 121, 210 (1986).

### Heteroconjugate Antibodies

Heteroconjugate antibodies are composed of two covalently joined antibodies. Such antibodies have, for example, been proposed to target immune system cells to unwanted cells (U.S. Pat. No. 4,676,980), and for treatment of HIV infection (PCT application publication Nos. WO 91/00360 and WO 92/200373; EP 03089). Heteroconjugate antibodies may be made using any convenient cross-linking methods. Suitable cross-linking agents are well known in the art, and are disclosed in U.S. Pat. No. 4,676,980, along with a number of cross-linking techniques.

### Antibody Conjugates

Antibody conjugates in which an antibody that preferentially or specifically binds pre-cancerous, cancerous, neoplastic, or hyperproliferative cell(s) is linked to a detectable labeling or contrast agent may also be used. Diagnostic antibody conjugates may be used both *in vitro* diagnostics, as in a variety of immunoassays, and *in vivo* diagnostics, such as in imaging technology as described herein. Certain antibody conjugates include those intended primarily for use *in vivo,* where the antibody is linked to a optically interrogated agent.

The covalent binding can be achieved either by direct condensation of existing side chains or by the incorporation of external bridging molecules. Many bivalent or polyvalent agents are useful in coupling protein molecules to other particles, nanoparticles, proteins, peptides or amine functions. Examples of coupling agents are carbodiimides, diisocyanates, glutaraldehyde, diazobenzenes, and hexamethylene diamines. This list is not intended to be exhaustive of the various coupling agents known in the art but, rather, is exemplary of the more common coupling agents that may be used.

It is contemplated that one may wish to first derivatize the antibody, and then attach the contrast agent to the derivatized product. As used herein, the term "derivatize" is used to describe the chemical modification of the antibody substrate with a suitable cross-linking agent. Examples of cross-linking agents for use in this manner include the disulfide-bond containing linkers SPDP (N-succinimidyl-3-(2-pyridyldithio)propionate) and SMPT (4-succinimidyl-oxycarbonyl-α-methyl-α(2-pyridyldithio)toluene).

The following specific steps are disclosed
(1) A library of probe molecules for cancer specific targets associated with pre-cancer/cancer cells may be created and characterized, using molecular engineering and combinatorial chemistry approaches.
(2) Contrast agents may be made based on metal nanoparticles for in *vivo* reflectance imaging. The agents may include two major parts: optically interrogated labels - metal nanoparticles - and probe molecules specific for cancer biomarkers. The optical properties of these labels may be synthesized and tailored, and conjugation chemistry may be used to couple labels and probe molecules.
(3) Contrast agents may be made based on quantum dots for *in vivo* fluorescence imaging. These agents may contain quantum dot particles as optically interrogated labels and probe molecules specific for cancer biomarkers. The optical properties of these labels may be synthesized and tailored, and conjugates may be made with probe molecules.
(4) If validation of the techniques of this disclosure is desired, molecular specific contrast agents for pre-cancer detection may be validated in at least two biological models. Suspensions of normal, pre-cancerous, and cancerous cervical epithelial cells may be used to assess relative binding efficiencies of contrast agents. Additionally, using three-dimensional, tissue phantoms containing multiple layers of epithelial cells atop a stroma, marker penetration and binding in model systems of normal, pre-cancerous and cancerous epithelial tissue may be examined.
(5) If specific testing of the techniques of this disclosure is desired, the contrast agents and optical imaging techniques may be tested in living normal and neoplastic cervical tissue. An ideal organ culture system of normal and pre-cancerous cervix may be used. Biopsies of normal and neoplastic cervix may be obtained, and transverse sections may immediately be prepared and maintained as an organ culture. Both types of contrast agents may be applied and interrogated to determine relative binding efficiency and penetration throughout the epithelium in living human cervical tissue.

The activities listed above and throughout this disclosure provide an example of a new venue for molecular-specific optical imaging modalities for disease detection that can be extended to many organ sites, including but not limited to the cervix.

### Methods

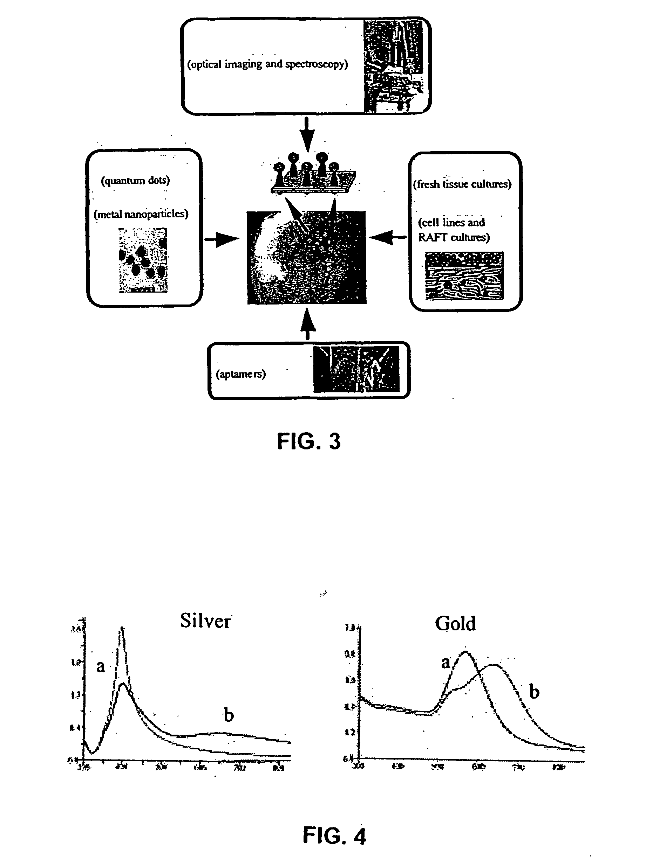

FIG. 3 illustrates the integration of interdisciplinary groups to make photonic probes and contrast agents for highly sensitive and selective detection of, for instance, pre-cancers *in vivo*. The approaches of combinatorial chemistry may be used to make a library of aptamer molecules specific for biomolecular targets on the surface of cervical cancerous and pre-cancerous cells. Aptamers exhibiting antiproliferative and antiangiogenic activity may be used. Well-established cervical cell lines at different stages of cancer development may be used. Photonic probes based on quantum dots and metal nanoparticles may be made. They may utilize custom-made aptamers or existing antibodies for well-known cancer biomarkers currently used in clinical histopathology. The developed conjugates may be used as molecular specific contrast agents using optical microscopy and spectroscopy. The cervical cancer cell lines, three-dimensional tissue phantoms, and fresh cervical tissue slices may all be used for imaging, testing, and/or validation. Experiments with all three biological systems representing properties of normal and neoplastic cervix at different levels of complexity may be used, if necessary, to assess and refine the performance and detection scheme for the contrast agents. This refinement may include preparing bio-engineered aptamers with high affinity to cancer specific targets, tailoring optical properties of metal nanoparticles and quantum dots, optimizing conjugation procedures, and/or generating optimal imaging geometries.

The following sections describe even further details associated with four major components of this disclosure.

### Creation of Library of Aptamers Specific for Pre-Cancerous and Cancerous Cells

Recent advancement in combinatorial chemistry provide an excellent tool for rapid screening of huge populations of biomolecules to find molecules with the best binding properties and selectivity to a specific target including whole cells. One may use chemically engineered binding species based on short nucleic acid sequences (aptamers) to create molecules with improved selectivity to pre-cancerous and cancerous cells as compared to the existing antibodies.

The same methods that have been used to select aptamers that bind tightly and specifically to protein targets may be used to select aptamers that bind to, for instance, tumor markers on the surfaces of cells. In fact, aptamers have previously been selected against cellular and organismal targets. For example, it has proven possible to use human red blood cell membranes as a target for the selection of single-stranded DNA aptamers. Several species of ssDNA were isolated that recognize distinct targets within the membranes. [89]. In addition, aptamers have been selected against whole African trypanosomes. Three classes of RNA were selected that bind with high affinity to a protein within the flagellar pocket of the parasite. [90].

Oligonucleotides may be generated that contain a random sequence core that spans 60 residues and flanking regions that allow PCR amplification and *in vitro* transcription. Following amplification of the nascent DNA library to generate double-stranded transcription templates, RNA molecules may be transcribed that contain 2' fluorinated pyrimidines. The presence of 2' modified residues has been shown to substantially stabilize nucleic acids against endogenous nucleases or other perturbants. For example, RNA molecules containing 2' modified pyrimidines have previously been stable for days in sera and urine. [91]. RNA libraries may be gel-purified and directly used for selection. Roughly 100 micrograms (ca. 10¹⁵ different sequences) may be applied to each of the target cell lines. Following the equilibration of binding species on the cell surfaces, non-binding or weakly binding species may be washed off using PBS. Binding species may be eluted by homogenizing the cells with detergent. While this procedure of course releases cellular nucleic acids, many of those may be destroyed by endogenous nucleases, may not be amplifiable with particular primer sets, and even if they are amplifiable may not be of the same size as the nucleic acid pool. Those aptamers in the extract may be directly amplified by reverse transcription and the polymerase chain reaction. Products of the correct size may be gel-isolated and used to transcribe the sieved RNA population for the next round of selection. In general, radiolabel (alpha-32P ATP) may be included in the transcription reaction, and the fraction of radioactive RNA that binds to a given cell line may be followed by scintillation counting. A relatively small fraction of the population may bind to cells in the early rounds of selection, but this fraction may progressively increase during the course of the selection.

The procedures described above may be used to identify aptamers that bind to a given cell line. Those having skill in the art will recognize that any number of different cell lines may be targeted. The following cell lines may be focused upon: two cervical cancer cell lines (HeLa and SiHa), one HPV infected cell line (TCL-1), and a normal cervical primary culture from Clonetics (CrEC-Ec). In those cell lines, in order to specifically identify aptamers that bind to pre-cancerous or cancerous cells, a coupled negative, positive selection may be employed.

First, the RNA population may be mixed with the parental, non-transformed line, CrEC-Ec, and those RNA species that do not bind to this cell line, or that are removed by the initial PBS washes, may be amplified. This delimited population may then be added to either the pre-cancerous (TCL-1) or cancerous (HeLa or SiHa line), and those RNA species that now bind may be selectively amplified. Multiple rounds of coupled negative and positive selection may yield aptamers that bind to proteins or , epitopes specific to transformed cells. Sequence comparisons within and between these families may aid in identifying which residues, motifs, and secondary structural features are most significant for binding. Based on such comparative results, a series of minimal aptamers may be readily synthesized and assayed for their ability to bind to cells. Those minimal aptamers that show the best binding characteristics may be selected.

Following the selection, aptamers with terminal functional groups may be synthesized to facilitate conjugation of the aptamers with nanoparticles. There are multiple different phosphoramidite reagents known in the art that can be introduced into the synthesis so that upon completion there are either alkyl amino or alkyl thiol groups at the 5' or 3' ends of the synthetic nucleic acid.

### Contrast Agents Based on Metal Nanoparticles

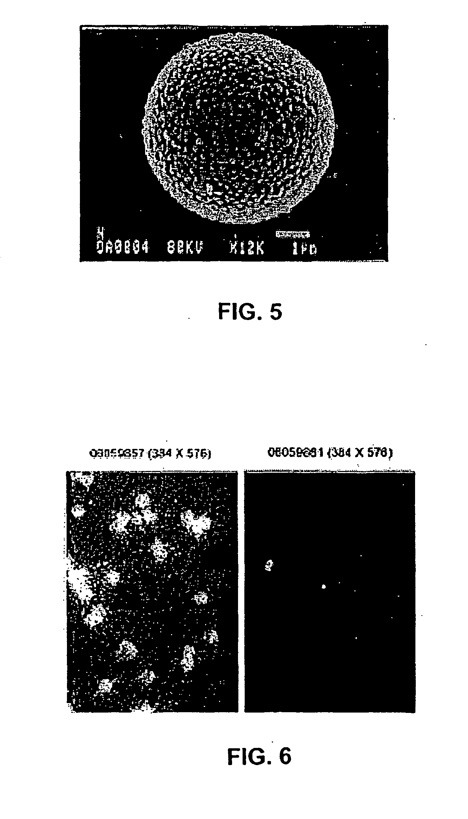

A potential problem in targeting cancer related biomarkers to screen for and detect neoplastic changes lies in the fact that many of the biomarkers are only overexpressed in tumors. It implies that they are also present in normal tissue and their amount increases with cancer development. This disclosure involves a novel detection scheme that provides enhanced contrast between normal and malignant tissue. One concept of these techniques is based on changes in optical properties of metal (or any other highly reflective) nanoparticles when they form closely spaced assembles. When gold or silver nanoparticles are brought in close vicinity, their plasmon resonances interact with each other. The interaction results in a red shift of plasmon resonances of particles' assemblies as compared to the individual particles (*see* **FIG. 4**). The extinction of the assembly in the red optical region relative to the position of resonance of the isolated nanoparticles is significantly higher than the sum of the extinction of individual particles forming the assembly. Therefore, excitation and collection conditions may be easily optimized to selectively detect closely spaced metal particles and their assemblies in the presence of the metal particles which are far apart. Application of this concept to contrast enhanced reflectance imaging of tissues is shown in **FIG. 5** and **FIG. 6****.**

First, the conjugates of metal nanoparticles with probe molecules specific for cancer related biomolecular targets may be allowed to interact with tissue and then the excess of the unbound contrast agents may be washed. Closely spaced assemblies of metal nanoparticles may be formed on the surface of neoplastic cells due to high concentration of biomarkers on their surface, while only individual particles spaced further apart may be present on normal cells. In this situation, imaging with a wavelength optimized for spectral properties of the assemblies may provide enhanced contrast between normal and neoplastic tissue.

With respect to this concept, the inventors have conducted an experiment with biotinilated polystyrene beads labeled with conjugates of silver particles with streptavidin. Beads with a high density (*see* **FIG. 5**) and a low density of silver particle conjugates were prepared and placed on the surface of a quartz prism. Then the beads were excited using 514.5 nm wavelength of Ar+ laser in total internal reflection mode. For illustration purposes, the sensitivity of the detector was reduced so that scattering from the beads with low density of silver conjugates would not be seen (*see* **FIG. 6**). As can be seen, the beads with closely spaced assemblies of silver particles exhibit dramatic scattering while the beads with just a few particles on the surface are not visible. Although this experiment may not provide a quantitative comparison between scattering properties of individual nanoparticles and their assemblies, because of significant differences in the amount of particles adsorbed on the surface of high and low density beads, it nevertheless illustrates and describes the use of metal nanoparticles as contrast probes in reflectance imaging.

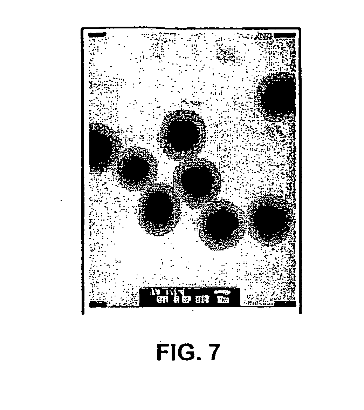

In different embodiments, gold and/or silver nanoparticles may be used. Each of these materials has their own advantages and disadvantages. It has been reported that silver particles exhibit higher extinction coefficients and provide higher enhancements of the local electromagnetic field and of other effects associated with optical excitation of surface plasmon resonances. [92]. However, silver particles are not as stable and not as biocompatible as gold nanoparticles. This issue can be addressed by encapsulating silver particles inside an inert material. For this purpose, a silica coating may be used (*see* **FIG. 7**). [68]. This coating stabilizes particles in high ionic strength solutions and provides a well-characterized surface for chemical immobilization ofbiomolecules.

Gold and silver colloidal particles may be prepared from chloroauric acid (HAuCl₄) and silver nitrate (AgNO₃) respectively by using a variety of reducing agents including phosphorous, [93] ascorbic acid, [94] sodium citrate, [95-97] borohydrate. [64,94]. Sodium citrate may be primarily used. Highly uniform gold colloids with particle sizes ranging from about 10 nm to ca. 100 nm may be prepared using sodium citrate reduction of chloroauric acid. [95]. This colloid exhibits a single extinction peak ranging from 500 nm to about 540 nm depending on the size of the particles (*see* **FIG. 4**). Sodium citrate reduction of silver nitrate results in a colloidal solution with about 35 nm diameter silver particles and a single peak at approximately 410 nm. [96,97]. The distribution of silver particles is significantly broader as compared to gold particles; however the procedure is highly reproducible from one preparation to another.

Silver particles with narrower distributions and different mean diameters may be prepared using a starter hydrosol with small silver particles that provides nucleation centers for growth of bigger silver colloid. [94]. In different embodiments, any of several standard, well-established procedures may be used in conjunction with the production of silver particles.

Positions of surface plasmon resonances of gold and silver may be significantly altered by using nanocomposite materials described in, for instance, [70,71]. The materials include a dielectric optically inert core particle and an optically active gold shell and can be prepared in a variety of sizes and in a highly uniform fashion. One may use the methods developed by Dr. N. J. Halas and Dr. J. L. West from Rice University in this regard.

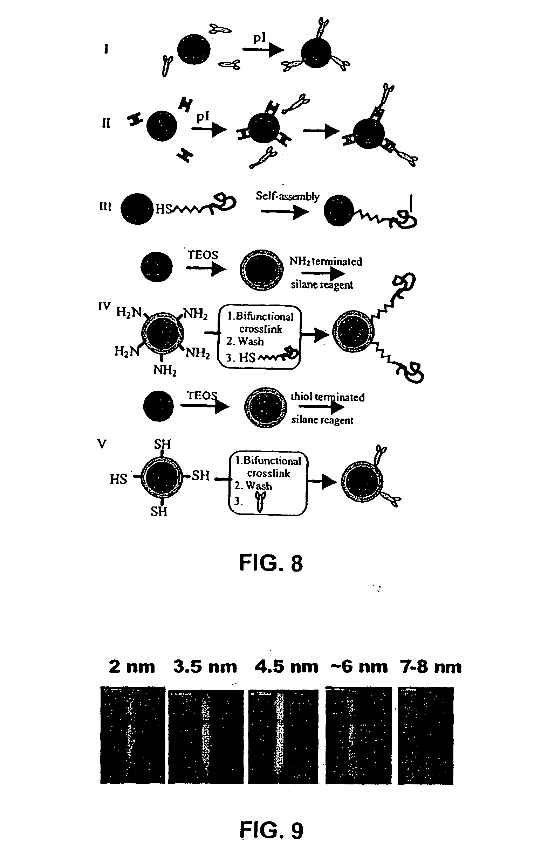

**FIG. 8** illustrates one embodiment for the preparation of conjugates of gold particles with cancer specific probe molecules. At least two different types of probe molecules may be used: well established antibodies for molecular biomarkers of cancer and aptamers specifically developed for cancer cells. Aptamers with antiangiogenic activity may be used. Such aptamers may be used to make conjugation chemistry with the metal particles for these type of molecules. For antibodies, conjugation protocols developed to prepare gold immunostains for electron microscopy may be used. [72,73].

Briefly, the procedure is based on non-covalent binding of proteins at their isoelectric point (point of zero net charge) to gold particles. The complex formation is irreversible and very stable. In fact, the shelf life of the conjugates is so long that the most commonly used gold immunostains can be routinely purchased from major biochemical companies. However, the described conjugation approach is not always successful. [72,73]. Therefore a second conjugation strategy for antibodies may include preparation of biotinilated antibody molecules and their consequent interaction with streptavidin/gold conjugates (*see* **FIG. 8**). This approach takes advantage of strong biospecific interaction between biotin and streptavidin and a well-developed protocol for immobilization of streptavidin on gold particles. [98].

Smaller aptamer molecules may not be directly adsorbed on the gold surface because that could significantly change their conformation and therefore lead to loss of binding properties. Aptamers with thiol terminated alkyl chains may be directly attached to the surface of gold particles similar to the procedures described in [69] for preparation of DNA probes. For conjugation procedure, one may use a mixture of thiol terminated aptamers and relatively small mercaptoacetic molecules to avoid high density immobilization of the aptamers (*see* **FIG. 8**).

The immobilization of antibodies and thiol terminated aptamers on silver particles may be accomplished using the strategies known in the art. The silver conjugates do not have the same stability and shelf life as the gold conjugates; however, they may be used to evaluate the silver based contrast agents. If silver particles are well suited for a particular application, one may use the conjugation protocols for silica capped silver particles (*see* **FIG. 8**). The silica layer may be formed using tetraethyl orthosilicate (TEOS) and the procedure described in detail in [68]. Many silanization reagents (Gelest, Inc.) are available to introduce functional groups to silica surface for subsequent immobilization of proteins, [99] nucleic acids including aptamers, [100-102] etc. It was shown that binding properties of aptamers are preserved after immobilization on glass cover slides [101] and silica microspheres. [102].

Described below are various testing techniques and issues relating, to forms of testing that may be used in conjunction with one or more embodiments of the present disclosure. Such techniques may, for example, allow for an assessment of the outcome of different immobilization protocols and/or simply verify that a study is proceeding as expected or desired.

### Testing - Contrast Agents

One may measure absorption for all prepared nanoparticles as well as excitation/emission spectra of qdots and scattering profiles of metal nanoparticles. To measure scattering properties of metal nanoparticles, a reflectance spectrometer may be used to measure scattering of cells and tissue slices. [14]. The range of nanoparticle optical parameters achievable with existing preparation methods may be determined to identify excitadon/collection geometries that can be used for *in vivo* imaging. The optical properties of bare nanoparticles, particles capped with a silica layer or mercaptoalkyl molecules, and particles conjugated with biomolecules may be compared. Immobilization of biomolecules on nanoparticles may be verified using at least three different techniques: UV-Vis spectroscopy (qdots, metal particles), anisotropic fluorescence spectroscopy (qdots), and conjugation assays. UV-Vis spectra of conjugates may exhibit absorption spectra characteristic for both antibodies and particles; controls will have only absorbance peaks of nanoparticles. Heavier conjugates may have slower rotation as compared to bare qdots that will result in higher anisotropy in their fluorescence. Conjugation assays may also be carried out, where purified target molecules which specifically bind to probe molecules attached to nanoparticles may be added to a suspension of probe/nanoparticles conjugates. This may result in aggregation of the conjugates which can be easily be monitored by UV-Vis (metal nanoparticles) or fluorescence anisotropy (qdots) spectroscopy. All these methods may allow for a quick assessment of the outcome of different immobilization protocols made in accordance with this disclosure. Stability of the prepared conjugates and bare nanoparticles under physiological conditions (pH, ionic strength, etc.) may be also studied.

### Biocompatibility of the Contrast Agents

Biocompatibility of the contrast agents disclosed herein may be a very important issue for *in vivo* applications. While it has been widely recognized that gold and, probably, Si based materials are inert with respect to biological tissue and are biocompatible (especially, gold), there are some concerns regarding silver and most semiconductor based materials. To address this issue, one may use silica capped CdS and silver nanocrystals. Silica is considered to be a biocompatible material except for the lung, where it can cause silicoses. A topical application of the contrast agents to cervical epithelium may be used. It is not anticipated that nanoparticles can penetrate inside the human body through layers of epithelial cells, basal membrane, and stroma. However, if desired, one may thoroughly study this issue before *in vivo* measurements are performed on a particular living subject. One may address issues relating to the penetration of the contrast agents using RAFT cultures and fresh tissue culture models. One may also perform standard cytotoxicity tests.

### Testing- Biological Systems

One may assess the interaction between labeled nanoparticles and normal and neoplastic cervical tissue using at least 3 biologically relevant models of cervical neoplasia: cell suspensions, RAFT cultures, and fresh tissue cultures. A similar approach may be used with all model systems. First, contrast agents may be applied to the model system by mixing (cell suspensions) or by topical application (RAFT cultures and fresh tissue slices) and may be allowed to interact with their specific targets. The incubation may be performed at the temperature characteristic for, for instance, the cervix (37°C). The contrast agents may be added in a solution formulated to prevent nonspecific binding of the probe molecules to the epithelial cells. High relative concentrations of "non-specific" proteins such as bovine serum albumin (BSA) are commonly used for this purpose. To determine non-specific binding, one may use "bare" (without attached probe molecules but with capping layers) qdots and metal nanoparticles as well as particles with attached biomolecules which do not specifically bind to cervical epithelial cells (*e.g.*, BSA). After incubation, the excess solution of probe molecules may be washed, and the optical characteristics of the labeled biological models may be measured. Following is a more detailed description for each system.

### Cell Suspensions

Suspensions of at least two cervical cancer cell lines (HeLa and SiHa) may be used, one transformed, HPV infected cell line (TCL-1), and a normal cervical primary culture from Clonetics (CrEC-Ec). Quantitative comparison of binding of different contrast agents to the cell lines may be carried out. Such experiments may identify or confirm the best contrast agents for discrimination between normal, pre-cancerous, and cancerous cervical cells. A combination of qdots based contrast agents of different sizes may be evaluated to improve detection of neoplastic cells in multi-color imaging strategies with a single excitation frequency (or multiple excitation frequencies). Excitation/emission wavelengths for fluorescence imaging with qdots and excitation wavelengths for reflectance imaging with metal nanoparticles may be optimized to provide the best contrast between normal and abnormal cells, to provide adequate penetration of the cervical epithelium and to match small, inexpensive laser diode or LED sources.

To quantify the binding of contrast agents to cells, one may use fluorescence (qdots) and UV-V is spectroscopy (metal nanoparticles). Scattering from cells and their relatively quick sedimentation in solution can significantly interfere with quantitative measurements. Furthermore, optical properties of particles can be altered as a result of binding. Therefore, one may centrifuge labeled cells and then measure the particles remaining in solution. The amount of bound agent may be determined based on the decrease in fluorescence and/or absorption. Standard confocal and deconvolution fluorescent microscopes and a confocal reflectance microscope may be used to image individual cells. Heterogeneity of binding and manner in which cells interact with the contrast agents (*e.g.*, if they undergo cellular uptake) may be determined.

Biocompatibility of the contrast agents may also be addressed by performing standard cytotoxicity assays. Labeled cells may be grown using standard cell culture techniques to address possible long term effects of the contrast agents on the cells.

### Organotypic (RAFT) Cultures

Conventional cell cultures provide homogeneous samples that can be used to study changes in normal and transformed epithelial cells. However, these cultures do not reflect the complex physical organization of the epithelium and underlying stroma present in real tissues. Recent developments in molecular biology, however, may be utilized to implement a series of progressively more complex culture models which have the desired physical and chemical properties and can be manipulated through the neoplastic process.

The maintenance of various tissue components in their normal anatomical relationship is important for regulation of growth and differentiation. [106,107]. Tumor cells, stromal fibroblasts, and endothelial cells may express a set of genes *in situ* that only partially overlaps the set expressed by each cell type in isolation from the other in primary cultures. Organotypic (RAFT) cultures have been developed initially for skin and then adapted for a variety of epithelial cancers as an approach to provide the three dimensional growth including epithelial cell-cell interactions that are major features of solid carcinomas. The method is based on the growth of epithelial cells at the air-liquid interface on top of a collagen gel containing fibroblasts (hence the name RAFT cultures for floating on the liquid phase). This organ culture provides conditions that preserve tissue architecture, growth, and function. It can be prepared with different cell layers, different cell types and can be analyzed as a tissue without restrictions involved in obtaining actual surgical specimens from patients or volunteers. RAFT cultures are also more reproducible than tissues obtained from different individuals.

One may prepare RAFT cultures using normal, pre-cancerous and cancerous cervical epithelial cells. Contrast agents may be added, washed, and then cultures may be examined using the optimized excitation/collection wavelengths determined from, for instance, experiments with cell suspensions. The contrast between images of RAFT cultures with normal, pre-cancerous and cancerous cells may be determined. This biological model of the epithelium may also provide an opportunity to measure the depth of penetration of the contrast agents through multiple layers of epithelial cells and to optimize binding kinetics before undertaking more difficult and resource-consuming experiments with human tissue samples. The same optical microscopic approaches as in the case of cells may be used.

### Fresh Tissue Slices

The contrast agents may be tested using a model system that most closely resembles living human epithelial tissue - fresh tissue slices. Dr. Richards-Kortum, Follen and Lotan have recently explored this model system to explore the biological basis for differences in the autofluorescence of normal and neoplastic cervix. [108,109]. To prepare fresh tissue slices, cervical biopsies may be obtained, and biopsies may be immediately placed in chilled culture medium, and then embedded in agarose. A Krumdieck Tissue Slicer may be used to obtain transverse, 200 µm thick fresh tissue slices, which can be maintained alive in culture for 7-10 days. Experiments with contrast agents may be performed within 1.5 to 5 hours of biopsy. The tissue slices can remain in culture medium during the imaging, and an image of a field of medium may be collected as a control. Following fluorescence microscopy, 4 µm sections may be made for histological evaluation and may be read by, for instance, a board certified pathologist to provide a diagnosis.

At least two different series of experiments may be performed. In one, contrast agents may be topically applied to the biopsy prior to preparation of tissue slices. Such studies allow one to evaluate the penetration depth of contrast agents inside the human epithelium, In the second, contrast agents may be applied to prepared tissue slices. In this case, one may assess the binding profile of different contrast agents throughout the whole thickness of epithelium. Contrast between normal, pre-cancerous and cancerous lesions in living cervical tissue may be determined. The best combinations of antibodies and/or aptamers labeled with qdots and metal particles for discrimination of pre-cancerous and cancerous lesions may be identified or confirmed.

One may initially conduct a pilot study of 18 patients, obtaining paired normal and abnormal biopsies from each patient. These data may be used to calculate the required sample size to achieve statistical significance to determine the sensitivity and selectivity of nanoparticles as compared to gold standard of histopathology. One may use a commercially available inverted fluorescence microscope and confocal reflectance microscope, which may also be used to image cervical biopsies and fresh tissue slices.

### Examples 1:

### Abstract

Recent developments in photonic technology provide the ability to non-invasively image cells *in vivo;* these new cellular imaging technologies may dramatically improve the prevention, detection and therapy of epithelial cancers. Endoscope-compatible microscopies, such as optical coherence tomography and reflectance confocal microscopy, image reflected light, providing a three-dimensional picture of tissue microanatomy with excellent spatial resolution (1 - 10 microns). However, their ability to image molecular biomarkers associated with cancer may be limited. In this example, we describe a new class of molecular specific contrast agents for vital reflectance imaging based on gold nanoparticles attached to probe molecules with high affinity for specific cellular biomarkers. The application of gold bioconjugates for vital imaging of pre-cancers is demonstrated using cancer cell suspensions, three-dimensional cell cultures, and normal and neoplastic fresh cervical biopsies. We show that gold conjugates can be delivered topically for imaging throughout the whole epithelium. These contrast agents may extend the ability of vital reflectance microscopies for *in vivo* molecular imaging, They may enable combined screening, detection and therapy of disease using inexpensive imaging systems; such tools may allow mass screening of diseases such as cancer in resource-poor settings.

### Introduction

Early diagnosis of premalignant and malignant lesions is essential for improving the current poor survival of patients with a variety of cancers. Non-invasive diagnostic methods are especially needed for the screening of large populations for the identification of high risk individuals who can then be followed up frequently and/or enrolled in chemoprevention trials. In the past decade a number of microscopic techniques have been developed to image living tissue with sub-cellular resolution. Vital microscopies, such as optical coherence tomography (OCT) and reflectance confocal microscopy (RCM), image reflected light, providing a detailed three-dimensional picture of tissue microanatomy without the need for physical sectioning. These technologies provide excellent spatial resolution (1 - 10 microns) with penetration depth ranging from 300 microns to 1-2 mm. The resulting histologic-quality images can identify and monitor neoplastic changes in epithelium. Recently, endoscope-compatible fiber optic OCT and RCM systems have been developed to image tissue microanatomy *in vivo* in near real time. These systems are portable and inexpensive compared to other high resolution imaging technologies such as MRI microscopy; as such they are ideally suited for early screening and diagnosis of superficial disease.

Tissue reflectance is produced by refractive index mismatches; sources of contrast in OCT and RCM images include structures with increased refractive index such as mitochondria, nuclear chromatin and melanin. Non-specific contrast agents, such as acetic acid, can perturb the nuclear refractive index distribution increasing the ability to visualize cellular anatomy. While OCT and RCM provide images of tissue microanatomy, their ability to image molecular changes associated with carcinogenesis is limited.

In the last few years, global analysis of gene expression by genomic and proteomic approaches have led to the discovery of new cancer related genes, proteins and biomarkers. Currently, most of these biomolecular signatures can only be assessed through invasive, painful biopsy. The ability to noninvasively image the expression of these biomarkers may translate into improved ability to screen and detect neoplastic changes, better ability to select and monitor therapy, and new tools to understand the pathobiology of the disease.

### Summary

In this example, we demonstrate a new class of molecular specific contrast agents for vital optical imaging of pre-cancers and cancers, based on gold nanoparticles conjugated to probe molecules with high affinity for cellular biomarkers. Conventional gold nanoparticles have been extensively used as molecular-specific stains in a different application-electron microscopy. As result, the fundamental principles of interactions between gold particles and biomolecules have been thoroughly studied. The nanoparticles also exhibit the ability to resonantly scatter visible and near infrared (NIR light). This property is the result of excitation of surface plasmon resonances and is extremely sensitive to the size, shape, and aggregation state of the particles. The ability to resonantly scatter visible and NIR light may be explored for vital microscopy in living specimens.

In this example, we describe bioconjugates of gold nanoparticles with monoclonal antibodies against EGFR, a transmembrane 170 kDa glycoprotein that is overexpressed in epithelial pre-cancers, for molecular specific optical imaging. A high level of EGFR expression is often associated with enhanced aggressiveness of epithelial cancers and poor prognosis. In these studies we used gold nanoparticles with ca. 12 nm in diameter. This size is approximately the same as the size of antibodies that are routinely used for molecular specific labeling and targeting.

To demonstrate the application of gold bioconjugates for vital reflectance imaging we used three biologically relevant models of cancer with increasing complexity. First, suspensions of cervical cancer cells were explored; SiHa cells are well-characterized cervical epithelial cancer cells that overexpress EGFR. Next, engineered tissue constructs, three-dimensional cell cultures that mimic major features of epithelial tissue, were explored. We prepared engineered tissue constructs consisting of densely packed, multiple layers of SiHa cells atop a collagen stroma. Finally, we demonstrated the application of contrast agents in normal and neoplastic fresh cervical biopsies - the model system that most closely resembles living human epithelial tissue.

### Methods

### Preparation of gold bioconjugates:

Colloidal gold of various sizes was prepared using citrate reduction of chloroauric acid (HAuCl₄) according to the method described in Frens, G. (1973) Nature Physical Science 241, 20-22. To prepare conjugates colloidal gold was diluted twice in 20 mM HEPES buffer, pH 7.4 and anti-EGFR monoclonal antibodies (host mouse, Sigma) were reconstituted in the same buffer at 100 µg/ml. Then the solutions were mixed at 1:1 volume ratio and were allowed to interact for 20 minutes at room temperature. Polyethyleneglycol (PEG, MW 20,000, Sigma) was added to the mixture up to a final concentration of 0.2 mg/mL and the solution was centrifuged twice at 5000 rpm for 2 hours to wash unbound antibodies. After the second wash the pellet was resuspended in phosphate buffered saline.

### Preparation of cells:

SiHa cells were grown inside tissue culture flasks covered with collagen type I (Roche) in DMEM plus 5% FBS at 37°C under 5% CO₂. Cells were harvested using 1 mg/mL collagenase (Roche) in phosphate buffered saline at 37°C for approximately 20 minutes, or until the collagen substrate was entirely disassociated, and were washed in DMEM. The cell suspension was labeled with gold conjugate at room temperature for ca. 30 minutes on a shaker to prevent sedimentation. The labeled cells were placed on top of a microscope slide coated with gelatin to eliminate background scattering from the glass substrate during reflectance imaging.

### Preparation of epithelial tissue constructs:

To prepare the constructs a suspension of epithelial cells was spun down and a very small amount of buffered collagen type I solution (3 mg/ml) was added to the pellet. The mixture was transferred to 6.5 mm Elisa plate wells and allowed to gel at 37°C for 20 minutes. The volume of the mixture was adjusted to form gels with thickness between 400 and 600 µm. The gel with embedded cells was kept in DMEM culture medium plus 5% FBS for 24-48 hours. During this time the cells continued to grow resulting in formation of a highly dense structure consisting of multiple layers of epithelial cells. The contrast agents were added on top of the tissue phantoms in 10% polyvinyl pyrrolidone (PVP) solution in PBS or in pure PBS. After incubation for ca. 30 minutes at room temperature the phantoms were transversely sectioned with a Krumdieck tissue slicer and the sections were imaged using Zeiss Leica inverted laser scanning confocal microscope.

### Preparation of fresh cervical biopsies:

Colposcopically normal and abnormal cervical biopsies were obtained, with written consent, from women seen in the University of Texas M.D. Anderson Cancer Center Colposcopy Clinic. Biopsies were immediately placed in chilled (4°C) culture medium (Dilbecco Modified Eagle Medium without phenol red), and then embedded in 4% agarose. Subsequently, a Krumdieck Tissue Slicer was used to obtain transverse, 200 □m thick fresh tissue slices. The slices were placed in a phosphate buffered saline solution of anti-EGFR/gold conjugates for ca. 30 minutes at room temperature. After incubation with contrast agents the sections were washed in PBS and were imaged. After imaging the sections were submitted for H&E staining and histopithological analysis.

The wavelength dependence of light scattering was measured using the optical set-up described in Sokolov, K., Drezek, R., Gossage, K., & Richards-Kortum, R. (1999) Optics Express 5 (13), 302-317. Briefly, samples were illuminated by a broad-band light source (halogen lamp, Dolan-Jenner Industries) and the scattered light was focused on the 250 µm entrance slit of a single grating spectrograph (F/3.8, 300 lines/mm grating, Monospec 18, Jarrel Ash) coupled to an intensified photodiode array detector (IRY-700, Princeton Instruments). Spectra were normalized by scattering from a "white" diffusely scattering substrate (Labsphere) to account for the wavelength dependence of the light source and the spectrometer.

### Confocal microscopy:

The series of through focus confocal images were acquired using Zeiss Leica inverted epi-fluorescence/reflectance laser scanning confocal microscope with a 40X oil immersion objective or a 10X objective. The excitation was provided by a Kr/Ar mixed gas laser.

### Figure Legends for Results and Discussion:

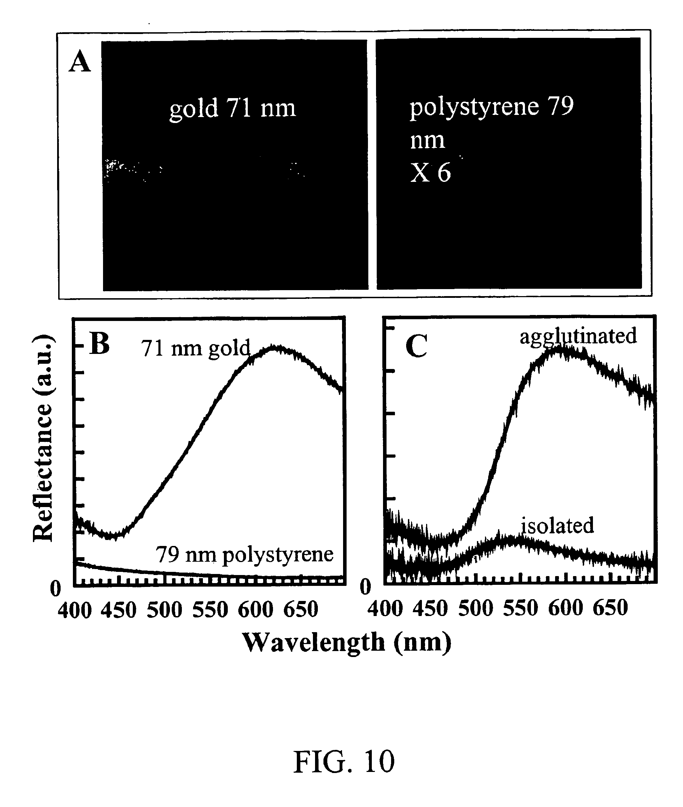

In the "Results and Discussion" section immediately below, reference is made to FIGS. 10-13. Corresponding figure legend are as follows:

### FIG. 10:

Scattering properties of gold nanoparticles are shown. FIGS. 10A and 10B compare scattering of gold particles and polystyrene beads of approximately the same diameter. In FIG. 10A suspensions of gold particles (left) and the polymeric spheres (right) were illuminated by a laser pointer which provides light in 630-680 nm region. The images were obtained using a regular web camera at a 90° angle relative to illumination. To acquire the images of both suspensions under the same conditions, the concentration of the polymeric beads (in particles per ml) was increased 6 fold relative to the concentration of the metal nanoparticles. FIG. 10B shows the wavelength dependence of visible light scattering by the polystyrene spheres and the gold nanoparticles. The spectra were obtained from suspensions with the same concentration of metal and polymeric nanospheres.

FIG. 10C compares scattering of isolated and closely spaced (agglutinated) conjugates of 12 nm gold nanoparticles with monoclonal antibodies for epidermal growth factor receptor (EGFR). Polyclonal antibodies specific for mouse IgG (Sigma) were added to induce agglutination of the conjugates.

### FIG. 11:

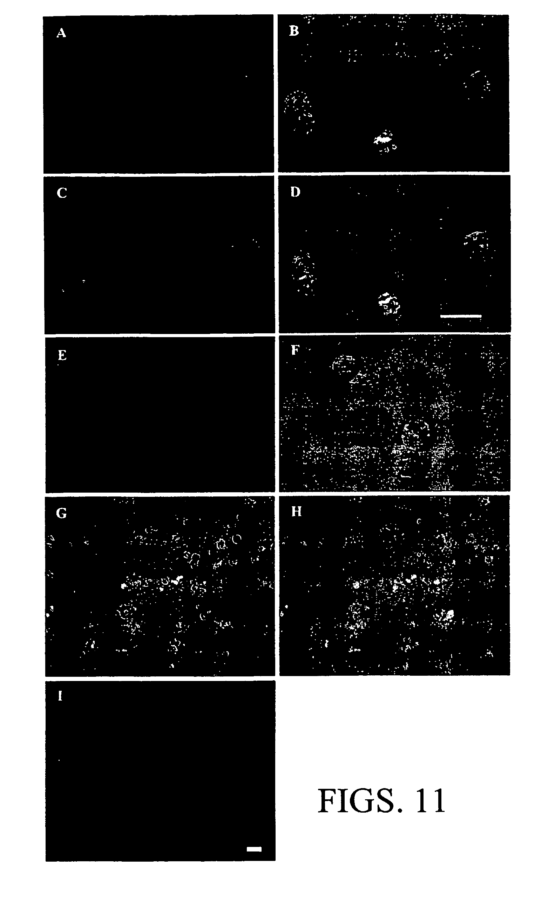

High (FIGS. 11A-11D) and low (FIGS. 11G-11I) resolution optical images of SiHa cells labeled with anti-EGFR/gold conjugates are shown. Non-specific labeling using gold conjugates with BSA is shown in FIGS. 11E and 11F. Laser scanning confocal reflectance (FIGS. 11A, 11C, and 11E) and combined confocal reflectance/transmittance (FIGS. 11B, 11D, and 11F) images of the labeled SiHa cells obtained with 40X objective. The scattering from gold conjugates is false-colored in red. In FIGS. 11A and 11B the focal plane is at the top of the cells. In FIGS. 11C and 11D the middle cross-section of the cells is in focus. The confocal reflectance and transmittance images were obtained independently and then overlaid. Reflectance images were obtained with 647 nm laser excitation. The scale bar is ca. 20 µm (FIGS. 11A-11F).

FIGS. 11G-11I: a series of bright-field and reflectance images of the labeled SiHa cells obtained with 20X objective using a combination of a white light and a laser-pointer illumination are shown: FIG. 11G white light illumination; FIG. 11H white light with a laser-pointer illumination at grazing incidence; FIG. 11I laser-pointer illumination at grazing incidence. The scattering of gold conjugates is false-colored in red. The laser pointer emits light in 630-680 nm region with power output less than 5 mW. The laser pointer illuminated an area ca. 3-5 mm in diameter. The scale bar is ca. 30 µm.

### FIG. 12:

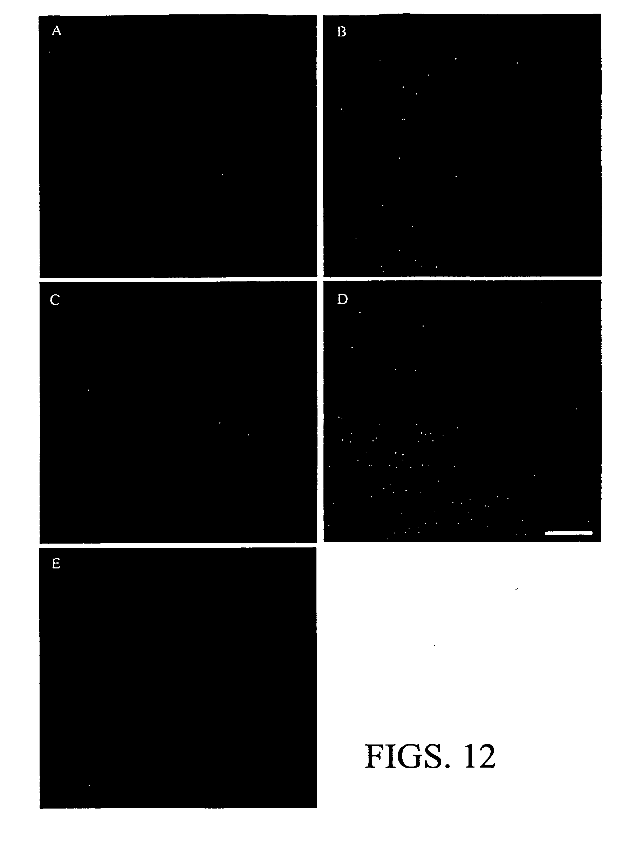

Laser scanning confocal reflectance (FIGS. 12A, 12C, and 12E) and confocal fluorescence (FIGS. 12B and 12D) images of pre-cancerous (FIGS. 12A and 12B) and normal (FIGS. 12C, 12D, and 12E) fresh cervical *ex vivo* tissue labeled with anti-EGFR/gold conjugates are shown. Reflectance images were obtained with 647 nm excitation wavelength and fluorescence images using 488 nm excitation and 515 nm long band-pass emission filter. Reflectance images FIG. 12A and 12C were obtained after labeling with gold conjugates under the same acquisition conditions. FIG. 12E was obtained after 6% acetic acid (AA) solution was added to the normal cervical biopsy and laser power was increased by ca. 6 fold. AA is a nonspecific contrast agent that is used in reflectance imaging of epithelium to increase scattering from nuclei. Confocal fluorescence images FIGS. 12B and 12D were obtained under the same acquisition conditions. The reflectance images are false-colored in red. The scale bar is ca. 20 µm.

### FIG. 13:

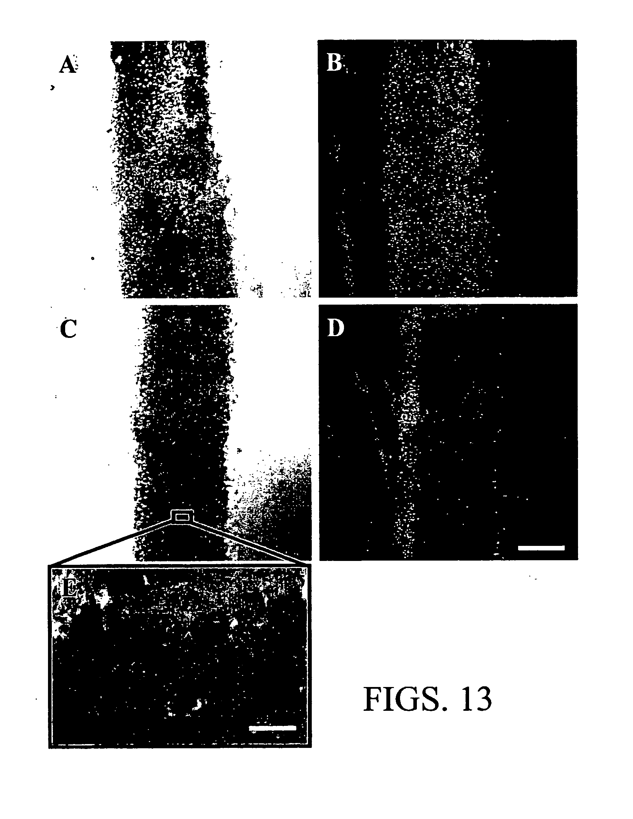

Transmittance (FIGS. 13A, 13C, and 13E) and reflectance (FIGS. 13B and 13D) images of engineered tissue constructs labeled with anti-EGFR/gold conjugates are shown. The tissue constructs consist of densely packed, multiple layers of cervical cancer (SiHa) cells. The contrast agents were added on top of the tissue phantoms in 10% polyvinyl pyrrolidone (PVP) solution in PBS (FIGS. 13A and 13B) or in pure PBS (FIGS. 13C and 13D). After incubation for ca. 30 minutes at room temperature the phantoms were transversely sectioned with a Krumdieck tissue slicer and the sections were imaged using the Zeiss Leica inverted laser scanning confocal microscope with 10X (FIGS. 13A-13D) objective. A small spot on a tissue construct was imaged using 40X oil immersion objective to show high density of the epithelial cells in the phantom (FIG. 13E). Reflectance images were obtained with 647 nm excitation. Arrows show the surfaces exposed to the contrast agents. The scale bars are ca. 200 µm (FIGS. 13A-13D) and ca. 20 µm (FIG. 13E).

### Results and Discussion

The scattering cross section of gold nanoparticles is extremely high compared to polymeric spheres of the same size (FIG. 10), especially in the red region of the spectrum. This property is important for development of contrast agents for optical imaging in living organisms because light penetration depth in tissue dramatically increases with increasing wavelength. Another interesting optical property of gold nanoparticles that can be exploited for vital optical imaging is the increase in scattering cross section per particle when the particles agglutinate (FIG. 10C). These changes produce a large optical contrast between isolated gold particles and assemblies of gold particles. This increase in contrast improves the ability to image markers which are not uniquely expressed in diseased tissue, but are expressed at higher levels relative to normal tissue (such as EGFR), and to develop highly sensitive labeling procedures which do not require intermediate washing steps to remove single unbound particles.

The preparation of gold bioconjugates may be based on non-covalent binding of the anti-EGFR IgG antibodies at their isoelectric point (point of zero net charge of the protein) to gold particles. The complex formation is irreversible and very stable. Specific optical changes in UV-Vis spectrum of gold nanoparticles indicate binding of the antibodies: a characteristic red shift (ca. 6 nm) of the maximum of the surface plasmon resonance and ca. 10 % decrease in transmission. These optical changes are associated with alterations in the local refractive index around the particles after binding of the monoclonal antibodies. An additional indication of protein binding to the surface of the nanoparticles is their stability in phosphate buffered saline (PBS). The gold conjugates are monodispersed in the saline solution while a suspension with "bare" gold particles quickly changes its color from red to blue upon addition of the saline as a result of aggregation of the nanoparticles. The anti-EGFR/gold complexes also undergo molecular specific agglutination when anti-IgG polyclonal antibodies are added to the suspension of the conjugates. The agglutination results in increased scattering by the conjugates (FIG. 10C).

FIGS. 11A-D show confocal reflectance images and combined transmittance/reflectance images of SiHa cells labeled with anti-EGFR/gold conjugates. In a series of through focus confocal reflectance images of labeled cells, the bound conjugates first appear as randomly distributed bright spots at the top of the cells, then bright rings can be seen in the optical cross-sections through the middle of the cells. Comparison of the labeling pattern with transmittance images of the cells indicates that labeling predominately occurs on the surface of the cellular cytoplasmic membrane. The labeling pattern is consistent with the fact that the monoclonal antibodies have molecular specificity to the extracellular domain of EGFR. The intensity of light scattering from the labeled SiHa cells is ca. 50 times higher than from unlabeled cells. Therefore unlabeled cells cannot be resolved on the dark background. No labeling was observed when gold conjugates with bovine serum albumin (BSA) were added to the cells (FIGS. 11E and 11F).

We conducted reflectance imaging before and after the unbound gold conjugates were washed from the cell suspension. The unbound gold particles were not visible before or after washing. UV-Vis measurements of a washed suspension of labeled cells showed an increase in extinction of the nanoparticles in the red optical region. This change is characteristic for agglutination of the nanoparticles and indicates that the particles form closely spaced assemblies on the surface of the cells. We demonstrated that agglutination of anti-EGFR gold conjugates results in increase of scattering of particles forming the assembly (FIG. 10C). We believe that similar effect can contribute to the contrast between the labeled cells and the isolated, unbound conjugates.

Using UV-Vis spectroscopy we estimated the average amount of gold conjugates bound per cell. Scattering from cells, their relatively quick sedimentation, and changes of optical properties of the particles upon binding make it difficult to measure the amount of bound nanoparticles directly. Instead, we centrifuged the labeled cells and measured the decrease in optical density of the supernatant relative to the original suspension of the conjugates. Using this approach we calculated that approximately 5x10⁴ conjugates are bound per cell. Our results correlate well with previously published studies, which report that most cell types express from 2x10⁴ to 20x10⁴ EGF receptors per cell.

We observed heterogeneous labeling of SiHa cells in suspension. To ensure that preparation of cell suspensions did not affect the extracellular domain of EGFR and produce heterogeneous labeling, we grew cells on collagen and used collagenase to harvest the cells. The same heterogeneity was also observed when the cells were labeled directly on the surface of the collagen matrix without harvesting. Heterogeneity of protein expression in cell lines is not uncommon and has been described before in the case of EGFR.

The light scattering from the labeled cells is so strong that it can be easily observed using low magnification optics and an inexpensive light source such as a laser pointer. FIGS. 11G-11I show a series of images of labeled SiHa cells placed on a microscope slide obtained using a 20X objective. In bright-field transmission, the cells with bound gold conjugates appear darker due to light absorption by the metal nanoparticles in the green optical region and the unlabeled cells appear more transparent (FIG. 11G). When the sample is illuminated by a laser pointer at grazing incidence, the labeled cells appear bright due to scattered light (FIG. 11H). Finally, after bright-field illumination is turned off, only labeled cells can be seen (FIG. 11I). No scattering was observed when cells labeled using gold conjugates with BSA were illuminated by a laser pointer under the same conditions.

Bright "honey-comb" like structures can be seen in laser scanning confocal reflectance images of abnormal cervical biopsies labeled with anti-EGFR/gold complexes (FIG. 12A). Scattering from the labeled cytoplasmic membranes of epithelial cells forms this pattern. No labeling of the normal biopsy can be seen when the sample is imaged under the same acquisition conditions as the abnormal sample (FIG. 12C). The morphology of the normal biopsy can be resolved after addition of a non-specific contrast agent - acetic acid - and increasing the laser power by ca. 6 fold (FIG. 12E). Acetic acid enhances fluctuations in the nuclear refractive index related to chromatin texture enhancing scattering from nuclei. An increase in scattering of stroma is also evident (FIG. 12E). There is no binding of anti-EGFR/gold conjugates to the stromal layer of cervical biopsies.

In corresponding autofluorescence confocal images obtained using 488 nm excitation, the epithelial cells exhibit cytoplasmic fluorescence (FIGS. 12B and 12D) due to mitochondrial flavin adenine dinucleotide (FAD). In the fluorescence image of the abnormal cervical biopsy the epithelial cells appear to be surrounded by black contours (FIG. 12B). These contours are formed by the bound gold conjugates which strongly absorb visible light in the green optical region where most of the autofluorescence signal is emitted (FIGS. 10B and 10C). The comparison of the reflectance and the fluorescence confocal images of the abnormal biopsy confirms predominant binding of the anti-EGFR/gold conjugates to the cytoplasmic membrane of the epithelial cells (FIGS. 12A and 12B).

Thus, the contrast agents presented here, coupled with vital reflectance microscopies, may yield both anatomic and molecular images of epithelial pathology. A particularly important application is the early detection of precancerous lesions. Early detection of curable precancers may dramatically reduce the incidence and mortality of cancer. *In vivo* application of these contrast agents requires the ability to deliver the agents throughout the epithelium in the organ site of interest. Pre-cancers of squamous epithelium originate at the basal layer, which can be located 300-500 µm beneath the tissue surface; therefore, to use diagnostic tools and to study the earliest molecular changes associated with cancer progression it is important to deliver the gold nanoparticles throughout the whole epithelium.

Using engineered tissue constructs, we demonstrated that penetration enhancers used for topical drug delivery, such as polyvinyl pyrrolidone (PVP), may be used to deliver the gold nanoparticles throughout the epithelium (FIG. 13). PVP is approved by FDA for human use as an excipient in topical formulations (e.g. Povidone). The anti-EGFR/gold conjugates were applied to the top of engineered tissue constructs in pure PBS buffer and in PBS in the presence of 10% PVP. After ca. 30 minutes incubation, constructs were washed in PBS and 200 µm thick transverse sections were prepared and imaged using transmittance and confocal reflectance microscopies (FIG. 13). When the conjugates are applied in the presence of PVP, uniform labelling is achieved throughout the whole depth (ca. 400 µm) (FIGS. 13A and 13B). When gold conjugates are applied in PBS, only the surface layer of epithelial cells in the engineered tissue constructs is labelled (FIGS. 13C and 13D).

The contrast agents presented here indicate the ability to extend vital reflectance microscopies for *in vivo* molecular imaging. Using these contrast agents, we demonstrated the ability to image the distribution of EGFR expression in living neoplastic cervical tissue - providing the ability to assess molecular pathology *in vivo.* Currently, the prognosis of patients with cancer is predicted mainly based on microanatomic features of disease; however, the use of molecular markers has recently shown promise to better predict patient outcomes and to select therapies. In the absence of contrast agents, OCM and RCM yield images of tissue microanatomy similar to that which can be obtained with conventional histopathology; contrast agents based on gold nanoparticles provide a strong source of signal with molecular specificity that is immune to photobleaching.

Other reflectance based technologies which been developed to image disease in deeper tissues with lower spatial resolution may also benefit from these contrast agents. Diffuse optical tomography (DOT) allows noninvasive in vivo imaging of oxygenated and deoxygenated hemoglobin and has been explored for detection of breast cancer; coupling DOT with the contrast agents presented here may provide more sensitive detection of smaller lesions.

Many properties of contrast agents based on gold nanoparticles make them ideally suited for vital imaging and *in vivo* diagnosis. By appropriately adjusting the size of the particles, surface plasmon resonances can be selected to take advantage of regions where tissue is most transparent depending on the degree of tissue penetration required. Using particles of different sizes conjugated to different probe molecules, multi-color labeling for many targets can be achieved. The enhanced scattering from closely spaced gold particles confers important advantages for *in vivo* imaging. First, the scattering from aggregates of bound particles is greatly enhanced compared to background scattering from unbound particles. Additionally, many markers are not uniquely expressed in disease, but are over- or under-expressed. The scattering from closely spaced aggregates associated with over-expression can magnify the signal difference owing to moderate levels of over-expression.

Contrast agents based on gold nanoparticle antibody conjugates may be put to *in vivo* use, with topical or systemic delivery. The inherent biocompatibility of gold means that they can be used directly *in vivo* without the need for protective layer growth. In fact, long term treatment of rheumatoid arthritis utilizes gold (up to a cumulative dose of 1.2-1.8 g/year for up to 10 years). We anticipate that less than 0.3 mg of gold may be required for diagnosis with topical delivery to the cervix. Humanized antibodies, where a mouse antibody-binding site is transferred to a human antibody gene, are much less immunogenic in humans, and many humanized antibodies are currently in clinical trials. Since 1997, the FDA has approved more than 10 monoclonal antibody based drugs, including Herceptin for metastatic breast cancer therapy. For surface lesions located in epithelial tissue, simple FDA approved agents, such as polyvinylpyrrolidone can be used to increase tissue permeability and deliver contrast agents topically.

### Example 2:

### Metal Nanoparticles and MMP

To demonstrate the imaging of metallo-proteases (MMPs) using contrast agents based on metal nanoparticles, we prepared gold conjugates with monoclonal antibodies for MMP-2 (data not shown) and MMP-9. The conjugates were used to label cervical epithelial cancer cells grown on two different substrates: a pure collagen I gel and a collagen I gel in the presence of 5% gelatin. SiHa cells were placed on a substrate and allowed to grow for 5-24 hrs in DMEM with 5% FBS at 37°C and 5% CO₂, and then antibody-gold conjugates were applied to a sample in PBS for 20-30 minutes under sterile conditions. Excess contrast agent was removed and the sample was imaged using a confocal reflectance/fluorescence microscope without intermediate washing. Reflectance images were obtained with 647 nm excitation and fluorescence was excited at 360 nm and collected using 405 band-pass emission filter.

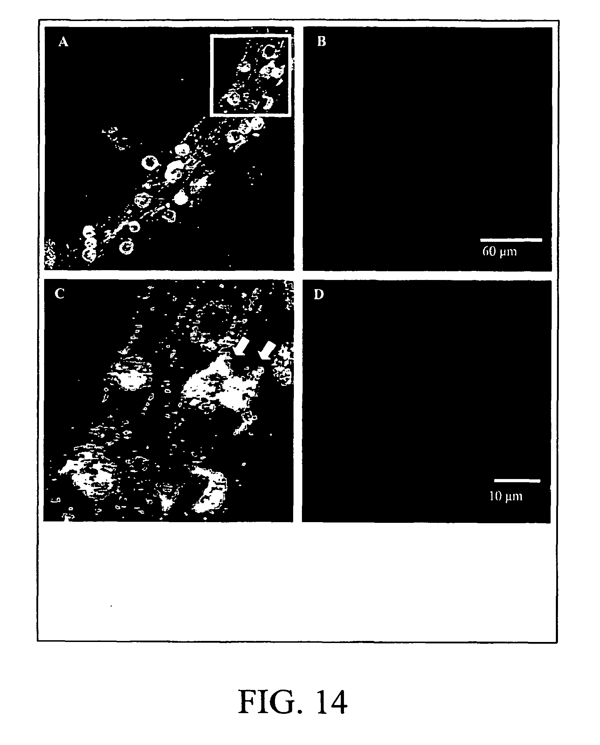

FIG. 14 shows the results of labeling of SiHa cells grown on collagen I substrate with anti-MMP-9/gold conjugates. Significant labeling of cellular cytoplasm was observed. Cytoplasmic labeling may be associated with internalization of labeled MMP-9 molecules from the plasma membrane. Rapid internalization and degradation of MMPs including MMP-9 is an important mechanism in regulating extracellular proteinase activity. We also observed strong labeling of collagen fibers located along clusters of cells (FIG. 14A and 14C). Previously, similar labeling was observed using fluorescent labeled antibodies in fixed 3D tissue cultures of cells inside collagen I matrix. It was attributed to membrane deposits which are shed by migrating cells along their tracks in collagen matrix. These deposits contain a variety of plasma membrane proteins including MMPs. Our images show a number of elongated polarized cells, indicative of cellular migration. Little cell or ECM labeling was observed in areas with low cell density.

### Metal Nanoparticles and Intracellular Targets

We can deliver metal nanoparticle based contrast agents to image intracellular targets. Contrast agents may be utilized to image molecular features of human papillomavirus (HPV) induced cervical carcinogenesis. Persistence and progression of cervical cancer is clearly related to expression of the viral oncoproteins, E6 and E7.

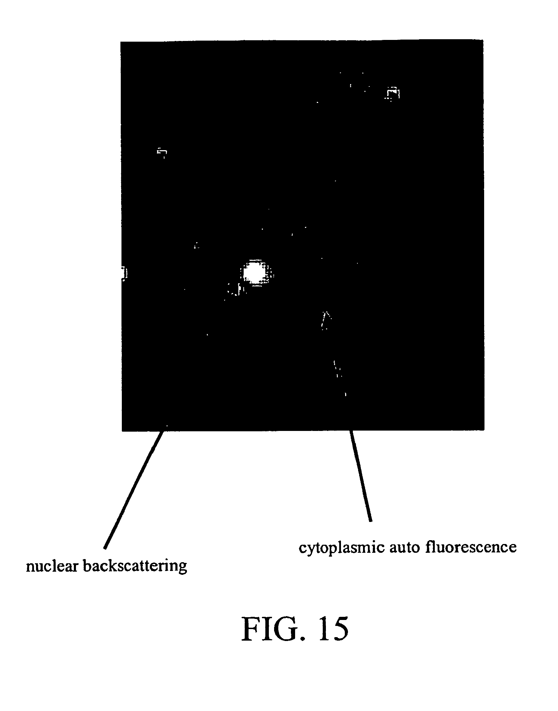

FIG. 15 shows preliminary results obtained labeling SiHa cells with 10 nm gold nanoparticles conjugated to anti-E7 monoclonal antibodies. Cells were incubated in contrast agent in PBS and in PBS with 10% PVP. Following incubation, cells were washed and imaged using laser scanning confocal microscopy. FIG. 15 shows the co-localized autofluorescence (green) and reflectance (white) images from SiHa cells incubated with PVP. Autofluorescence is limited to the cytoplasm, whereas backscattering is produced by nanoparticles within the nucleus. No backscattering was observed in cells incubated with contrast agent in PBS alone. Thus, delivery and detection of contrast agent is feasible. In data, we observed at least a three fold, statistically significant increase in nuclear backscattering from nanoparticles in cells with high E7 expression compared to cells with low E7 expression.

### Safety of Metal Nanoparticles for In Vivo Use

Contrast agents based on gold nanoparticle antibody conjugates may be used *in vivo,* with topical or systemic delivery. The inherent biocompatibility of gold implies they can be used directly *in vivo* without the need for protective layer growth. In fact, long term treatment of rheumatoid arthritis utilizes gold (up to a cumulative dose of 1.2-1.8 g/year for up to 10 years). We anticipate that less than 0.3 mg of gold may be required for diagnosis with topical delivery to the cervix. Humanized antibodies, where a mouse antibody-binding site is transferred to a human antibody gene, are much less immunogenic in humans, and many humanized antibodies are currently in clinical trials. Since 1997, the FDA has approved more than 10 monoclonal antibody based drugs, including Herceptin for metastatic breast cancer therapy. For surface lesions located in epithelial tissue, simple FDA approved agents, such as polyvinylpyrrolidone can be used to increase tissue permeability and deliver contrast agents topically.

### Optically active contrast agents to target the molecular signatures of neoplasia.

### Metal NPs

Vital reflectance imaging using both gold and silver nanoparticles may be accomplished using the techniques of this disclosure. Contrast agents based on gold nanoparticles may be used because gold is biocompatible and can be used directly for *in vivo* applications. Silver particles may be used also. Silver may exhibit higher extinction coefficients and provide higher enhancements of the local electromagnetic field and exhibit other effects associated with optical excitation of surface plasmon resonances. Silver particles are not as stable and not as biocompatible as gold nanoparticles. This issue can be addressed by encapsulating silver particles inside an inert material. One may use a silica coating developed by Dr. Sokolov in Sokolov, K., G. Chumanov, and T.M. Cotton, Enhancement of Molecular Fluorescence near the Surface of Colloidal Metal Films. Analytical Chemistry, 1998. 70(18): p. 3898-3905 . This coating stabilizes particles in high ionic strength solutions and provides a well characterized surface for chemical immobilization of biomolecules.

Gold and silver colloidal particles can be prepared from chloroauric acid (HAuCl₄) and silver nitrate (AgNO₃) respectively by using a variety of reducing agents including phosphorous, ascorbic acid, sodium citrate, borohydrate. Highly uniform gold colloids with particle sizes ranging from about 10 nm to *ca.* 100 nm can be prepared using sodium citrate reduction of chloroauric acid. This colloid exhibits a single extinction peak ranging from 500 nm to about 540 nm depending on the size of the particles. Sodium citrate reduction of silver nitrate results in a colloidal solution with about 35 nm diameter silver particles and a single peak at approximately 410 nm. The distribution of silver particles is significantly broader as compared to gold particles; however the procedure is highly reproducible from one preparation to another. Silver particles with narrower distributions and different mean diameters can be prepared using a starter hydrosol with small gold or silver particles that provides nucleation centers for growth of bigger silver colloid.

Scattering properties of metal nanoparticles depend on their size and shape. By changing sizes metal nanoparticles that exhibit different colors in reflected light can be produced. Additionally, position of surface plasmon resonances of gold and silver can be significantly altered by using nanocomposite materials described in (a) Sershen, S.R., et al., Temperature-sensitive polymer-nanoshell composites for photothermally modulated drug delivery. Journal of biomedical materials research, 2000. 51(3): p. 293-8, and (b) Averitt, R.D., S.L. Westcott, and N.J. Halas, The Linear Optical Properties of Gold Nanoshells. J. Opt Soc Am B, 1999. 16: p. 1824-32. The materials include a dielectric optically inert core particle and an optically active gold shell and can be prepared in a variety of sizes and in a highly uniform fashion.

Another approach to tune optical properties of metal nanoparticles is based on template synthesis such as that in Haes, A.J. and R.P. Van Duyne, A nanoscale optical biosensor: Sensitivity and selectivity of an approach based on the localized surface plasmon resonance spectroscopy of triangular silver nanoparticles. Journal of the American Chemical Society, 2002. 124(35): p. 10596-10604. In one method, metal nanoparticles of pyramidal shape with different sizes can be synthesized inside cavities formed by a dense monolayer of polystyrene beads on a flat substrate. After synthesis the nanoparticles can be removed from the surface by a simple one step procedure. These approaches may be used to optimize scattering properties of nanoparticles to take advantage of optical regions where tissue is most transparent depending on the degree of tissue penetration required.

Another venue may be to use particles of different sizes conjugated to different probe molecules to achieve multi-color labeling for many targets.

FIG. 8 summarizes preparation of conjugates of gold particles with cancer specific probe molecules. At least two different types of probe molecules may be used: well established commercially available antibodies for molecular biomarkers of cancer and peptides specifically developed to bind to cancer biomarkers. One may utilize humanized antibodies. For antibodies, one may use conjugation protocols developed to prepare gold immunostains for electron microscopy. See Horisberger, M., Colloidal gold : a cytochemical marker for light and fluorescent microscopy and for transmission and scanning electron microscopy. Scan Electron Microsc, 1981. 2: p. 9-31 and Geoghegan, W.D. and G.A. Ackerman, Adsorption of horseradish peroxidase, ovomucoid and anti-immunoglobulin to colloidal gold for the indirect detection of concanavalin A, wheat germ agglutinin and goat anti-human immunoglobulin G on cell surfaces at the electron microscopic level: a new method, theory and application. The journal of histochemistry and cytochemistry : official journal of the Histochemistry Society, 1977. 25(11): p. 1187-200. Briefly, the procedure is based on non-covalent binding of proteins at their isoelectric point (point of zero net charge) to gold particles. The complex formation is irreversible and very stable. In fact, the shelf life of the conjugates is so long that the most commonly used gold immunostains can be routinely purchased from major biochemical companies. A colloidal gold drug delivery system was recently used to specifically target tumors in live mice. The reported results demonstrate stability of gold bioconjugates *in vivo.*

A second conjugation strategy for antibodies may include preparation of biotinylated antibody molecules and their consequent interaction with streptavidin/gold conjugates (FIG. 8). This approach takes advantage of strong biospecific interaction between biotin and streptavidin and a well-developed protocol for immobilization of streptavidin on gold particles. Smaller peptide molecules can not be directly adsorbed on the gold surface because that could significantly change their conformation and lead to loss of binding properties. Peptides with thiol terminated alkyl chains may be directly attached to the surface of gold particles similar to the procedures described in Elghanian, R., et al., Selective colorimetric detection of polynucleotides based on the distance-dependent optical properties of gold nanoparticles. Science, 1997. 277(5329): p. 1078-1080 for preparation of DNA probes. For conjugation procedure one may use a mixture of thiol terminated peptides and relatively small mercaptoacetic molecules to avoid high density immobilization of the peptides (FIG. 8).

The immobilization of antibodies and thiol terminated peptides on silver particles may be accomplished using strategies similar to those utilized for gold particles. Silver conjugates may not have the same stability and shelf life as the gold conjugates, however they can be used to evaluate the silver based contrast agents. Conjugation protocols for silica capped silver particles may be formed (FIG. 8). The silica layer may be formed using tetraethyl orthosilicate (TEOS). See Sokolov, K., G. Chumanov, and T.M. Cotton, Enhancement of Molecular Fluorescence near the Surface of Colloidal Metal Films. Analytical Chemistry, 1998. 70(18): p. 3898-3905. Many silanization reagents (Gelest, Inc.) are available to introduce functional groups to silica surfaces for subsequent protein immobilization.

For vital imaging with contrast agents based on metal nanoparticles it may be important to develop bioconjugates that have very low nonspecific binding and are not accumulated by the reticuloendothelial system (RES), namely the liver and spleen. To address this issue one may prepare hybrid conjugates by co-adsorbing polyethylene glycol (PEG) and probe (antibodies, peptides) molecules on the surface on nanoparticles. This strategy has been recently demonstrated in experiments on *in vivo* molecular specific imaging of embryogenesis using quantum dots and in colloidal gold drug delivery system in live mice. One may use commercially available thiol terminated PEG from Shearwater Polymers, AL. In the case of antibodies, one may first prepare conjugates of gold nanoparticles with the proteins and then expose the prepared complexes to thiol-PEG. Because strong interactions between thiol groups and the metal surface can lead to replacement of bound antibodies by PEG molecules it is important to carefully control PEG co-adsorption. This can be achieved by adjusting PEG concentration and by using compounds with high affinity to thiol groups to timely terminate the reaction. Very small (few nanometers in diameter) gold nanoparticles may be used to inhibit free thiol molecules. The smaller gold particles and their complexes with PEG may be isolated from the gold immunoconjugates by centrifugation. Another possibility is to use zinc (Zn²⁺) ions which can form stable complexes with thiol groups. To co-adsorb thiol terminated peptide molecules and thiol-PEG, one may mix the two compounds together and apply them to gold nanoparticles at the same time. In this case the reaction can be simply controlled by adjusting relative amount of peptides and PEG molecules.

### References

1. The 1988 Bethesda System for reporting cervical/vaginal cytologic diagnoses. National Cancer Institute Workshop. JAMA 1989;262:931-934.
2. Koss LG. The Papanicolaou test for cervical cancer detection. A triumph and a tragedy [see comment citation in Medline]. JAMA 1989;261:737-743.
3. Kelloff GJ, Boone CW, Crowell JA, Steele VE, Lubet R, Doody LA. Surrogate endpoint biomarkers for phase II cancer chemoprevention trials. J Cell Biochem Suppl 1994;19:1-9.
4. Daly MB. The chemoprevention of cancer: Directions for the future. Cancer Epidemiol Biomarkers Prev 1993;2:509-12.
5. S. Lam, T. Kennedy, M. Unger, Y.E. Miller, D. Gelmont, V. Rush, B. Gipe, D. Howard, J.C. LeRiche, A. Coldman, and A.F. Gazdar, "Localization of Bronchial Intraepithelial Neoplastic Lesions by Fluorescence Bronchoscopy,"Chest 113 (2), 696-702 (1998).
6. S. Lam, C. MacAulay, J. Hung, J. LeRiche, A.E. Profio, and B. Palcic, "Detection of Dysplasia and Carcinoma In Situ with a Lung Imaging Fluorescence Endoscope Device," J of Thoracic & Cardiovascular Surgery 105 (6), 1035-40 (1993).
7. B.W. Pogue, G.C. Burke, J. Weaver, D.M. Harper, "Development of a Spectrally Resolved Colposcope for early detection of Cervical Cancer," in Biomedical Optical Spectroscopy and Diagnostics Technical Digest (Optical Society of America, Washington DC,1998), 87-89.
8. S. L. Jacques, J.R. Roman, and K. Lee, "Imaging Superficial Tissues with Polarized Light," Lasers Surg. Med. 26,119-129 (2000).
9. Smithpeter C, Dunn A, Drezek R, Collier T, Richards-Kortum R; Real Time Confocal Microscopy of In Situ Amelanotic Cells: Sources of Signal, Contrast Agents and Limits of Contrast, Journal of Biomedical Optics, 3:429-36,1998.
10. L. T. Perelman, V. Backman, M. Wallace, G. Zonios, R. Manoharan, A. Nusrat, S. Shields, M. Seiler, C. Lima, T. Hamano, I. Itzkan, J. Van Dam, J. M. Crawford, and M. S. Feld, "Observation of Periodic Fine Structure in Reflectance from Biological Tissue: A New Technique for Measuring Nuclear Size Distribution," Physical Review Letters, vol. 80, pp. 627-630, 1998.
11. J. R. Mourant, I. J. Bigio, J. Boyer, R. L. Conn, T. Johnson, and T. Shimada, "Spectroscopic Diagnosis of Bladder Cancer with Elastic Light Scattering," Lasers in Surgery and Medicine, vol. 17, pp. 350-357, 1995.
12. I. J. Bigio, J. R. Mourant, J. D. Boyer, T. M. Johnson, T. Shimada, and R. L. Conn, "Noninvasive Identification of Bladder Cancer with Subsurface Backscattered Light," presented at Advances in Laser and Light Spectroscopy to Diagnose Cancer and Other Diseases, Los Angeles, CA, 1994.
13. I. J. Bigio, J. D. Boyer, T. M. Johnson, J. Lacey, and J. R. Mourant, "Detection of Gastrointestinal Cancer by Elastic Scattering and Absorption Spectroscopies with the Los Alamos Optical Biopsy System," presented at Advances in Laser and Light Spectroscopy to Diagnose Cancer and Other Diseases II, San Jose, CA, 1995.
14. Sokolov K., Drezek R., Gossage K., and Richards-Kortum R. Reflectance Spectroscopy with Polarized Light: Is it Sensitive to Cellular and Nuclear Morphology. - Optics Express, 1999, v. 5, No. 13, pp. 302-317.
15. R. R. Alfano, G. C. Tang, A. Pradham, W. Lam, D. S. J. Choy, and E. Opher, "Fluorescence Spectra from Cancerous and Normal Human Breast and Lung Tissues," IEEE Journ. Quant. Electron., vol. QE-23, pp. 1806-1811, 1987.
16. R. M. Cothren, R. Richards-Kortum, M. V. Sivak, M. Fitzmaurice, R. P. Rava, G. A. Boyce, M. Doxtader, R. Blackman, T. B. Ivanc, G. B. Hayes, M. S. Feld, and R. E. Petras, "Gastrointestinal tissue diagnosis by laser-induced fluorescence spectroscopy at endoscopy," Gastrointest. Endosc., vol. 36, pp. 105-111, 1990.
17. R. M. Cothren, M. V. Sivak, J. Van Dam, R. E. Petras, M. Fitzmaurice, J. M. Crawford, J. Wu, J. F. Brennan, R. P. Rava, R. Manoharan, and M. S. Feld, "Detection of Dysplasia at Colonoscopy Using Laser-Induced Fluorescence: A Blinded Study," Gastrointest. Endosc., pp. in press, 1995.
18. S. Lam, C. MacAulay, J. Hung, J. LeRiche, A. E. Profio, and B. Palcic, "Detection of dysplasia and carcinoma in situ with a lung imaging fluorescence endoscope device," J. Thorac. Cardiovasc. Surg., vol. 105, pp. 1035-1040, 1993.
19. K. Svanberg, S. Andersson-Engels, R. Berg, J. Johansson, S. Svanberg, and I. Wang, "Tissue Characterization in Different Malignant Tumors Utilizing Laser-Induced Fluorescence," presented at Advances in Laser and Light Spectroscopy to Diagnose Cancer and Other Diseases II, San Jose, CA, 1995.
20. T. Vo-Dinh, M. Panjehpour, B. F. Overholt, C. Farris, F. P. Buckley, and R. Sneed, "In Vivo Cancer Diagnosis of the Esophagus Using Differential Normalized Fluorescence (DNF) Indices," Las. Surg. Med., vol. 16, pp. 41-47, 1995.
21. A. Mahadevan, M. Follen Mitchell, E. Silva, S. Thomsen, and R. Richards-Kortum, "Study of the Fluorescence Properties of Normal and Neoplastic Human Cervical Tissue," Lasers in Surgery and Medicine, vol. 13, pp. 647-655, 1993.
22. N. Ramanujam, M. Follen Mitchell, A. Mahadevan, S. Thomsen, A. Malpica, T. Wright, N. Atkinson, and R. Richards-Kortum, "Development of a Multivariate Statistical Algorithm to Analyze Human Cervical Tissue Fluorescence Spectra Acquired in vivo," Lasers in Surgery and Medicine, vol. 19, pp. 46-62, 1996.
23. N. Ramanujam, M. Follen Mitchell, A. Mahadevan-Jansen, S. L. Thomsen, G. Staerkel, A. Malpica, T. Wright, N. Atkinson, and R. Richards-Kortum, "Cervical Precancer Detection Using a Multivariate Statistical Algorithm Based on LIF Spectra at Multiple Excitation Wavelengths," Photochemistry and Photobiology, vol. 64, pp. 720-735, 1996.
24. K. Turner, N. Ramanujam, J. Ghosh, and R. Richards-Kortum, "Ensembles of Radial Basis Function Networks for Spectroscopic Detection of Cervical Pre-Cancer," IEEE Transactions on Biomedical Engineering, pp. in press, 1998.
25. Anderson, M., Jordan, J., Morse, M., and Sharp, F. A Text and Atlas of Integrated Colposcopy. St. Louis: Mosby, 1991.
26. Burke, L., Antonioli, D., and Ducatman, B. "Colposcopy: Text and Atlas." Norwalk, CT: Appleton&Lange, 1991.
27. M. Follen and D. Schottenfeld, "Surrogate Endpoint Biomarkers: their Modulation in Cervical Chemoprevention Trials," Cancer, submitted, 2001.
28. Xu XC, Mitchell MF, Silva E, Jetten A, Lotan R, "Decreased expression of retinoic acid receptors, transforming growth factor beta, involucrin, and comifin in cervical intraepithelial neoplasia," Clin Cancer Res 1999 Jun;5(6): 503-8
29. Boone CW, Kelloff GJ, Steele VE. Natural history of intraepithelial neoplasia in humans with implications for cancer chemoprevention strategy. Cancer Res 1992;52:1651-59.
30. Boone CW, Bacus JW, Bacus JV, Steele VE, Kelloff GJ. Properties of intraepithelial neoplasia relevant to cancer chemoprevention and to the development of surrogate end points for clinical trials (44165). Proc Soc Exp Biol Med 1997;216:151-65.
31. Heatley MK. Proliferation in the normal cervix and in preinvasive cervical lesions [editorial]. J Clin Pathol 1996;49:957.
32. Heatley MK. What is the value of proliferation markers in the normal and neoplastic cervix? Histol Histopathol 1998;13:249-54.
33. Ho GYF, Burk RD, Klein S, Kadish AS, Chang CJ, Palan P, et al. Persistent genital human papillomavirus infection as a risk factor for persistent cervical dysplasia. J Natl Cancer Inst 1995;87:1365-71.
34. Fujimoto J, Sakaguchi H, Hirose R, Ichigo S, Tamaya T. Expression of vascular endothelial growth factor (VEGF) and its mRNA in uterine cervical cancers. Br J Cancer 1999;80:827-33.
35. "Diagnostic Cytology," L.G. Koss, Ed., J.B. Lippincott Co., Philadelphia, 1992.
36. E. Mastrobattista, G.A. Koning, and G. Storm, "Immonoliposomes for the Targeted Delivery of Antitumor Drugs, " Adv Drug Delivery Reviews 1999, 40: 103-27.
37. J. Sudimack and R.J. Lee, "Targeted Drug Delivery Via Folate Receptor," Adv Drug Delivery Reviews 2000, 41: 147-62.
38. S.P. Vyas and V. Sihorkar, "Endogenous Carriers and Ligands in Non-Immunogenic Site-Specific Drug Delivery," Adv Drug Delivery Reviews 2000, 43: 101-64.
39. D.B. Kirpotin, J.W. Park, K. Hong, S. Zalipsky, W.L. Li, P. Carter, C.C. Benz, and D. Papahadjopoulos, "Sterically Stabilized anti-HER2 immunoliposomes: design and targeting to human breast cancer cells in vitro, " Biochemistry 1997, 36: 66-75.
40. Conrad, R. C., L. Giver, et al. (1996). "In vitro selection of nucleic acid aptamers that bind proteins." Methods Enzymol 267: 336-67.
41. Osborne, S. E., I. Matsumura, et al. (1997). "Aptamers as therapeutic and diagnostic reagents: problems and prospects." Curr Opin Chem Biol 1(1): 5-9.
42. Famulok, M. and G. Mayer (1999). "Aptamers as tools in molecular biology and immunology." Curr Top Microbiol Immunol 243: 123-36.
43. Hesselberth, J., M. P. Robertson, et al. (2000). "In vitro selection of nucleic acids for diagnostic applications [In Process Citation]." J Biotechnol 74(1): 15-25.
44. Bartel, D. P. and J. W. Szostak (1993). "Isolation of new ribozymes from a large pool of random sequences [see comment]." Science 261(5127): 1411-8.
45. Xu, W. and A. D. Ellington (1996). "Anti-peptide aptamers recognize amino acid sequence and bind a protein epitope." Proc Natl Acad Sci U S A 93(15): 7475-80
46. Weiss, S., D. Proske, et al. (1997). "RNA aptamers specifically interact with the prion protein PrP." J Virol 71(11): 8790-7
47. Convery, M. A., S. Rowsell, et al. (1998). "Crystal structure of an RNA aptamer-protein complex at 2.8 A resolution." Nat Struct Biol 5(2): 133-9.
48. Homann, M. and H. U. Goringer (1999). "Combinatorial selection of high affinity RNA ligands to live African trypanosomes." Nucleic Acids Res 27(9): 2006-14.
49. Tuerk, C. and L. Gold (1990). "Systematic evolution of ligands by exponential enrichment: RNA ligands to bacteriophage T4 DNA polymerase." Science 249(4968): 505-10.
50. Giver, L., D. Bartel, et al. (1993). "Selective optimization of the Rev-binding element of HIV-1." Nucleic Acids Res 21(23): 5509-16.
51. Ellington, A. D., F. Leclerc, et al. (1996). "An RNA groove [news]." Nat Struct Biol 3(12): 981-4.
52. Conrad, R., L. M. Keranen, et al. (1994). "Isozyme-specific inhibition of protein kinase C by RNA aptamers." J Biol Chem 269(51): 32051-4.
53. Bell, S. D., J. M. Denu, et al. (1998). "RNA molecules that bind to and inhibit the active site of a tyrosine phosphatase." J Biol Chem 273(23): 14309-14.
54. Jellinek, D., C. K. Lynott, et al. (1993). "High-affinity RNA ligands to basic fibroblast growth factor inhibit receptor binding." Proc Natl Acad Sci U S A 90(23): 11227-31.
55. Jellinek, D., L. S. Green, et al. (1995). "Potent 2'-amino-2'-deoxypyrimidine RNA inhibitors of basic fibroblast growth factor." Biochemistry 34(36): 11363-72.
56. Jellinek, D., L. S. Green, et al. (1994). "Inhibition of receptor binding by high-affinity RNA ligands to vascular endothelial growth factor." Biochemistry 33(34): 10450-6.
57. Green, L. S., D. Jellinek, et al. (1995). "Nuclease-resistant nucleic acid ligands to vascular permeability factor/vascular endothelial growth factor." Chem Biol 2(10):683-95.
58. Tsai, D. E., D. J. Kenan, et al. (1992). "In vitro selection of an RNA epitope immunologically cross-reactive with a peptide." Proc Natl Acad Sci U S A 89(19): 8864-8.
59. Tuerk, C., S. MacDougal, et al. (1992). "RNA pseudoknots that inhibit human immunodeficiency virus type 1 reverse transcriptase." Proc Natl Acad Sci U S A 89(15): 6988-92.
60. Eaton, B. E., L. Gold, et al. (1995). "Let's get specific: the relationship between specificity and affinity." Chem Biol 2(10): 633-8.
61. Hirao, I., M. Spingola, et al. (1998). "The limits of specificity: an experimental analysis with RNA aptamers to MS2 coat protein variants." Mol Divers 4(2): 75-89.
62. P. Mulvaney, "Surface Plasmon Spectroscopy of Nanosized Metal Particles," Langmuir 1996, 12:788-800.
63. M. Moscovits, "Surface-Enhanced Spectroscopy," Rev Mod. Phys. 1985, 57:783-826.
64. Cotton, T.M. In Spectroscopy of Surfaces; Clark, R.J.H., Hester, R.E., Eds.; Wiley: New York, 1988, 91.
65. Nabiev I., Sokolov K., Manfait M. Surface Enhanced Raman Spectroscopy and its Biomedical Applications, in Biomolecular Spectroscopy. / Eds. R.J.H. Clark and R.A. Hester. Chichester, New York, Brisbane, Toronto, Singapore: John Wiley & Sons, 1993, v.20, p. 267-338.
66. Nabiev, I. R.; Morjani, H.; Manfait, M, "Selective analysis of antitumor drug interaction with living cancer cells as probed by surface-enhanced Raman spectroscopy," Eur. Biophys. J. 1991, 19(6): 311-16.
67. S. Nie and S.R. Emory, "Probing Single Molecules and Single Nanoparticles by Surface-Enhanced Raman Scattering," Science 1997, 275: 1102-06.
68. Sokolov K., Chumanov G.D., and Cotton T.M. Enhancement of Molecular Fluorescence near the Surface of Colloidal Metal Films. - Anal. Chem., 1998, v. 70, pp. 3898-3905.
69. R. Elghanian, J.J. Storhoff, R.C. Mucic, R.L. Letsinger, and C. A. Mirkin, "Selective Colorimetric Detection of Polynucleotides Based on the Distance-Dependent Optical Properties of Gold Nanoparticles," Science 1997, 277: 1078-81.
70. S.R. Sershen, S.L. Westcott, N.L. Halas, and J.L. West, "Temperature-Sensitive Polymer-Nanoshell Composites for Photothermally Modulated Drug Delivery," J. Biomed. Mater. Res. 2000, 51(3)
71. R.D. Averitt, S.L. Westcott, and N.J. Halas, "The Linear Optical Properties of Gold Nanoshells," J. Opt Soc Am B 1999, 16: 1824-32.
72. M. Horisberger, "Colloidal Gold: a Cytochemical Marker for Light and Fluorescent Microscopy and for Transmission and Scanning Electron Microscopt, " Scanning Electron Microscopy 1981, 11: 9-31.
73. W.D. Geoghegan and G.A. Ackerman, "Adsorption of Horseradish Peroxidase, Ovomucoid and Anti-Immunoglobulin to Colloidal Gold for the Indirect Detection of Concanavalin A, Wheat Germ Agglutination and Goat Anti-Human immunoglobulin G on Cell Surface at the Electron Microscope Level: a New Method, Theory and Application," J of Histochemistry and Cytochemistry 1977, 25: 1187-1200.
74. Sokolov K., Chumanov G., Cotton T.M. New Substrates for Enhanced Spectroscopies, in Laser Techniques for Surface Science II. / Eds. J.M. Hicks, W. Ho, and H.-L. Dai. Proc. SPIE, 1995, v. 2547, pp. 117-124.
75. D. Andreyev, T.M. Cotton, and K. Sokolov, "Template Synthesis of Thin Films and Particulates With Uniform Nanosized Channels," JACS, submitted 2001.
76. Fritzsche W., Sokolov K., Chumanov G.D., Cotton T.M., and Henderson E. Ultrastructural Characterization of Colloidal Metal Films for Bioanalytical Applications by SFM. - J. Vac. Sci. Technol. A, 1996, v. 14 (3), pp. 1766-1769.
77. Chumanov G., Sokolov K., Cotton T.M. Unusual Extinction Spectra of Nanometer-Sized Silver Particles Arranged in Two-Dimensional Arrays. - J. Phys. Chem., 1996, v. 100, pp. 5166-5168.
78. Sokolov K.V., Byramova N.E., Mochalova L.V., Tuzikov A.B., Shiyan S.D., Bovin N.V., Nabiev I.R. Detection of sialic acid residues and studies of their organization in normal and tumor 0- acid glycoproteins as probed by surface-enhanced Raman spectroscopy. - Appl. Spectrosc., 1993, v. 47, N 5, p. 535-538.
79. Sokolov K.V., Khodorchenko P.V., Petukhov A.V., Nabiev I.R., Chumanov G., Cotton T.M. Contribution of short-range and classical electromagnetic mechanisms to surface-enhanced Raman scattering from several types of biomolecules adsorbed on cold-deposited island films. - Appl. Spectrosc., 1993, v. 47, p. 515-522.
80. U. Banin, C. J. Lee, A. A. Guzelian, A. V. Kadavanich, A. P. Alivisatos, W. Jaskolski, G. W. Bryant, Al. L. Efros, M. Rosen, "Size-dependent Electronic Level Structure ofInAs Nanocrystal Quantum Dots: Test of Multiband Effective Mass Theory," J. Chem. Phys. 109, 2306 (1998).
81. C. B. Murray, D. J. Norris, M. G. Bawendi, "Synthesis and Characterization of Nearly Monodisperse CdE (E = sulfur, selenium, tellurium) Semiconductor Nanocrystallites," J. Am. Chem. Soc. 115, 8706-8715 (1993).
82. T. D. Lacoste, X. Michalet, F. Pinaud, D.S. Chemla, A.P. Alivisatos, "Ultrahigh-Resolution Multicolor Colocalization of Single Fluorescent Probes," PNAS 2000, 97: 9461-66.
83. M. Bruchez Jr., M. Maronne, P. Gin, S. Weiss, A. P. Alivisatos, "Semiconductor Nanocrystals as Fluorescent Biological Labels," Science 1998, 281, 2013.
84. W. C. W. Chan, S. Nie, "Quantum dot bioconjugates for ultrasensitive nonisotopic detection," Science 1998, 281, 2016.
85. M. C. Willingham, in Methods in Molecular Biology, Vol. 115: Immunocytochemical Methods and Protocols, (Ed: L. C. Javois), Humana Press Inc., Totowa, NJ, 1999, ch 16.
86. M. E. Hemler, "VLA proteins in the integrin family-structures, functions and their role on leukocytes," Annu. Rev. Immunol. 1990, 8, 365
87. T. Yoshihara, N. Esumi, M. J. Humphries, S. Imashuku, "Unique expression of integrin fibronectin receptors in human neuroblastoma cell lines," Int. J. Cancer 1992, 51, 620.
88. M. D. Pierschbacher, E. Ruoslahti, "Cell attachment activity of fibronectin can be duplicated by small synthetic fragments of the molecule," Nature 1984, 309, 30.
89. Morris, K.N., Jensen, K.B., Julin, C.M., Weil, M. & Gold, L. High affinity ligands from in vitro selection: complex targets. Proc Natl Acad Sci U S A 95, 2902-2907. (1998).
90. Homann, M. & Goringer, H.U. Combinatorial selection of high affinity RNA ligands to live African trypanosomes. Nucleic Acids Res 27, 2006-2014. (1999).
91. Green, L. S., D. Jellinek, et al. (1995). "Nuclease-resistant nucleic acid ligands to vascular permeability factor/vascular endothelial growth factor." Chem Biol 2(10): 683-95.
92. E.J. Zeman and G.C. Schatz, "An Accurate Electromagnetic Theory Study of Surface Enhancement Factors for Ag, Au, Cu, Li, Na, Al, Ga, In, Zn, and Cd," J Phys Chem 1987, 91: 634-43.
93. R.M. Wilenzick, D.C. Russell, and R.H. Morriss, "Uniform Microcrystals of Platinum and Gold," J Chem Phys 1967, 47: 533-536.
94. S. Schneider, P. Halbig, H. Grau, and U. Nickel, "Reproducible Preparation of Silver Sols with Uniform Particle Size for Application in Surface-Enhanced Raman Spectroscopy," Photochemistry and Photobiology 60 (6): 605-10.
95. G. Frens, "Controlled Nucleation for the Regulation of the Particle Size in Monodisperse Gold Suspensions," Nature Physical Science 1973, 241: 20-22.
96. P.C. Lee and T.J. Meisel, "Adsorption and Surface-Enhanced Raman of Dyes on Silver and Gold Soles," J Phys Chem 1982, 86: 3391-95.
97. P. Hildebrandt and M. Stockburger, "Surface-Enhanced Resonance Raman spectroscopy of Rhodamine 6G Adsorbed on Colloidal Silver," J Phys Chem 1984, 88: 5935-5944.
98. G.R. Newman and J.A. Hobot, "Modem Acrylics for Post-Embedding Immunostaining Techniques," J Hictochem Cytochem 1987, 35 (9): 971-81.
99. S.K. Bhatia, L.C. Shriver-Lake, K.J. Prior, J.H. Georger, J.M. Calvert, R. Bredehorst, and F.S. Ligler, "Use of Thiol-Terminal Silanes and Heterobifunctional Crosslinkers for Immobilization of Antibodies on Silica Surfaces," Anal Biochem 1989, 178: 408-13.
100. L.A. Chrisey, G.U. Lee, and C.E. O'Ferrall, "Covalent Attachment of Synthetic DNA to Self-Assembled Monolayer Films," Nucleic Acid Research 1996, 24 (15): 3031-39.
101. R.A. Potyrailo, R.C. Conrad, A.D. Ellington, and G.M. Hieftje, "Adapting Selected Nucleic Acid Ligands (Aptamers) to Biosensors," Anal Chem 1998, 70: 3419-25.
102. M. Lee and D.R. Walt, "A Fiber-Optic Microarray Biosensor Using Aptamers as Receptors," Anal Biochem 2000, 282: 142-146.
103. Korgel and D. Fitzmaurice, "Small Angle X-Ray Scattering Study of Silver Nanocrystal Disorder-Order Phase Transitions," Phys. Rev. B, 59, 14191 (1999).
104. H. M. Chen, X. F. Huang, L. Xu, J. Xu, K. J. Chen, D. Feng, "Self-assembly and photoluminescence of CdS-mercaptoacetic clusters with internal clusters," Superlattices and Microstructures 2000, 27, 1.
105. J. D. Holmes, K. J. Ziegler, R. C. Doty, L. E. Pell, K. P. Johnston, B. A. Korgel, "Highly Luminescent Silicon Nanocrystals With Discrete Optical Transitions," Journal of the American Chemical Society, (2001) in press.
106. F. R. Miller, D. McEachern, and B. E. Miller, "Growth Regulation of Mouse Mammary Tumor Cells in Collagen Gel Cultures by Diffusible Factors Produced by Normal Mammary Gland Epithelium and Stromal Fibroblasts," Cancer Research, vol. 49, pp. 6091-6097, 1989.
107. R. M. Hoffman, "Three Dimensional Histoculture: Origin and Applications in Cancer Research," Cancer Cells, vol. 3, pp. 86-92, 1991
108. Brookner, C. K., M. Follen, I. Boiko, J. Galvan, S. Thomsen, A. Malpica, S. Suzuki, R. Lotan, and R.Richards-Kortum (2000) Autofluorescence patterns in short-term cultures of normal cervical tissue. Photochem.Photobiol. 71, 730-736.
109. R. Drezek, C. Brookner, I. Pavlova, I., I. Boiko, A. Malpica, R. Lotan, M. Follen, and R. Richards-Kortum, "Autofluorescence microscopy of fresh cervical tissue sections reveals alterations in tissue biochemistry with dysplasia," Photochem. Photobiol, accepted, 2001.
110. Durkin, Jaikumar, Richards-Kortum, "Dilute, Absorbing and Turbid Phantoms for Fluorescence Spectroscopy," Appl Spec, 47, pp. 2114-2121 (1993).
111. Durkin, Jaikumar, Ramanujam, Richards-Kortum, "Relation Between Fluorescence of Dilute and Turbid Samples," Appl Opt, 33, pp. 414-423 (1994).
112. Durkin and Richards-Kortum, "Methods to Determine Chromophore Concentrations from Fluorescence Spectra," Las Sury Med, 19, pp. 75-89 (1996).
113. Richards-Kortum, Durkin, Zeng, "Description and Performance of a Fiber Optic Confocal Spectrometer," App Spec, 48, pp. 350-355 (1994).
114. Dunn, Smithpeter, Welch, Richards-Kortum, "Sources of Contrast in Confocal Reflectance Imaging," App Op, 35, pp. 3441-3446 (1996).
115. Smithpeter, Dunn, Welch, Richards-Kortum, "Penetration Depth Limits of in vivo Confocal Reflectance Imaging," Appl Opt, 37, pp. 2749-2754 (1998).
116. Dunn, Smithpeter, Welch, Richards-Kortum, "FDTD Simulation of Light Scattering from Single Cells," J Bio Optics, 2, pp. 262-266 (1997).
117. U.S. Patent No. 6,258,576
118. U.S. Patent No. 6,241,662
119. U.S. Patent No. 6,187,289
120. U.S. Patent No. 6,135,965
121. U.S. Patent No. 6,111,095
122. U.S. Patent No. 6,095,982
123. U.S. Patent No. 5,991,653
124. U.S. Patent No. 5,929,985
125. U.S. Patent No. 5,920,399
126. U.S. Patent No. 5,861,501
127. U.S. Patent No. 5,842,995
128. U.S. Patent No. 5,792,613
129. U.S. Patent No. 5,780,449
130. U.S. Patent No. 5,756,291
131. U.S. Patent No. 5,699,795
132. U.S. Patent No. 5,697,373
133. U.S. Patent No. 5,631,146
134. U.S. Patent No. 5,623,932
135. U.S. Patent No. 5,612,540
136. U.S. Patent No. 5,562,100
137. U.S. Patent No. 6,275,726
138. Huang, D., Swanson, E. A., Lin, C. P., Schuman, J. S., Stinson, W. G., Chang, W., Hee, M. R., Flotte, T., Gregory, K., & Puliafito, C. A. (1991) Science 254, 1178-1181.
139. Rajadhyaksha, M., Grossman, M., Esterowitz, D., Webb, R.H., & Anderson, R.R. (1995) J. Invest. Dermatol. 104, 946-952.
140. Collier, T., Lacy, A., Malpica, A., Follen, M., & Richards-Kortum, R. (2002) Academic Radiology 9, 504.
141. Teamey, G.J., Brezinski, M. E., Bouma, B. E., Boppart, S. A., Pitris, C., Southern, J. F., & Fujimoto, J. G. (1997) Science 276, 2037-2039.
142. Liang, C., Sung, K.-B., Richards-Kortum, R., & Descour, M.R. (2001) Optics Express 9, 821-830.
143. Drezek, R., Collier, T., Brookner, C. K., Malpica, A., Lotan, R., Richards-Kortum, R., & Follen, M. (2000) AM. J. Obstet. Gynecol., 182, 1135-1139.
144. Horisberger, M. (1981) Scanning Electron Microscopy 11, 9-31.
145. Geoghegan, W.D. & Ackerman, G.A. (1977) J of Histochemistry and Cytochemistry 25, 1187-1200.
146. Mulvaney, P. (1996) Langmuir 12, 788-800.
147. Carpenter, G. (1987) Ann. Rev. Biochem. 56, 881-914.
148. Maruo, T., Yamasaki, M., Ladines-Llave, C.A., & Mochizuki, M. (1992) Cancer 69(5), 1182-1187.
149. Pfeifer, D., Stellwag, B., Pfeifer, A., Borlinghaus, P., Meier, W. (1989) Gynecol. Oncol. 33, 146-150.
150. Todd, R. & Wong, D.T.W. (1999) Histol. Histopathol. 14, 491-500.
151. Wobus, M., Kuns, R., Wolf, C., Horn, L.-C., Koehler, U., Sheyn, I., Wemess, B. A., & Sherman, L. S. (2001) Gynecol. Oncol. 83, 227-234.
152. Saltzman, M.W., Parkhurst, M.R., Parsons-Wingerter, P., & Zhu, W.-H. (1992) Annals New York Academy Sciences 665, 259-273.
153. Sokolov, K., Galvan, J., Myakov, A., Lacy, A., Lotan, R., & Richards-Kortum, R. (2002) J Biomedical Optics 7 (1), 148-156.
154. Drezek, R., Brookner, C., Pavlova, I., Boiko, I., Malpica, A., Lotan, R., Follen, M., & Richards-Kortum, R. (2001) Photochem. Photobiol. 73(6), 636-641.
155. Frens, G. (1973) Nature Physical Science 241, 20-22.
156. Sokolov, K., Drezek, R., Gossage, K., & Richards-Kortum, R. (1999) Optics Express 5 (13), 302-317.
157. Monaghan, P., Ormerod, M.G., & O'Hare, M.J. (1990) Int. J. Cancer 46, 935-943.
158. Grizzle, W.E., Manne, U., Jhala, N.C. & Weiss, H.L. (2001) Archives of Pathology and Laboratory Medicine 125(1), 91-98.
159. Ntziachristos, V. & Chance, B. (2001) Breast Cancer Research 3, 41-46.
160. Schultz, S., Smith, D., Mock, J., & Schultz, D. (2000) Proc. Natl. Acad. Sci. U.S.A. 97(3), 996-1001.
161. Abrams, M.J. & Murrer, B.A. (1993) Science 261, 725-730.
162. Berkower, I. (1996) Current Opinion in Biotechnology 7(6), 622-628.
163. Holliger, P. & Bohlen, H. (1999) Cancer and Metastasis Reviews 18(4), 411-419.
164. Weiner, L.M. (1999) Seminars in Oncology 26(4 Suppl 12), 43-51.
165. Organization, W.H., Cancer Control. 1996.
166. Cotran, R.S., Vinay Kumar, Tucker Collins, Pathologic basis of disease. 6 ed. 1999, Philadelphia: Saunders.
167. Fujimoto, J.G., et al., Optical coherence tomography: an emerging technology for biomedical imaging and optical biopsy. Neoplasia, 2000. 2(1-2): p. 9-25.
168. Richards-Kortum, R. and E. Sevick-Muraca, Quantitative optical spectroscopy for tissue diagnosis. Annual review of physical chemistry, 1996. 47: p. 555-606.
169. Wagnières, G.A., W.M. Star, and B.C. Wilson, In vivo fluorescence spectroscopy and imaging for oncological applications. Photochemistry and photobiology, 1998. 68(5): p. 603-32.
170. Pisani, P., F. Bray, and D.M. Parkin, Estimates of the world-wide prevalence of cancer for 25 sites in the adult population. International journal of cancer, 2002. 97(1): p. 72-81.
171. Kurman, R.J., et al., Interim guidelines for management of abnormal cervical cytology. The 1992 National Cancer Institute Workshop. JAMA : the journal of the American Medical Association, 1994. 271(23): p. 1866-9.
172. Ollayos, C.W. and K.A. Swogger, Abnormal cervical smears in the military recruit population. Military medicine, 1995. 160(11): p. 577-8.
173. Koss, L.G., The Papanicolaou test for cervical cancer detection. A triumph and a tragedy. JAMA : the journal of the American Medical Association, 1989. 261(5): p. 737-43.
174. Society, A.C., Cancer Facts and Figures, Publication No. 93-400M, No. 5008-03. 1993.
175. Blair, E.A. and D.L. Callender, Head and neck cancer. The problem. Clinics in plastic surgery, 1994. 21(1): p. 1-7.
176. Silverman, S., Jr., M. Gorsky, and F. Lozada, Oral leukoplakia and malignant transformation. A follow-up study of 257 patients. Cancer, 1984. 53(3): p. 563-8.
177. Greenlee, R.T., et al., Cancer statistics, 2001. CA: a cancer journal for clinicians, 2001. 51(1): p. 15-36.
178. Welch, H.G., L.M. Schwartz, and S. Woloshin, Are increasing 5-year survival rates evidence of success against cancer? JAMA : the journal of the American Medical Association, 2000. 283(22): p. 2975-8.
179. McCrory DC, M.D., Bastian L, Datta S, Hasselblad V, Hickey J, Myers E, et al, Evaluation of cervical cytology: Evidence Report/Technology Assessment. 1999, Agency for Health Care Policy and Research (AHCPR). Rockville, MD.
180. Incze, J., et al., Premalignant changes in normal appearing epithelium in patients with squamous cell carcinoma of the upper aerodigestive tract. American journal of surgery, 1982. 144(4): p. 401-405.
181. Brennan, J.A. and D. Sidransky, Molecular staging of head and neck squamous carcinoma. Cancer and metastasis reviews, 1996. 15(1): p. 3-10.
182. Kelloff, G.J., et al., Surrogate endpoint biomarkers for phase II cancer chemoprevention trials. Journal of cellular biochemistry Supplement, 1994. 19: p. 1-9.
183. Daly, M.B., The chemoprevention of cancer: directions for the future. Cancer epidemiology, biomarkers & prevention : a publication of the American Association for Cancer Research, cosponsored by the American Society of Preventive Oncology, 1993. 2(6): p. 509-12.
184. Armstrong, W.B. and F.L. Meyskens, Jr., Chemoprevention of head and neck cancer. Otolaryngology--head and neck surgery : official journal of American Academy of Otolaryngology-Head and Neck Surgery, 2000. 122(5): p. 728-35.
185. Kelloff, G.J., et al., Perspectives on surrogate end points in the development of drugs that reduce the risk of cancer. Cancer epidemiology, biomarkers & prevention : a publication of the American Association for Cancer Research, cosponsored by the American Society of Preventive Oncology, 2000. 9(2): p. 127-37.
186. Geyer, C., V. Papadimitrakopoulou, and W.K. Hong, Chemoprevention in head and neck cancer: basic science and clinical application. Seminars in radiation oncology, 1998. 8(4): p. 292-301.
187. Sporn, M.B., The war on cancer. Lancet, 1996. 347(9012): p. 1377-81.
188. Sporn, M.B. and D.L. Newton, Chemoprevention of cancer with retinoids. Federation proceedings, 1979. 38(11): p. 2528-34.
189. Greenwald, P., et al., Chemoprevention. CA: a cancer journal for clinicians, 1995. 45(1): p. 31-49.
190. Li, N., et al., The chemopreventive effects of tea on human oral precancerous mucosa lesions. Proceedings of the Society for Experimental Biology and Medicine Society for Experimental Biology and Medicine, 1999. 220(4): p. 218-24.
191. Papadimitrakopoulou, V.A., et al., Biochemoprevention for dysplastic lesions of the upper aerodigestive tract. Archives of otolaryngology -- head & neck surgery, 1999. 125(10): p. 1083-9.
192. Mao, L., et al., Phenotype and genotype of advanced premalignant head and neck lesions after chemopreventive therapy. Journal of the National Cancer Institute, 1998. 90(20): p. 1545-51.
193. Lam S, I.J., Zheng Y, Coldman A, MacAulay CE, Hawk E, Kelloff G, Gazdar AF, Sex-related differences in bronchial epithelial changes associated with tobacco smoking. J Natl Cancer Inst, 1999. 91: p. 691-696.
194. Wistuba II, L.S., Behrens C, Virmani AK, Fong KM, LeRiche JC, Samet J, Srivastava S, Minna JD, Gazdar AF, Molecular damage in the bronchial epithelium of current and former smokers. J Natl Cancer Inst, 1997. 89: p. 1366-1373.
195. Takaha, N., et al., High mobility group protein I(Y): a candidate architectural protein for chromosomal rearrangements in prostate cancer cells. Cancer Res, 2002.
196. Condeelis, J., The biochemistry of animal cell crawling, in Motion Analysis of Living Cells, D. Soll, Editor. 1988, Wiley Liss, Inc. p. 85-100.
197. Bailly, M., et al., Relationship between Arp2/3 complex and the barbed ends of actin filaments at the leading edge of carcinoma cells after epidermal growth factor stimulation. J Cell Biol, 1999.
198. Condeelis, J.S., J. Wyckoff, and J.E. Segall, Imaging of cancer invasion and metastasis using green fluorescent protein. European journal of cancer, 2000. 36(13 Spec No): p. 1671-80.
199. Gohongi, T., et al., Tumor-host interactions in the gallbladder suppress distal angiogenesis and tumor growth: involvement of transforming growth factor beta1. Nature medicine, 1999. 5(10): p. 1203-8.
200. Brown, E.B., et al., In vivo measurement of gene expression, angiogenesis and physiological function in tumors using multiphoton laser scanning microscopy. Nature medicine, 2001. 7(7): p. 864-8.
201. Carmeliet, P., Jain, RK, Angiogenesis in cancer and other diseases. Nature(London), 2000. 407(6801): p. 249-257.
202. van de Vijver, M.J., et al., A Gene-Expression Signature as a Predictor of Survival in Breast Cancer. The New England journal of medicine, 2002. 347(25): p. 1999-2009.
203. Ang, K.K., et al., Impact of Epidermal Growth Factor Receptor Expression on Survival and Pattern of Relapse in Patients with Advanced Head and Neck Carcinoma. Cancer research, 2002. 62(24): p. 7350-7356.
204. Hanahan, D. and R.A. Weinberg, The hallmarks of cancer. Cell, 2000. 100(1): p. 57-70.
205. Lin, C.R., et al., Expression cloning of human EGF receptor complementary DNA: gene amplification and three related messenger RNA products in A431 cells.
206. Ulrich, A., Nature, 1984. 309: p. 418-425.
207. Xu, Y.H., et al., Human epidermal growth factor receptor cDNA is homologous to a variety of RNAs overproduced in A431 carcinoma cells. Nature, 1984. 309(5971): p. 806-10.
208. Kersemaekers, A.M., et al., Oncogene alterations in carcinomas of the uterine cervix: overexpression of the epidermal growth factor receptor is associated with poor prognosis. Clinical cancer research : an official journal of the American Association for Cancer Research, 1999. 5(3): p. 577-86.
209. Chang, J.L., et al., The expression of type I growth factor receptors in the squamous neoplastic changes of uterine cervix. Gynecologic oncology, 1999. 73(1): p. 62-71.
210. Nishioka, T., et al., Prognostic significance of c-erbB-2 protein expression in carcinoma of the cervix treated with radiotherapy. Journal of cancer research and clinical oncology, 1999. 125(2): p. 96-100.
211. Mathevet, P., L. Frappart, and W. Hittelman, Dysplasies du col utérin: étude des expressions des gènes Rb et p53, et corrélation avec l'activité mitotique. Gynecologie, obstetrique & fertilite, 2000. 28(1): p. 44-50.
212. Shin, D.M., et al., Expression of epidermal growth factor receptor, polyamine levels, ornithine decarboxylase activity, micronuclei, and transglutaminase I in a 7,12-dimethylbenz(a)anthracene-induced hamster buccal pouch carcinogenesis model. Cancer research, 1990. 50(8): p. 2505-10.
213. Todd, R. and D.T. Wong, Epidermal growth factor receptor (EGFR) biology and human oral cancer. Histology and histopathology, 1999. 14(2): p. 491-500.
214. Averbuch, S.D., Lung cancer prevention: retinoids and the epidermal growth factor receptor-a phoenix rising? Clinical cancer research : an official journal of the American Association for Cancer Research, 2002. 8(1): p. 1-3.
215. Sen, S., et al., A comparative study of telomerase activity in sputum, bronchial washing and biopsy specimens of lung cancer. Lung cancer, 2001. 33(1): p. 41-9.
216. Thomas, G.T., M.P. Lewis, and P.M. Speight, Matrix metalloproteinases and oral cancer. Oral oncology, 1999. 35(3): p. 227-33.
217. Garzetti, G.G., et al., Microinvasive cervical carcinoma and cervical intraepithelial neoplasia: biologic significance and clinical implications of 72-kDa metalloproteinase immunostaining. Gynecologic oncology, 1996. 61(2): p. 197-203:
218. Talvensaari, A., et al., Matrix metalloproteinase 2 immunoreactive protein appears early in cervical epithelial dedifferentiation. Gynecologic oncology, 1999. 72(3): p. 306-11.
219. Daneri-Navarro, A., et al., Proteolytic activity in extracts of invasive cervical carcinoma and precursor lesions. Biomedicine & pharmacotherapy, 1995. 49(6): p. 304-10.
220. McCluggage, W.G., P. Maxwell, and H. Bharucha, Immunohistochemical detection of metallothionein and MIB1 in uterine cervical squamous lesions. International journal of gynecological pathology : official journal of the International Society of Gynecological Pathologists, 1998. 17(1): p. 29-35.
221. Davidson, B., et al., Expression of Matrix Metalloproteinase-9 in Squamous Cell Carcinoma of the Uterine Cervix--Clinicopathologic Study Using Immunohistochemistry and mRNAin SituHybridization. Gynecologic Oncology, 1999. 72(3): p. 380-386.
222. Sienel, W., et al., Prognostic impact of matrix metalloproteinase-9 in operable non-small cell lung cancer. International journal of cancer, 2003. 103(5): p. 647-651.
223. Passlick, B., et al., Overexpression of matrix metalloproteinase 2 predicts unfavorable outcome in early-stage non-small cell lung cancer. Clinical cancer research : an official journal of the American Association for Cancer Research, 2000. 6(10): p. 3944-8.
224. Hrabec, E., et al., Activity of type IV collagenases (MMP-2 and MMP-9) in primary pulmonary carcinomas: a quantitative analysis. Journal of cancer research and clinical oncology, 2002. 128(4): p. 197-204.
225. Adachi, M., et al., Significance of integrin alpha5 gene expression as a prognostic factor in node-negative non-small cell lung cancer. Clinical cancer research : an official journal of the American Association for Cancer Research, 2000. 6(1): p. 96-101.
226. Chatterjee, N. and A. Chatterjee, Role of alphavbeta3 integrin receptor in the invasive potential of human cervical cancer (SiHa) cells. Journal of environmental pathology, toxicology and oncology : official organ of the International Society for Environmental Toxicology and Cancer, 2001. 20(3): p. 211-21.
227. Hamidi, S., et al., Expression of alpha(v)beta6 integrin in oral leukoplakia. British journal of cancer, 2000. 82(8): p. 1433-40.
228. Pomper, M.G., Molecular imaging: an overview. Academic radiology, 2001. 8(11): p. 1141-53.
229. Rajadhyaksha, M., et al., In Vivo Confocal Scanning Laser Microscopy of Human Skin: Melanin Provides Strong Contrast. Journal of Investigative Dermatology, 1995. 104(6): p. 946-952.
230. Rajadhyaksha, M., R.R. Anderson, and R.H. Webb, Video-rate Confocal Scanning Laser Microscope for Imaging Human Tissues In Vivo. Applied Optics, 1999. 38(10): p. 2105-2115.
231. Rajadhyaksha, M., et al., In Vivo Confocal Scanning Laser Microscopy of Human Skin II: Advances in Instrumentation and Comparison with Histology. Journal of Investigative Dermatology, 1999. 113(3): p. 293-303.
232. Rajadhyaksha, M., et al., Confocal examination of nonmelanoma cancers in thick skin excisions to potentially guide mohs micrographic surgery without frozen histopathology. The Journal of investigative dermatology, 2001. 117(5): p. 1137-43.
233. Rajadhyaksha, M., et al., Confocal Cross-Polarized Imaging of Skin Cancers to Potentially Guide Mohs Micrographic Surgery. Optics & Photonics News, 2001: p. 30.
234. Selkin, B., et al., In vivo confocal microscopy in dermatology. Dermatologic clinics, 2001. 19(2): p. 369-77, ix-x.
235. White, W.M., et al., Noninvasive Imaging of Human Oral Mucosa In Vivo by Confocal Reflectance Microscopy. Laryngoscope, 1999. 109(10): p. 1709-1717.
236. Gonzalez, S., et al., Confocal Reflectance Imaging of Folliculitis In Vivo: Correlation with Routine Histology. Journal of Cutaneous Pathology, 1999. 26: p. 201-205.
237. González, S., et al., Allergic contact dermatitis: correlation of in vivo confocal imaging to routine histology. Journal of the American Academy of Dermatology, 1999. 40(5) Pt 1: p. 708-13.
238. González, S., et al., Characterization of psoriasis in vivo by reflectance confocal microscopy. Journal of medicine, 1999. 30(5-6): p. 337-56.
239. González, S., et al., In vivo abnormal keratinazation in Darier-White's disease as viewed by real-time confocal imaging. Journal of cutaneous pathology, 1999. 26(10): p. 504-8.
240. González, S., et al., Confocal imaging of sebaceous gland hyperplasia in vivo to assess efficacy and mechanism of pulsed dye laser treatment. Lasers in surgery and medicine, 1999. 25(1): p. 8-12.
241. Huzaira, M., et al., Topographic variations in normal skin, as viewed by in vivo reflectance confocal microscopy. The Journal of investigative dermatology, 2001. 116(6): p. 846-52.
242. Langley, R.G., et al., Confocal scanning laser microscopy of benign and malignant melanocytic skin lesions in vivo. Journal of the American Academy of Dermatology, 2001. 45(3): p. 365-76.
243. Collier, T., et al., Near Real-Time Confocal Microscopy of Amelanotic Tissue: Detection of Dysplasia in ex Vivo Cervical Tissue. Academic Radiology, 2002. 9(5): p. 504-512.
244. Collier, T., et al., Near Real Time Confocal Microscopy of Amelanotic Tissue: Dynamics of Aceto-Whitening Enable Nuclear Segmentation. Optics Express, 2000. 6(2): p. 40-48.
245. Collier, T., et al. Fiber-Optic Confocal Microscope for Biological Imaging. in Conference on Lasers and Electro-Optics. 1998. San Francisco, CA: Institute of Electrical and Electronics Engineers.
246. Das, A., et al., High-resolution endoscopic imaging of the GI tract: a comparative study of optical coherence tomography versus high-frequency catheter probe EUS. Gastrointestinal endoscopy, 2001. 54(2): p. 219-24.
247. Pitris, C., et al. Cellular and Neoplastic Tissue Imaging with Optical Coherence Tomography. in Conference on Lasers and Electro-Optics Europe. 1998. San Francisco, CA: Institute of Electrical and Electronics Engineers.
248. Pitris, C., et al., High-resolution imaging of gynecologic neoplasms using optical coherence tomography. Obstetrics and gynecology, 1999. 93(1): p. 135-9.
249. Jesser, C.A., et al., High resolution imaging of transitional cell carcinoma with optical coherence tomography: feasibility for the evaluation of bladder pathology. The British journal of radiology, 1999. 72(864): p. 1170-6.
250. Li, X.D., et al., Optical coherence tomography: advanced technology for the endoscopic imaging of Barrett's esophagus. Endoscopy, 2000. 32(12): p. 921-30.
251. Bouma, B.E., et al., High-resolution imaging of the human esophagus and stomach in vivo using optical coherence tomography. Gastrointestinal endoscopy, 2000. 51(4) Pt 1: p. 467-74.
252. Porter, D.A., et al., A SAGE (serial analysis of gene expression) view of breast tumor progression. Cancer research, 2001. 61(15): p. 5697-702.
253. Polyak, K. and G.J. Riggins, Gene discovery using the serial analysis of gene expression technique: implications for cancer research. Journal of clinical oncology : official journal of the American Society of Clinical Oncology, 2001. 19(11): p. 2948-58.
254. Grigorenko, E., ed. DNA Arrays: Technologies and Experimental Strategies. 2002, CRC Press: Boca Raton, FL.
255. Hunt, S.P. and F.J. Livesey, Functional Genomics: A Practical Approach. 2000, Oxford: Oxford University Press.
256. Miglani, G.S., Advanced Genetics. 2002, Pangboume, U. K.: Alpha Science International Ltd.
257. Sutcliffe, J.G., et al., TOGA: an automated parsing technology for analyzing expression of nearly all genes. Proceedings of the National Academy of Sciences of the United States of America, 2000. 97(5): p. 1976-81.
258. Diehn, M. and D.A. Relman, Comparing functional genomic datasets: lessons from DNA microarray analyses of host-pathogen interactions. Current opinion in microbiology, 2001. 4(1): p. 95-101.
259. Green, C.D., et al., Open systems: panoramic views of gene expression. Journal of immunological methods, 2001. 250(1-2): p. 67-79.
260. Brent, R., Genomic biology. Cell, 2000. 100(1): p. 169-83.
261. Rampal, J.B., ed. DNA Arrays: Methods and Protocols. Methods in Molecular Biology. Vol. 170. 2001, Humana Press: Totowa, N. J.
262. Leslie, R.A. and H.A. Robertson, eds. Differential Display: A Practical Approach. Practical Approach Series. 2000, Oxford University Press: Oxford, U. K.
263. Chen, B.Y. and H.W. Janes, eds. PCR Cloning Protocols: From Molecular Cloning to Genetic Engineering. 2nd ed. Methods in molecular biology. Vol. 192. 2002, Humana Press: Totowa, N.J.
264. Kononen, J., et al., Tissue microarrays for high-throughput molecular profiling of tumor specimens. Nature medicine, 1998. 4(7): p. 844-7.
265. Datson, N.A., et al., MicroSAGE: a modified procedure for serial analysis of gene expression in limited amounts of tissue. Nucleic acids research, 1999. 27(5): p. 1300-7.
266. St Croix, B., et al., Genes expressed in human tumor endothelium. Science, 2000. 289(5482): p. 1197-202.
267. Hibi, K., et al., Advances in Brief - Serial Analysis of Gene Expression in Non-Small Cell Lung Cancer. Cancer research : the official organ of the American Association for Cancer Research, 1998. 58(24): p. 5690-5694.
268. Lal, A., et al., Advances in Brief - A Public Database for Gene Expression in Human Cancers. Cancer research : the official organ of the American Association for Cancer Research, Inc. 59, no. 21, (1999): 5403 (5 pages), 1999. 59(21): p. 5403-5407.
269. Ewing, R., O. Poirot, and J.M. Claverie, Comparative analysis of the Arabidopsis and rice expressed sequence tag (EST) sets. In silico biology, 1999. 1(4): p. 197-213.
270. Ewing, B. and P. Green, Analysis of expressed sequence tags indicates 35,000 human genes. Nature genetics, 2000. 25(2): p. 232-4.
271. Kolonin, M., R. Pasqualini, and W. Arap, Molecular addresses in blood vessels as targets for therapy. Current opinion in chemical biology, 2001. 5(3): p. 308-13.
272. Arap, W., et al., Targeting the prostate for destruction through a vascular address. Proceedings of the National Academy of Sciences of the United States of America, 2002. 99(3): p. 1527-31.
273. Smith, G.P. and J.K. Scott, Libraries of peptides and proteins displayed on filamentous phage.
274. Arap, W., R. Pasqualini, and E. Ruoslahti, Chemotherapy targeted to tumor vasculature. Current opinion in oncology, 1998. 10(6): p. 560-5.
275. Brooks, P.C., R.A. Clark, and D.A. Cheresh, Requirement of vascular integrin alpha v beta 3 for angiogenesis. Science, 1994. 264(5158): p. 569-71.
276. Smith, G.P., Filamentous fusion phage: novel expression vectors that display cloned antigens on the virion surface. Science, 1985. 228(4705): p. 1315-7.
277. Smith, G.P. and J.K. Scott, Libraries of peptides and proteins displayed on filamentous phage. Methods Enzymol, 1993. 217: p. 228-57.
278. Arap, W., R. Pasqualini, and E. Ruoslahti, Cancer treatment by targeted drug delivery to tumor vasculature in a mouse model. Science, 1998. 279(5349): p. 377-80.
279. Curnis, F., et al., Enhancement of tumor necrosis factor alpha antitumor immunotherapeutic properties by targeted delivery to aminopeptidase N (CD13). Nat Biotechnol, 2000. 18(11): p. 1185-90.
280. Ellerby, H.M., et al., Anti-cancer activity of targeted pro-apoptotic peptides. Nat Med, 1999. 5(9): p. 1032-8.
281. Giordano, R.J., et al., Biopanning and rapid analysis of selective interactive ligands. Nat Med, 2001. 7(11): p. 1249-53.
282. Hammes, H.P., et al., Subcutaneous injection of a cyclic peptide antagonist of vitronectin receptor-type integrins inhibits retinal neovascularization. Nat Med, 1996. 2(5): p. 529-33.
283. Hashizume, H., et al., Openings between defective endothelial cells explain tumor vessel leakiness. Am J Pathol, 2000. 156(4): p. 1363-80.
284. Koivunen, E., et al., Identification of receptor ligands with phage display peptide libraries. J Nucl Med, 1999. 40(5): p. 883-8.
285. McDonald, D.M., G. Thurston, and P. Baluk, Endothelial gaps as sites for plasma leakage in inflammation. Microcirculation, 1999. 6(1): p. 7-22.
286. Watson, C.A., et al., Variability among human umbilical vein endothelial cultures. Science, 1995. 268(5209): p. 447-8.
287. Rajotte, D. and E. Ruoslahti, Membrane dipeptidase is the receptor for a lung-targeting peptide identified by in vivo phage display. J Biol Chem, 1999. 274(17): p. 11593-8.
288. Pasqualini, R., et al., Aminopeptidase N is a receptor for tumor-homing peptides and a target for inhibiting angiogenesis. Cancer Res, 2000. 60(3): p. 722-7.
289. Boiko, I.V., et al., Epidermal growth factor receptor expression in cervical intraepithelial neoplasia and its modulation during an alpha-difluoromethylomithine chemoprevention trial. Clinical cancer research : an official journal of the American Association for Cancer Research, 1998. 4(6): p. 1383-91.
290. Boiko, I.V., et al., DNA image cytometric measurement as a surrogate end point biomarker in a phase I trial of alpha-difluoromethylomithine for cervical intraepithelial neoplasia. Cancer epidemiology, biomarkers & prevention : a publication of the American Association for Cancer Research, cosponsored by the American Society of Preventive Oncology, 1997. 6(10): p. 849-55.
291. Cho, S.H., et al., Sialyl-Tn antigen expression occurs early during human mammary carcinogenesis and is associated with high nuclear grade and aneuploidy. Cancer research, 1994. 54(24): p. 6302-5.
292. Dhingra, K., et al., Strategies for the application of biomarkers for risk assessment and efficacy in breast cancer chemoprevention trials. Journal of cellular biochemistry Supplement, 1993. 17G: p. 37-43.
293. Follen, M., et al., A randomized clinical trial of 4-hydroxyphenylretinamide for high-grade squamous intraepithelial lesions of the cervix. Clinical cancer research : an official journal of the American Association for Cancer Research, 2001. 7(11): p. 3356-65.
294. Hamada, K., et al., Adenovirus-mediated transfer of a wild-type p53 gene and induction of apoptosis in cervical cancer. Cancer research, 1996. 56(13): p. 3047-54.
295. Hittelman, W.N., Clones and subclones in the lung cancer field. Journal of the National Cancer Institute, 1999. 91(21): p. 1796-9.
296. Hittelman, W.N., Genetic instability in epithelial tissues at risk for cancer. Annals of the New York Academy of Sciences, 2001. 952: p. 1-12.
297. Hittelman, W.N., Head and Neck Field Carcinogenesis, in Head and Neck Cancers, J.F. Ensley, et al., Editors. 2003, Academic Press: San Diego.
298. Hittelman, W.N., et al., Detection of chromosome instability of tissue fields at risk: in situ hybridization. Journal of cellular biochemistry Supplement, 1996. 25: p. 57-62.
299. Hittelman, W.N., J.M. Kurie, and S. Swisher, Molecular Events in Lung Cancer and Implications for Prevention and Therapy, in Anderson Associates Monograph on Lung Cancer, F. Fosella, R. Komaki, and J. Putnam, J., Editors. 2002. p. 280-298.
300. Hittelman, W.N., et al., Persistent Genetic Instability Despite Decreased Proliferation in Human Lung Tissue following Smoking Cessation. Proceedings of the AACR, 1998. 39(336).
301. Hittelman, W.N., et al., Early genetic changes during upper aerodigestive tract tumorigenesis. Journal of cellular biochemistry Supplement, 1993. 17F: p. 233-6.
302. Hu, W., et al., Progressive dysregulation of proliferation during cervical carcinogenesis as measured by MPM-2 antibody staining. Cancer epidemiology, biomarkers & prevention : a publication of the American Association for Cancer Research, cosponsored by the American Society of Preventive Oncology, 1997. 6(9): p. 711-8.
303. Kim, J., et al., Chromosome polysomy and histological characteristics in oral premalignant lesions. Cancer epidemiology, biomarkers & prevention : a publication of the American Association for Cancer Research, cosponsored by the American Society of Preventive Oncology, 2001. 10(4): p. 319-25.
304. Kurie, J.M., et al., Increased epidermal growth factor receptor expression in metaplastic bronchial epithelium. Clinical cancer research : an official journal of the American Association for Cancer Research, 1996. 2(10): p. 1787-93.
305. Lee, J.J., et al., Predicting cancer development in oral leukoplakia: ten years of translational research. Clinical cancer research : an official journal of the American Association for Cancer Research, 2000. 6(5): p. 1702-10.
306. Lee, J.J., et al., Long-term impact of smoking on lung epithelial proliferation in current and former smokers. Journal of the National Cancer Institute, 2001. 93(14): p. 1081-8.
307. Lee, J.S., et al., Detection of chromosomal polysomy in oral leukoplakia, a premalignant lesion. Journal of the National Cancer Institute, 1993. 85(23): p. 1951-4.
308. Lippman, S.M., et al., Retinoic acid and interferon combination studies in human cancer. European journal of cancer, 1993. 29A Suppl 5: p. S9-13.
309. Lippman, S.M., et al., Recent advances in cancer chemoprevention. Cancer cells, 1991. 3(2): p. 59-65.
310. Lippman, S.M., et al., p53 and retinoid chemoprevention of oral carcinogenesis. Cancer research, 1995. 55(1): p. 16-9.
311. Mao, L., et al., Frequent microsatellite alterations at chromosomes 9p21 and 3p14 in oral premalignant lesions and their value in cancer risk assessment. Nature medicine, 1996. 2(6): p. 682-5.
312. Mitchell, M.F., et al., Chemoprevention trials and surrogate end point biomarkers in the cervix. Cancer, 1995. 76(10) Suppl: p. 1956-77.
313. Mitchell, M.F., et al., The natural history of cervical intraepithelial neoplasia: an argument for intermediate endpoint biomarkers. Cancer epidemiology, biomarkers & prevention : a publication of the American Association for Cancer Research, cosponsored by the American Society of Preventive Oncology, 1994. 3(7): p. 619-26.
314. Mitchell, M.F., et al., Chemoprevention trials in the cervix: design, feasibility, and recruitment. Journal of cellular biochemistry Supplement, 1995. 23: p. 104-12.
315. Mitchell, M.F., et al., Polyamine measurements in the uterine cervix. Journal of cellular biochemistry Supplement, 1997. 28-29: p. 125-32.
316. Mitchell, M.F., et al., Phase I dose de-escalation trial of alpha-difluoromethylomithine in patients with grade 3 cervical intraepithelial neoplasia. Clinical cancer research : an official journal of the American Association for Cancer Research, 1998. 4(2): p. 303-10.
317. Nishikawa, K., et al., Resistance of human cervical carcinoma cells to tumor necrosis factor correlates with their increased sensitivity to cisplatin: evidence of a role for DNA repair and epidermal growth factor receptor. Cancer research, 1992. 52(17): p. 4758-65.
318. Papadimitrakopoulou, V., et al., Frequent inactivation of p16INK4a in oral premalignant lesions. Oncogene, 1997. 14(15): p. 1799-803.
319. Poulin, N., et al., Nuclear morphometry as an intermediate endpoint biomarker in chemoprevention of cervical carcinoma using alpha-difluoromethylornithine. Cytometry : the journal of the Society for Analytical Cytology, 1999. 38(5): p. 214-23.
320. Shin, D.M., et al., p53 protein accumulation and genomic instability in head and neck multistep tumorigenesis. Cancer epidemiology, biomarkers & prevention : a publication of the American Association for Cancer Research, cosponsored by the American Society of Preventive Oncology, 2001. 10(6): p. 603-9.
321. Shin, D.M., et al., Activation of ribosomal protein S2 gene expression in a hamster model of chemically induced oral carcinogenesis. Carcinogenesis, 1993. 14(1): p. 163-6.
322. Shin, D.M., W.N. Hittelman, and W.K. Hong, Biomarkers in upper aerodigestive tract tumorigenesis: a review. Cancer epidemiology, biomarkers & prevention : a publication of the American Association for Cancer Research, cosponsored by the American Society of Preventive Oncology, 1994. 3(8): p. 697-709.
323. Shin, D.M., et al., Activation of p53 gene expression in premalignant lesions during head and neck tumorigenesis. Cancer research, 1994. 54(2): p. 321-6.
324. Shin, D.M., et al., Dysregulation of epidermal growth factor receptor expression in premalignant lesions during head and neck tumorigenesis. Cancer research, 1994. 54(12): p. 3153-9.
325. Shin, D.M., et al., Sequential increases in proliferating cell nuclear antigen expression in head and neck tumorigenesis: a potential biomarker. Journal of the National Cancer Institute, 1993. 85(12): p. 971-8.
326. Shin, D.M., et al., Accumulation of p53 protein and retinoic acid receptor beta in retinoid chemoprevention. Clinical cancer research : an official journal of the American Association for Cancer Research, 1997. 3(6): p. 875-80.
327. Singletary, E., et al., Novel translational model for breast cancer chemoprevention study: accrual to a presurgical intervention with tamoxifen and N-[4-hydroxyphenyl] retinamide. Cancer epidemiology, biomarkers & prevention : a publication of the American Association for Cancer Research, cosponsored by the American Society of Preventive Oncology, 2000. 9(10): p. 1087-90.
328. Soria, J.C., et al., Effects of N-(4-hydroxyphenyl)retinamide on hTERT expression in the bronchial epithelium of cigarette smokers. Journal of the National Cancer Institute, 2001. 93(16): p. 1257-63.
329. Tae, K., et al., Expression of vascular endothelial growth factor and microvessel density in head and neck tumorigenesis. Clinical cancer research : an official journal of the American Association for Cancer Research, 2000. 6(7): p. 2821-8.
330. Voravud, N., et al., Increased polysomies of chromosomes 7 and 17 during head and neck multistage tumorigenesis. Cancer research, 1993. 53(12): p. 2874-83.
331. Drezek, R.A., et al., Laser scanning confocal microscopy of cervical tissue before and after application of acetic acid. American journal of obstetrics and gynecology, 2000. 182(5): p. 1135-9.
332. Delaney, P.M. and M.R. Harris, Fiber Optics in Confocal Microscope, in Handbook of Confocal Microscopy, J.B. Pawley, Editor. 1995, Plenum Press: New York. p. 515-523.
333. Smithpeter, C.L., et al., Near Real Time Confocal Microscopy of Cultured Amelanotic Cells: Sources of Signal, Contrast Agents, and Limits of Contrast. Journal of Biomedical Optics, 1998. 3(4): p. 429-436.
334. Minsky, M., Microscopy Apparatus. 1961: U. S.
335. Liang, C., et al., Design of a high-numerical-aperture miniature microscope objective for an endoscopic fiber confocal reflectance microscope. Applied Optics, 2002. 41 (22): p. 4603-4610.
336. Sung, K.B., et al., Near real time in vivo fibre optic confocal microscopy: sub-cellular structure resolved. Journal of microscopy, 2002. 207(Pt) 2: p. 137-45.
337. Sung, K.-B., et al., Fiber-Optic Confocal Reflectance Microscope With Miniature Objective for In Vivo Imaging of Human Tissues. IEEE Transactions on Biomedical Engineering, 2002. 49(10): p. 1168-1172.
338. Hornbeck, L.J. Digital light processing and MEMS: Reflecting the digital display needs of the networked society, in SPIE/EOS European Symposium on Lasers, Optics, and Vision for productivity in Manufacturing 1, Conference on Micro-Optical Tech. For measurement, Sensors & Microsystems,. 1996. Besacon, France.
339. Lane, P.M., et al., Medical Optics and Biotechnology - Fiber-optic confocal microscopy using a spatial light modulator. Optics letters, 2000. 25(24): p. 1780-1782.
340. Descour, M.R., et al., Toward the development of miniaturized imaging systems for detection of pre-cancer. IEEE Journal of Quantum Electronics, 2002. 38(2): p. 122-130.
341. Sokolov, K., G. Chumanov, and T.M. Cotton, Enhancement of Molecular Fluorescence near the Surface of Colloidal Metal Films. Analytical Chemistry, 1998. 70(18): p. 3898-3905.
342. Ari H. O. Kärkkäinen, J.T.R., Michael R. Descour. Photolithographic processing of hybrid glasses for micro-optics applications, in Optical EMS IEEE/LEOS, Okinawa, Japan.
343. J. D. Rogers, M.R.R.a.A.H.O.K. Fabrication and assembly of miniature imaging systems using lithographically patterned micro-optics and silicon microstructures, in Diffractive Optics and Micro-optics. 2000: Optical Society of America.
344. Horisberger, M., Colloidal gold : a cytochemical marker for light and fluorescent microscopy and for transmission and scanning electron microscopy. Scan Electron Microsc, 1981. 2: p. 9-31.
345. Geoghegan, W.D. and G.A. Ackerman, Adsorption of horseradish peroxidase, ovomucoid and anti-immunoglobulin to colloidal gold for the indirect detection of concanavalin A, wheat germ agglutinin and goat anti-human immunoglobulin G on cell surfaces at the electron microscopic level: a new method, theory and application. The journal of histochemistry and cytochemistry : official journal of the Histochemistry Society, 1977. 25(11): p. 1187-200.
346. Mulvaney, P., Surface Plasmon Spectroscopy of Nanosized Metal Particles. Langmuir, 1996. 12(3): p. 788-800.
347. Nabiev, I.R., H. Morjani, and M. Manfait, Selective analysis of antitumor drug interaction with living cancer cells as probed by surface-enhanced Raman spectroscopy. European biophysics journal : EBJ, 1991. 19(6): p. 311-6.
348. Kneipp, K., et al., Surface-Enhanced Raman Spectroscopy in Single Living Cells Using Gold Nanoparticles. Applied Spectroscopy, 2002. 56(2): p. 150 - 154.
349. Nie, S. and S.R. Emory, Probing single molecules and single nanoparticles by surface-enhanced Raman scattering. Science, 1997. 275(5303): p. 1102-1106.
350. Elghanian, R., et al., Selective colorimetric detection of polynucleotides based on the distance-dependent optical properties of gold nanoparticles. Science, 1997. 277(5329): p. 1078-1080.
351. Yasuda, R., et al., Resolution of distinct rotational substeps by submillisecond kinetic analysis of F1-ATPase. Nature, 2001. 410(6831): p. 898-904.
352. Schultz, S., et al., Single-target molecule detection with nonbleaching multicolor optical immunolabels. Proceedings of the National Academy of Sciences of the United States of America, 2000. 97(3): p. 996-1001.
353. Monaghan, P., M.G. Ormerod, and M.J. O'Hare, Epidermal growth factor receptors and EGF-responsiveness of the human breast-carcinoma cell line PMC42. International journal of cancer Journal international du cancer, 1990. 46(5): p. 935-43.
354. Carpenter, G., Receptors For Epidermal Growth Factor And Other Polypeptide Mitogens. Annual Review of Biochemistry, 1987. 56: p. 881-914.
355. Hahn-Dantona, E., et al., The low density lipoprotein receptor-related protein modulates levels of matrix metalloproteinase 9 (MMP-9) by mediating its cellular catabolism. The Journal of biological chemistry, 2001. 276(18): p. 15498-503.
356. Friedl, P. and U.o.W.G.p.f.m.u.-w.d. Bröcker Eb Affiliation: Department of Dermatology, The biology of cell locomotion within three-dimensional extracellular matrix.
357. Abrams, M.J. and J.M.I.W.C.P.A. Murrer Ba Affiliation: Biomedical Research Worldwide, Metal compounds in therapy and diagnosis.
358. Berkower I Affiliation: Laboratory of Immunoregulation, D.o.A.P., O.o.V.R.C.f.B.F. Parasitology, and B.M.D.U.S.A.B.a.c.f.g. Drug Administration, The promise and pitfalls of monoclonal antibody therapeutics.
359. Holliger, P. and C.U.K.p.m.-l.c.a.u. Bohlen H Affiliation: Mrc Laboratory for Molecular Biology, Engineering antibodies for the clinic.
360. Weiner, L.M., An overview of monoclonal antibody therapy of cancer. Seminars in oncology, 1999. 26(4) Suppl 12: p. 41-50.
361. Soukos, N.S., et al., Epidermal growth factor receptor-targeted immunophotodiagnosis and photoimmunotherapy of oral precancer in vivo. Cancer research, 2001. 61 (11): p. 4490-6.
362. Achilefu, S., et al. New approach to optical imaging of tumors. in Proceedings of SPIE: Biomarkers and Biological Spectral Imaging. 2001. San Jose, CA.
363. Achilefu, S., et al. Site-specific tumor targeted fluorescent contrast agents. in Proceedings of SPIE: Clinical Lasers and Diagnostics. 2001. Amsterdam.
364. Bugaj, J.E., et al., Novel fluorescent contrast agents for optical imaging of in vivo tumors based on a receptor-targeted dye-peptide conjugate platform. Journal of biomedical optics, 2001. 6(2): p. 122-33.
365. Tung, C.H., et al., A receptor-targeted near-infrared fluorescence probe for in vivo tumor imaging. Chembiochem, 2002. 3(8): p. 784-786.
366. Becker, A., et al., Receptor-targeted optical imaging of tumors with near-infrared fluorescent ligands. Nature biotechnology, 2001. 19(4): p. 327-31.
367. Bremer, C., C.H. Tung, and R. Weissleder, In vivo molecular target assessment of matrix metalloproteinase inhibition. Nature medicine, 2001. 7(6): p. 743-8.
368. Tung, C.H., et al., In vivo imaging of proteolytic enzyme activity using a novel molecular reporter. Cancer research, 2000. 60(17): p. 4953-8.
369. Farina, K.L., et al., Cell motility of tumor cells visualized in living intact primary tumors using green fluorescent protein. Cancer research, 1998. 58(12): p. 2528-32.
370. Bomhop, D.J., et al., Advance in contrast agents, reporters, and detection. Journal of biomedical optics, 2001. 6(2): p. 106-10.
371. Desmettre, T., J.M. Devoisselle, and S. Mordon, Fluorescence properties and metabolic features of indocyanine green (ICG) as related to angiography. Survey of ophthalmology, 2000. 45(1): p. 15-27.
372. Slakter, J.S., et al., Indocyanine-green angiography. Current opinion in ophthalmology, 1995. 6(3): p. 25-32.
373. Haglund, M.M., M.S. Berger, and D.W. Hochman, Enhanced optical imaging of human gliomas and tumor margins. Neurosurgery, 1996. 38(2): p. 308-17.
374. Anikijenko, P., et al., In vivo detection of small subsurface melanomas in athymic mice using noninvasive fiber optic confocal imaging. The Journal of investigative dermatology, 2001. 117(6): p. 1442-8.
375. Ito, S., et al., Detection of human gastric cancer in resected specimens using a novel infrared fluorescent anti-human carcinoembryonic antigen antibody with an infrared fluorescence endoscope in vitro. Endoscopy, 2001. 33(10): p. 849-53.
376. Banin, U., et al., Size-dependent electronic level structure of InAs nanocrystal quantum dots: test of multiband effective mass theory. Journal of Chemical Physics, 1998. 109(6): p. 2306.
377. Murray, C.B., D.J. Norris, and M.G. Bawendi, Synthesis and Characterization of Nearly Monodisperse CdE (E = sulfur, selenium, tellurium) Semiconductor Nanocrystallites. Journal of the American Chemical Society, 1993. 115: p. 8706-8715.
378. Lacoste, T.D., et al., Ultrahigh-resolution multicolor colocalization of single fluorescent probes. Proceedings of the National Academy of Sciences of the United States of America, 2000. 97(17): p. 9461-6.
379. Chan, W.C., et al., Luminescent quantum dots for multiplexed biological detection and imaging. Current opinion in biotechnology, 2002. 13(1): p. 40-6.
380. Parak, W.J., et al., Cell motility and metastatic potential studies based on quantum dot imaging of phagokinetic tracks. Advanced Materials, 2002. 14(12): p. 882-885.
381. Akerman, M.E., et al., Nanocrystal targeting in vivo. Proceedings of the National Academy of Sciences of the United States of America, 2002. 99(20): p. 12617-21.
382. Dubertret, B., et al., In vivo imaging of quantum dots encapsulated in phospholipid micelles. Science, 2002. 298(5599): p. 1759-1762.
383. Bruchez, M., Jr., et al., Semiconductor nanocrystals as fluorescent biological labels. Science, 1998. 281(5385): p. 2013-6.
384. Chan, W.C. and S. Nie, Quantum dot bioconjugates for ultrasensitive nonisotopic detection. Science, 1998. 281(5385): p. 2016-8.
385. Willingham, M.C., Immunocytochemical Methods and Protocols, in Methods in Molecular Biology, L.C. Javois, Editor. 1999, Humana Press Inc.: Totowa, NJ. p. 121-130.
386. Hemler, M.E., VLA proteins in the integrin family: structures, functions, and their role on leukocytes. Annual review of immunology, 1990. 8: p. 365-400.
387. Yoshihara, T., et al., Unique expression of integrin fibronectin receptors in human neuroblastoma cell lines. International journal of cancer, 1992. 51(4): p. 620-6.
388. Pierschbacher, M.D. and E. Ruoslahti, Cell attachment activity of fibronectin can be duplicated by small synthetic fragments of the molecule. Nature, 1984. 309(5963): p. 30-3.
389. Fry, W.A., H.R. Menck, and D.P. Winchester, The National Cancer Data Base report on lung cancer. Cancer, 1996. 77(9): p. 1947-55.
390. Henderson, S., et al. Serial Analysis of Gene Expression (SAGE) in Oral Premalignant Lesions. in 31st Annual Meeting and Exposition of the AADR. 2003.
391. Lonergan, K., et al., Comparing Expression Profiles of Lung Cancer Progression by SAGE. American Association for Cancer Research Proceedings, 2002. 43: p. 472.
392. Ma, S., et al., A concerted genomic and proteomic approach to understanding the early steps in the genesis of prostate cancer. American Association for Cancer Research Proceedings, 2002. 43: p. 683.
393. Ma, S., et al., Optimization for integrity and yield of DNA extraction from laser capture microdissected cells from archival biopsies. American Association for Cancer Research Proceedings, 2001. 42: p. 532.
394. Gorski, S., et al., A SAGE Approach to Discovery of Genes Involved in Autophagic Cell Death. Current Biology, Accepted.
395. Arap, W., et al., Steps toward mapping the human vasculature by phage display. Nat Med, 2002. 8(2): p. 121-7.
396. Rajotte, D., et al., Molecular heterogeneity of the vascular endothelium revealed by in vivo phage display. J Clin Invest, 1998. 102(2): p. 430-7.
397. Koivunen, E., et al., Tumor targeting with a selective gelatinase inhibitor. Nat Biotechnol, 1999. 17(8): p. 768-74.
398. Wickham, T.J., et al., Targeting endothelium for gene therapy via receptors up-regulated during angiogenesis and inflammation. Cancer Immunol Immunother, 1997. 45(3-4): p. 149-51.
399. Zeman, E.J. and G.C. Schatz, An Accurate Electromagnetic Theory Study of Surface Enhancement Factors for Ag, Au, Cu, Li, Na, Al, Ga, In, Zn, and Cd. Journal of Physical Chemistry, 1987. 91(3): p. 634-43.
400. Wilenzick, R.M., D.C. Russell, and R.H. Morriss, Uniform Microcrystals of Platinum and Gold. Journal of Chemical Physics, 1967. 47: p. 533-536.
401. Schneider, S., et al., Reproducible Preparation of Silver Sols with Uniform Particle Size for Application in Surface-Enhanced Raman Spectroscopy. Photochemistry and Photobiology, 1988. 60(6): p. 605-610.
402. Frens, G., Controlled Nucleation for the Regulation of the Particle Size in Monodisperse Gold Suspensions. Nature Physical Science, 1973. 241: p. 20-22.
403. Lee, P.C. and T.J. Meisel, Adsorption and Surface-Enhanced Raman of Dyes on Silver and Gold Soles. Journal of Physical Chemistry, 1982. 86: p. 3391-95.
404. Hildebrandt, P. and M. Stockburger, Surface-Enhanced Resonance Raman spectroscopy of Rhodamine 6G Adsorbed on Colloidal Silver. Journal of Physical Chemistry, 1984. 88(24): p. 5935-5944.
405. Cotton, T.M. Spectroscopy of Surfaces, ed. R.E. Hester. 1988, New York: Wiley. 91.
406. Sershen, S.R., et al., Temperature-sensitive polymer-nanoshell composites for photothermally modulated drug delivery. Journal of biomedical materials research, 2000. 51(3): p. 293-8.
407. Averitt, R.D., S.L. Westcott, and N.J. Halas, The Linear Optical Properties of Gold Nanoshells. J. Opt Soc Am B, 1999. 16: p. 1824-32.
408. Haes, A.J. and R.P. Van Duyne, A nanoscale optical biosensor: Sensitivity and selectivity of an approach based on the localized surface plasmon resonance spectroscopy of triangular silver nanoparticles. Journal of the American Chemical Society, 2002. 124(35): p. 10596-10604.
409. G.F. Paciotti, L.D.M., T.H. Kim, S. Wang, H.R. Alexander, D. and a.L.T. Weinrech. Colloidal gold: a novel colloidal nanoparticle vector for tumor-directed drug dlivery. in Proceedings of of AACR-NCI-EORTC International Conference on "Molecular Targets and Cancer Therapeutics: Discovery, Biology, and Clinical Applications. 2001. Miami Beach, Florida.
410. Newman, G.R. and J.A. Hobot, Modem acrylics for post-embedding immunostaining techniques. The journal of histochemistry and cytochemistry : official journal of the Histochemistry Society, 1987. 35(9): p. 971-81.
411. Bhatia, S.K., et al., Use of thiol-terminal silanes and heterobifunctional crosslinkers for immobilization of antibodies on silica surfaces. Analytical biochemistry, 1989. 178(2): p. 408-13.
412. Deutscher, M.P., ed. Guide to protein purification. Methods in enzymology. Vol. 182. 1990, Academic Press: San Diego, CA.
413. Korgel, B.A. and D. Fitzmaurice, Small-angle x-ray-scattering study of silver-nanocrystal disorder-order phase transitions. Physical Review B: Condensed Matter and Materials Physics, 1999. 59(22): p. 14191-14201.
414. Holmes, J.D., et al., Highly Luminescent Silicon Nanocrystals with Discrete Optical Transitions. Journal of the American Chemical Society, 2001. 123(16): p. 3743-3748.
415. Chen, H.M., et al., Self-assembly and photoluminescence of CdS-mercaptoacetic clusters with internal clusters. Superlattices and Microstructures, 2000. 27(1).
416. Muguruma, N., et al., Antibodies labeled with fluorescence-agent excitable by infrared rays. Journal of gastroenterology, 1998. 33(4): p. 467-71.
417. Lehr, C.M., et al., Effects of the mucoadhesive polymer polycarbophil on the intestinal absorption of a peptide drug in the rat. The Journal of pharmacy and pharmacology, 1992. 44(5): p. 402-7.
418. Luessen, H.L., et al., Mucoadhesive polymers in peroral peptide drug delivery. II. Carbomer and polycarbophil are potent inhibitors of the intestinal proteolytic enzyme trypsin. Pharmaceutical research, 1995. 12(9): p. 1293-8.
419. Lehr, C.M., Y.H. Lee, and V.H. Lee, Improved ocular penetration of gentamicin by mucoadhesive polymer polycarbophil in the pigmented rabbit. Investigative ophthalmology & visual science, 1994. 35(6): p. 2809-14.
420. Bemkop-Schnurch, A., S. Scholler, and R.G. Biebel, Development of controlled drug release systems based on thiolated polymers. Journal of controlled release, 2000. 66(1): p. 39-48.
421. Eouani, C., et al., In-vitro comparative study of buccal mucoadhesive performance of different polymeric films. European Journal of Pharmaceutics and Biopharmaceutics, 2001. 52(1): p. 45-55.
422. Kerec, M., et al., Mucoadhesion on pig vesical mucosa: influence of polycarbophil/calcium interactions. International journal of pharmaceutics, 2002. 241(1): p. 135-43.
423. Chang, J.Y., et al., Rheological evaluation of thermosensitive and mucoadhesive vaginal gels in physiological conditions. International journal of pharmaceutics, 2002. 241 (1): p. 155-63.
424. Yun Chang, J., et al., Prolonged antifungal effects of clotrimazole-containing mucoadhesive thermosensitive gels on vaginitis. Journal of controlled release, 2002. 82(1): p. 39-50.
425. Park, H. and J.R. Robinson, Mechanisms of mucoadhesion of poly(acrylic acid) hydrogels. Pharmaceutical research, 1987. 4(6): p. 457-64.
426. Lee, C.H. and Y.W. Chien, In vitro permeation study of a mucoadhesive drug delivery system for controlled delivery of nonoxynol-9. Pharmaceutical development and technology, 1996. 1(2): p. 135-45.
427. Warren, S.J. and I.W. Kellaway, The synthesis and in vitro characterization of the mucoadhesion and swelling of poly(acrylic acid) hydrogels. Pharmaceutical development and technology, 1998. 3(2): p. 199-208.
428. Rossi, S., et al., Drug release and washability of mucoadhesive gels based on sodium carboxymethylcellulose and polyacrylic acid. Pharmaceutical development and technology, 1999. 4(1): p. 55-63.
429. Oechsner, M. and S. Keipert, Polyacrylic acid/polyvinylpyrrolidone bipolymeric systems. I. Rheological and mucoadhesive properties of formulations potentially useful for the treatment of dry-eye-syndrome. European Journal of Pharmaceutics and Biopharmaceutics, 1999. 47(2): p. 113-8.
430. Chun, M.K., C.S. Cho, and H.K. Choi, Mucoadhesive drug carrier based on interpolymer complex of poly(vinyl pyrrolidone) and poly(acrylic acid) prepared by template polymerization. Journal of controlled release, 2002. 81(3): p. 327-34.
431. Ugwoke, M.I., et al., Scintigraphic evaluation in rabbits of nasal drug delivery systems based on carbopol 971p((R)) and carboxymethylcellulose. Journal of controlled release, 2000. 68(2): p. 207-14.
432. Singla, A.K. and M. Chawla, Chitosan: some pharmaceutical and biological aspects--an update. The Journal of pharmacy and pharmacology, 2001. 53(8): p. 1047-67.
433. Ferrari, F., et al., Characterization of rheological and mucoadhesive properties of three grades of chitosan hydrochloride. Farmaco, 1997. 52(6-7): p. 493-7.
434. Bernkop-Schnurch, A., Chitosan and its derivatives: potential excipients for peroral peptide delivery systems. International journal of pharmaceutics, 2000. 194(1): p. 1-13.
435. Filipovic-Grcic, J., N. Skalko-Basnet, and I. Jalsenjak, Mucoadhesive chitosan-coated liposomes: characteristics and stability. Journal of microencapsulation, 2001. 18(1): p. 3-12.
436. Dodane, V., M. Amin Khan, and J.R. Merwin, Effect of chitosan on epithelial permeability and structure. Int J Pharm, 1999. 182(1): p. 21-32.
437. Kotze, A.F., et al., Enhancement of paracellular drug transport with highly quaternized N-trimethyl chitosan chloride in neutral environments: in vitro evaluation in intestinal epithelial cells (Caco-2). Journal of pharmaceutical sciences, 1999. 88(2): p. 253-7.
438. Kotze, A.F., et al., N-trimethyl chitosan chloride as a potential absorption enhancer across mucosal surfaces: in vitro evaluation in intestinal epithelial cells (Caco-2). Pharmaceutical research, 1997. 14(9): p. 1197-202.
439. Thanou, M., J.C. Verhoef, and H.E. Junginger, Oral drug absorption enhancement by chitosan and its derivatives. Advanced Drug Delivery Reviews, 2001. 52(2): p. 117-26.
440. Thanou, M., J.C. Verhoef, and H.E. Junginger, Chitosan and its derivatives as intestinal absorption enhancers. Advanced Drug Delivery Reviews, 2001. 50 Suppl 1: p. S91-101.
441. Senel, S. and A.A. Hincal, Drug permeation enhancement via buccal route: possibilities and limitations. Journal of controlled release, 2001. 72(1-3): p. 133-44.
442. Thanou, M., et al., N-trimethylated chitosan chloride (TMC) improves the intestinal permeation of the peptide drug buserelin in vitro (Caco-2 cells) and in vivo (rats). Pharmaceutical research, 2000. 17(1): p. 27-31.
443. Kast, C.E., et al., Design and in vitro evaluation of a novel bioadhesive vaginal drug delivery system for clotrimazole. Journal of controlled release, 2002. 81(3): p. 347-54.
444. Genta, I., et al., Microparticulate drug delivery systems. Exs, 1999. 87: p. 305-13.
445. McIntyre, G.M.M.a.K.J., Optical system design with diffractive optics, in Diffractive Optics for Industrial and Commercial Applications, J.T.a.F. Wyrowski, Editor. 1997, Akademie Verlag. p. 95.
446. Coghlan, L., et al., Fluorescence spectroscopy of epithelial tissue throughout the dysplasia-carcinoma sequence in an animal model: spectroscopic changes precede morphologic changes. Lasers in surgery and medicine, 2001. 29(1): p. 1-10.
447. Brookner, C.K., et al., Autofluorescence patterns in short-term cultures of normal cervical tissue. Photochemistry and photobiology, 2000. 71(6): p. 730-6.
448. Drezek, R., et al., Autofluorescence microscopy of fresh cervical-tissue sections reveals alterations in tissue biochemistry with dysplasia. Photochemistry and photobiology, 2001. 73(6): p. 636-41.
449. Miller, F.R., D. McEachern, and B.E. Miller, Growth regulation of mouse mammary tumor cells in collagen gel cultures by diffusible factors produced by normal mammary gland epithelium and stromal fibroblasts. Cancer research, 1989. 49(21): p. 6091-7.
450. Hoffman, R.M., Three-dimensional histoculture: origins and applications in cancer research. Cancer cells, 1991. 3(3): p. 86-92.
451. Krumdieck, C.L., J.E. dos Santos, and K.J. Ho, A new instrument for the rapid preparation of tissue slices. Analytical biochemistry, 1980. 104(1): p. 118-23.
452. Smith, P.F., et al., Dynamic organ culture of precision liver slices for in vitro toxicology. Life sciences, 1985. 36(14): p. 1367-75.
453. Smith, P.F., et al., Maintenance of adult rat liver slices in dynamic organ culture. In vitro cellular & developmental biology : journal of the Tissue Culture Association, 1986. 22(12): p. 706-12.
454. Gimenez-Conti, I.B., et al., Changes in keratin expression during 7,12-dimethylbenz[a]anthracene-induced hamster cheek pouch carcinogenesis. Cancer research, 1990. 50(14): p. 4441-5.
455. Zenklusen, J.C., et al., Transforming growth factor-beta 1 expression in Syrian hamster cheek pouch carcinogenesis. Molecular carcinogenesis, 1994. 9(1): p. 10-6.
456. Hamada, K., et al., The nude rat as an orthotopic model for cervical cancer. submitted to Gynecologic Oncology, 2002.
457. Sumida, T., et al., Telomerase activation and cell proliferation during 7,12-dimethylbenz[a]anthracene-induced hamster cheek pouch carcinogenesis. Molecular carcinogenesis, 1999. 25(3): p. 164-8.
458. Maekawa, K., et al., Inhibition of cervical lymph node metastasis by marimastat (BB-2516) in an orthotopic oral squamous cell carcinoma implantation model. Clinical & experimental metastasis, 2002. 19(6): p. 513-8.
459. Shinohara, M., et al., Expression of integrins in squamous cell carcinoma of the oral cavity. Correlations with tumor invasion and metastasis. American journal of clinical pathology, 1999. 111(1): p. 75-88.
460. Yguerabide, J. and E. Yguerabide, Resonance Light Scattering Particles as Ultrasensitive Labels for Detection of Analytes in a Wide Range of Applications. Journal of Cellular Biochemistry, 2001. S37: p. 71-81.
461. Charous, S.J., et al., Expression of matrix metalloproteinases and tissue inhibitor of metalloproteinases in head and neck squamous cell carcinoma. The Annals of otology, rhinology, and laryngology,1997. 106(4): p. 271-8.
462. Sutinen, M., et al., Expression of matrix metalloproteinases (MMP-1 and -2) and their inhibitors (TIMP-1, -2 and -3) in oral lichen planus, dysplasia, squamous cell carcinoma and lymph node metastasis. British journal of cancer, 1998. 77(12): p. 2239-45.
463. Ke, L.D., et al., Expression of human papillomavirus E7 mRNA in human oral and cervical neoplasia and cell lines.
464. Ke, L.D., et al., Differential expression of epidermal growth factor receptor in human head and neck cancers.
465. Hoffman, P., et al., Adenovirus E3 Protein Causes Constitutively Internalized Epidermal Growth Factor Receptor to Accumulate in a Prelysosomal Compartment, Resulting in Enhanced Degradation. Molecular and Cellular Biology, 1994. 14 (6) : p. 3695-3706.

## Claims

1. One or more metallic nanoparticle(s) attached to a molecular probe specific for a biomarker for use in *in vivo* diagnosis of cervical cancer through reflectance imaging, wherein the metallic nanoparticle(s) exhibit characteristic optical scattering when subjected to reflectance imaging.

2. The one or more metallic nanoparticle(s) for the use according to claim 1, wherein the one or more metallic nanoparticle(s) comprise(s) gold or silver.

3. The one or more metallic nanoparticles(s) for the use according to claim 1 or 2, wherein the biomarker is EGFR, MMP-2 or MMP-9.

4. Use of one or more metallic nanoparticle(s) attached to a molecular probe specific for a biomarker in the manufacture of a diagnostic agent for the *in vivo* diagnosis of cervical cancer through reflectance imaging, wherein the metallic nanoparticle(s) exhibit characteristic optical scattering when subjected to reflectance imaging.

5. The use according to claim 4, wherein the one or more metallic nanoparticles(s) comprise(s) gold or silver.

6. The use according to claim 4 or 5, wherein the biomarker is EGFR, MMP-2 or MMP-9.

## Patentansprüche

1. Ein oder mehrere metallische Nanopartikel, die an einer molekularen Sonde angeheftet sind, die spezifisch für einen Biomarker ist, zur Verwendung für die *in vivo*-Diagnose von Gebärmutterhalskrebs durch Reflexionsbildgebung, wobei der/die metallische(n) Nanopartikel eine charakteristische optische Streuung aufweist/aufweisen, wenn er/sie einer Reflexionsbildgebung unterzogen wird/werden.

2. Der eine oder mehrere metallische Nanopartikel zur Verwendung nach Anspruch 1, wobei der eine oder mehrere metallische Nanopartikel Gold oder Silber umfasst/umfassen.

3. Der eine oder mehrere metallische Nanopartikel zur Verwendung nach Anspruch 1 oder 2, wobei der Biomarker EGFR, MMP-2 oder MMP-9 ist.

4. Verwendung eines oder mehrerer metallischen/metallischer Nanopartikel(s), der/die an eine molekulare Sonde angeheftet ist/sind, die spezifisch für einen Biomarker ist, zur Herstellung eines diagnostischen Mittels für die *in vivo*-Diagnose von Gebärmutterhalskrebs durch Reflexionsbildgebung, wobei der/die metallische(n) Nanopartikel eine charakteristische optische Streuung aufweist/aufweisen, wenn er/sie einer Reflexionsbildgebung unterzogen werden.

5. Die Verwendung nach Anspruch 4, wobei der eine oder mehrere metallische Nanopartikel Gold oder Silber umfasst/umfassen.

6. Die Verwendung nach Anspruch 4 oder 5, wobei der Biomarker EGFR, MMP2 oder MMP9 ist.

## Revendications

1. Une ou plusieurs nanoparticule(s) métallique(s) attachée(s) à une sonde moléculaire spécifique d'un biomarqueur pour une utilisation dans le diagnostic *in vivo* du cancer cervical par imagerie par réflexion, dans laquelle la(les) nanoparticule(s) métallique(s) présentent une diffusion de la lumière caractéristique quand elle(s) est(sont) soumise(s) à une imagerie par réflexion.

2. Une ou plusieurs nanoparticule(s) métallique(s) pour l'utilisation selon la revendication 1, dans laquelle la ou les nanoparticule(s) métallique(s) comporte(nt) de l'or ou de l'argent.

3. Une ou plusieurs nanoparticule(s) métallique(s) pour l'utilisation selon la revendication 1 ou 2, dans laquelle le biomarqueur est EGFR, MMP-2 ou MMP-9.

4. Utilisation d'une ou plusieurs nanoparticule(s) métallique(s) attachée(s) à une sonde moléculaire spécifique d'un biomarqueur dans la fabrication d'un agent de diagnostic pour le diagnostic *in vivo* du cancer cervical par imagerie par réflexion, dans laquelle la(les) nanoparticule(s) métallique(s) présentent une diffusion de la lumière caractéristique quand elle(s) est(sont) soumise(s) à une imagerie par réflexion.

5. Utilisation selon la revendication 4, dans laquelle la ou les nanoparticule(s) métallique(s) comporte(nt) de l'or ou de l'argent.

6. Utilisation selon la revendication 4 ou 5, dans laquelle le biomarqueur est EGFR, MMP-2 ou MMP-9.
